(19) Europäisches Patentamt
European Patent Office
Office européen des brevets

(11) EP 4 484 412 B1

(12) **EUROPEAN PATENT SPECIFICATION**

(45) Date of publication and mention of the grant of the patent:
**29.07.2026 Bulletin 2026/31**

(21) Application number: **22930534.7**

(22) Date of filing: **17.08.2022**

(51) International Patent Classification (IPC):
***C07D 235/02*** (2006.01) ***C07D 409/06*** (2006.01)
***C07D 405/06*** (2006.01) ***C07D 403/06*** (2006.01)
***C07D 405/04*** (2006.01) ***C07D 409/04*** (2006.01)
***C07D 405/12*** (2006.01) ***A61K 31/4184*** (2006.01)
***A61K 31/497*** (2006.01) ***A61P 35/00*** (2006.01)
***A61P 31/04*** (2006.01) ***A61P 31/12*** (2006.01)

(52) Cooperative Patent Classification (CPC):
**C07D 235/02; A61P 31/04; A61P 31/12; A61P 35/00; C07D 401/04; C07D 401/14; C07D 403/06; C07D 405/04; C07D 405/06; C07D 405/12; C07D 409/04; C07D 409/06; C07D 471/04**

(86) International application number:
**PCT/CN2022/112932**

(87) International publication number:
**WO 2023/168908 (14.09.2023 Gazette 2023/37)**

(54) **FUSED QUINONE OXIME IMIDAZOLE, ONIUM DERIVATIVE THEREOF, PREPARATION THEREFOR AND USE THEREOF**

KONDENSIERTE CHINONOXIMIMIDAZOLE, DEREN ONIUMDERIVATE, DEREN HERSTELLUNG UND VERWENDUNG

QUINONE OXIME IMIDAZOLE FUSIONNÉ, SON DÉRIVÉ ONIUM, PRÉPARATION CORRESPONDANTE ET UTILISATION ASSOCIÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.03.2022 CN 202210224058**

(43) Date of publication of application:
**01.01.2025 Bulletin 2025/01**

(73) Proprietor: **Shanghai Jiao Tong University**
**Shanghai 200240 (CN)**

(72) Inventors:
- **ZHANG, Wanbin**
  **Shanghai 200240 (CN)**
- **YAN, Deyue**
  **Shanghai 200240 (CN)**
- **YUAN, Jing**
  **Shanghai 200240 (CN)**
- **ZHANG, Zhenfeng**
  **Shanghai 200240 (CN)**

(74) Representative: **Novagraaf Group**
**Chemin de l'Echo 3**
**1213 Onex / Geneva (CH)**

(56) References cited:
**WO-A2-92/19211** **CN-A- 1 400 969**
**CN-A- 108 530 365** **CN-A- 108 794 403**
**RU-C1- 2 545 091**

- **SHANAB, K. ET AL.: "Synthesis and Biological Evaluation of Novel Cytotoxic Azanaphthoquinone Annelated Pyrrolo Oximes", BIOORGANIC & MEDICINAL CHEMISTRY LETTERS, vol. 17, no. 22, 18 September 2007 (2007-09-18), XP022297103, ISSN: 0960-894X, DOI: 10.1016/j.bmcl.2007.09.054**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- PUNKVANG, A. ET AL.: "Molecular Dynamics Simulations of Azanaphthoquinone Annelated Pyrrole Derivatives as Anticancer Agent in DNA Duplex", INTERNATIONAL JOURNAL OF QUANTUM CHEMISTRY, vol. 113, no. 4, 7 April 2012 (2012-04-07), XP071308132, ISSN: 0020-7608, DOI: 10.1002/qua.24120
- KAMSRI PHARIT, PUNKVANG AURADEE, PONGPROM NIPAWAN, SRISUPAN APINYA, SAPARPAKORN PATCHREENART, HANNONGBUA SUPA, WOLSCHANN PETER, PU: "Key Structural Features of Azanaphthoquinone Annelated Pyrrole Derivative as Anticancer Agents Based on the Rational Drug Design Approaches", MOLECULAR INFORMATICS, JOHN WILEY & SONS, INC., HOBOKEN, USA, vol. 32, no. 5-6, 1 June 2013 (2013-06-01), Hoboken, USA, pages 541 - 554, XP093090523, ISSN: 1868-1743, DOI: 10.1002/minf.201200132

## Description

### TECHNICAL FIELD

The present invention falls within the field of pharmaceuticals, and relates to a fused quinonoxime imidazole, an onium derivative thereof, preparation and use thereof, and in particular to a category of antibacterial, antiviral quinonoxime imidazolium derivatives useful for the treatment of cancer and preparation thereof, newly synthesized intermediates and preparation thereof.

### BACKGROUND TECHNOLOGY

Compounds containing quinones extensively exist in natural world, and many of them have good bioactivity; for example, doxorubicin and mitomycin C can be used as chemotherapeutic agents in clinical application to treat a variety of cancers; alkannin and its derivatives have anti-inflammatory, anticancer, antiviral, anti-thrombotic, glucose-lowering, liver-protecting, and other multiple biological activities. Synthesized quinone-containing drugs also have been applied widely, e.g., doxorubicin and pharmorubicin are very good front-line anticancer agents. In recent years, people have concerned studies on the antitumor activity of active substances containing quinones. N-heterocyclic carbene is a category of extensively-applied metal organic-catalyst ligands and small-molecular catalysts with excellent activity; and it is efficient, easy to get, easy to modify, cheap, etc., which is highly identical to the features of pharmaceutical molecules.

There are two categories of structure modifications on quinone imidazole derivatives reported by the existing literatures; one is accidental, unsystematic sporadic development of mono-substituted imidazole products (US patent US 20090163545 A1, published on June 25, 2009; international patents WO 2009023773 A2, published on February 19, 2009 and WO 2012149523 A1, published on November 01, 2012); another one is to synthesize 2-substituted naphthoquinone imidazole non-carbene precursor derivatives by introducing a fixed group at position 2 of naphthoquinone imidazole and then modifying the imidazole structure (Japanese patent JP 2014156400 A, published on August 29, 2014; US patents US 20150086535 A1, published on March 26, 2015; international patents 2001060803 A1 20010823, 2004092160 A1 20041028, 2012161177 A1 20121129, 2013148649 A1 20131003, and 2014142220 A1 20140918). In the present invention, quinonoids are organically combined with carbene and then subjected to oximation by fusion of subjects, which designs and synthesizes a series of quinonoxime imidazolium derivatives with efficient antitumor activity for the first time.

### CONTENT OF THE INVENTION

At present, cancer is mainly treated by chemotherapy, but toxicity of chemotherapeutic agents used clinically cannot be ignored. Therefore, there is an urgent need for developing high-activity, low-toxic, safe and reliable anti-cancer drugs. It has been found through in-depth studies on quinone drugs in the present invention that toxicity of quinones is mainly derived from ROS generated in the transformation of quinone into quinonoxime. In addition, natural quinonoxime product alkannin has good antitumor activity. The objective of the present invention is thus to provide a fused quinonoxime imidazole, an onium derivative thereof, preparation and use thereof. These compounds are novel in structure and easy to be prepared. Based on *in vitro* and *in vivo* test results, these compounds show good antitumor activity, low toxicity, safety and reliability.

The objective of the present invention is achieved by the following technical solutions:

In a first aspect, the present invention relates to a fused quinonoxime imidazolium derivative represented by the following Formula (I) and (II);

the structural formula is shown in:

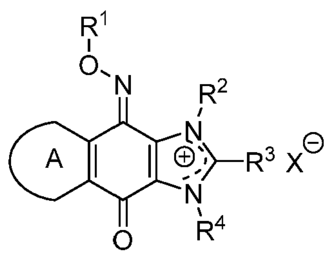

(I)

and

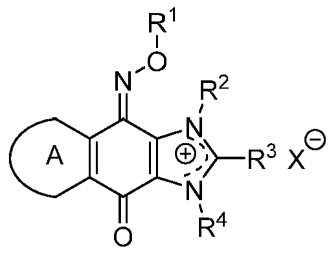

(II)

In the formula, the symbols denote the following meanings:

a ring A is selected from an aromatic ring having zero or one substituent, or a heteroaromatic ring having zero or one substituent;
$R^1$, $R^2$, $R^3$, and $R^4$ are the same or different, and each represent -hydrogen atom, -lower linear/branched alkyl having zero or one substituent, -lower alkenyl having zero or one substituent, -lower alkynyl having zero or one substituent, -saturated heterocyclyl having zero or one substituent, -cycloalkyl having zero or one substituent, -cycloalkenyl having zero or one substituent, -aryl having zero or one substituent, and -heteroaryl having zero or one substituent; and the substituents on the above ring A as well as in the $R^1$, the $R^2$, the $R^3$, and the $R^4$ are selected from a radical in group B;
group B: -lower linear/branched alkyl having zero or one substituent, -lower alkenyl having zero or one substituent, -lower alkynyl having zero or one substituent, -saturated heterocyclyl having zero or one substituent, -cycloalkyl having zero or one substituent, -cycloalkenyl having zero or one substituent, -aryl having zero or one substituent, -heteroaryl having zero or one substituent, $-OR^a$, $-SR^a$, -O-lower alkylene-$OR^a$, -O-lower alkylene-O-lower alkylene-$OR^a$, -O-lower alkylene-O-lower alkylene-O-lower alkylene-$OR^a$, -O-lower alkylene-$NR^aR^b$, -O-lower alkylene-O-lower alkylene-$NR^aR^b$, -O-lower alkylene-$NR^c$-lower alkylene-$NR^aR^b$, -O-CO-$NR^aR^b$, $-SOR^a$, $-SO_2R^a$, $-SO_2NR^aR^b$, $-NR^a-SO_2R^b$, $-NR^aR^b$, $-NR^c$-lower alkylene-$NR^aR^b$, -N(-lower alkylene-$NR^aR^b)_2$, $-NO_2$, -CN, -halogen, $-CO_2R^a$, $-COO^-$, $-CONR^aR^b$, $-CONR^a-O-R^b$, $-NR^a-COR^b$, $-NR^a-CO-NR^bR^c$, $-OCOR^a$, and $-COR^a$;
$R^a$, $R^b$, and $R^c$ are the same or different, and each represent -hydrogen atom, -lower linear/branched alkyl having zero or one substituent, -lower alkenyl having zero or one substituent, -lower alkynyl having zero or one substituent, -saturated heterocyclyl having zero or one substituent, -cycloalkyl having zero or one substituent, -cycloalkenyl having zero or one substituent, -aryl having zero or one substituent. -heteroaryl having zero or one substituent, -lower alkylene-(5 to 7-membered saturated heterocycle having zero or one substituent), -lower alkylene-(cycloalkyl having zero or one substituent), -lower alkylene-(aryl having zero or one substituent), and -lower alkylene-(heteroaryl having zero or one substituent), wherein the substituents refer to lower alkyl or heteroalkyl;
$X^-$ is a coanion including a halide ion, a sulfonate ion (methane sulfonate ion, trifluoromethanesulfonate ion, phenylsulfonate ion, p-toluene sulfonate, etc.), an acetate ion, a trifluoroacetate ion, a carbonate ion, a sulfate ion.; and when an anion on a substituent and an imidazolium cation form an inner salt, $X^-$ is absent.

As an embodiment of the present invention, in the structural formula,

the ring A is selected from an aromatic ring having zero, one or more substituents, and the substituent is $NO_2$;
the $R^1$ is selected from -hydrogen atom, -lower linear/branched alkyl having zero or one substituent, -lower alkenyl having zero or one substituent, -lower alkynyl having zero or one substituent, and -aryl having zero or one substituent;
the substituent is selected from -cycloalkyl, -aryl, $-OR^a$, -O-lower linear/branched alkyl-$OR^a$, -O-lower linear/branched alkyl-O-lower linear/branched alkyl-$OR^a$, -aryl substituted by $-OR^a$, -aryl substituted by halogen, -aryl substituted by $-CF_3$, -aryl substituted by $-NO_2$, and $-NH_2$, where $R^a$ is -hydrogen atom, -aryl, -lower linear/branched alkyl, or lower linear/branched alkyl substituted by aryl;
the $R^2$ is selected from lower linear/branched alkyl having zero or one substituent;
the $R^3$ is selected from -hydrogen atom, -lower linear/branched alkyl having zero or one substituent, -cycloalkyl, -aryl, heteroaryl, -aryl substituted by lower linear/branched alkyl, -aryl substituted by $-OR^a$ and/or halogen, -heteroaryl, and -heteroaryl substituted by lower linear/branched alkyl;
the substituent is -heteroaryl, -halogen, and -heteroaryl substituted by lower linear/branched alkyl; $R^a$ is -lower linear/branched alkyl;
the $R^4$ is selected from -hydrogen atom, -lower linear/branched alkyl having zero or one substituent, -lower alkenyl having zero or one substituent, -lower alkynyl having zero or one substituent, cycloalkyl, and -aryl having zero or one substituent;
the substituent is -CN, lower linear/branched alkyl, halogen, $OR^a$-substituted aryl, $OR^a$, heteroaryl, aryl, halogen-

substituted aryl, $-CO_2R^a$, saturated heterocyclyl, and -O-lower linear/branched alkyl-$OR^a$; $R^a$ is lower linear/branched alkyl.

As a further embodiment of the present invention, in the structural formula,

the ring A is selected from a benzene ring, a naphthalene ring, or a $NO_2$-substituted benzene ring;
the $R^1$ is selected from -hydrogen atom, -lower alkyl, -lower alkynyl, -lower alkenyl, -lower linear/branched alkyl-O-lower linear/branched alkyl, -lower linear/branched alkyl-O-aryl, -lower linear/branched alkyl-O-lower linear/-branched alkyl-O-lower linear/branched alkyl, -lower linear/branched alkyl-O-lower linear/branched alkyl-O-lower linear/branched alkyl-O-lower linear/branched alkyl, -aryl, -(5 to 7-membered) saturated heterocycle, and -heteroaryl;
the $R^2$ is selected from lower linear/branched alkyl;
the $R^3$ is selected from -hydrogen atom, -lower linear/branched alkyl, -(3 to 7-membered) saturated cycloalkyl, -heterocyclyl, -aryl, -aryl substituted by lower linear/branched alkyl, -aryl substituted by halogen, -heteroaryl, -heteroaryl substituted by lower linear/branched alkyl, -O-lower linear/branched alkyl, -S-lower linear/branched alkyl, -acyl, and acyl substituted by lower alkyl; and
the $R^4$ is selected from -hydrogen atom, -lower linear/branched alkyl, -lower alkenyl, -lower alkynyl, -aryl, -CN, -(3 to 7-membered) saturated cycloalkyl, -aryl substituted by halogen, -aryl substituted by lower alkyl, -heteroaryl, $-CO_2R^a$, -saturated heterocyclyl, -lower linear/branched alkyl-$OR^a$, -lower linear/branched alkyl-O-lower linear/branched alkyl, -lower linear/branched alkyl-S-lower linear/branched alkyl, -lower linear/branched alkyl-O-lower linear/-branched alkyl-$OR^a$, where the $R^a$ is -lower linear/branched alkyl.

As a further embodiment of the present invention, in the structural formula,

the ring A is selected from a benzene ring, a naphthalene ring, or a $NO_2$-substituted benzene ring;
$R^1$ is selected from -hydrogen atom, -(C1 to C6) linear alkyl or branched alkyl, -benzyl, -(C4 to C6) methylcycloalkyl, -(C3 to C6) alkenyl, $-CH_2(C_6H_{5-m}-X_m)$ (m=1-5, X is a halogen atom, $NO_2$, a hydrogen atom or $-OC_nH_{2n+1}$, n=1-6), $-CH_2(C_6H_4-CH_nX_{3-n})$ (n=1-3, X is a halogen atom), $-(C_nH_{2n}-O)_y-C_mH_{2m+1}$ (n=1-6, m=1-6, y=1-3), $-C_nH_{2n}-O-C_mH_{2m}-Ph$ (n=1-6, m=1-6), $-C_nH_{2n}-NH_2$(n=1-6) or $-CH_2CH(-OCH_2CH_2O-)$;
the $R^2$ is selected from lower linear/branched alkyl;
the $R^3$ is selected from -hydrogen atom, -(C1 to C6) linear alkyl or branched alkyl, -(C4 to C6) methylcycloalkyl, -heteroaryl, -phenyl, or substituted phenyl;
the $R^4$ is selected from -(C1 to C6) linear alkyl or branched alkyl, -(C3 to C6) methylcycloalkyl, -lower alkylene-0-lower alkyl, -(C3 to C6) alkenyl, -benzyl or substituted benzyl, an -ester group or a substituted ester group, -phenyl or substituted phenyl, $-CH_2$(thienyl) or $-CH_2CH(-OCH_2CH_2O-)$.

As a further embodiment of the present invention, in the structural formula,

the ring A is selected from a benzene ring, a naphthalene ring, or a $NO_2$-substituted benzene ring;
$R^1$ is selected from -hydrogen atom, -(C1 to C6) linear alkyl or branched alkyl, -benzyl, -(C4 to C6) methylcycloalkyl, -(C3 to C6) alkenyl, $-CH_2(C_6H_{5-m}-X_m)$ (m=1-5, X is a halogen atom, $NO_2$, a hydrogen atom or $-OC_nH_{2n+1}$, n=1-6), $-CH_2(C_6H_4-CH_nX_{3-n})$ (n=1-3, X is a halogen atom), $-(C_nH_{2n}-O)_y-C_mH_{2m+1}$ (n=1-6, m=1-6, y=1-3), $-C_nH_{2n}-O-C_mH_{2m}-Ph$ (n=1-6, m=1-6), $-C_nH_{2n}-NH_2$(n=1-6) or $-CH_2CH(-OCH_2CH_2O-)$;
the $R^2$ is selected from lower linear/branched alkyl;
the $R^3$ is selected from -hydrogen atom, -methyl, -phenyl, or substituted phenyl;
the $R^4$ is selected from -(C1 to C6) linear alkyl or branched alkyl; and
X is selected from Br, I, Oms, and OTs.

As a further embodiment of the present invention, the derivative includes:

(E)-or (Z)-4-(hydroxylimino)-1,3-dimethyl-9-oxo-4,9-dihydro-1H-naphtol[2,3-d]imidazole-3-oniu m, (E)-or (Z)-1-ethyl-4-(hydroxylimino)-3-methyl-9-oxo-4,9-dihydro-1H-naphthol[2,3-d] imidazol-3-onium, (E)-or (Z)-1-propyl-4-(hydroxylimino)-3-methyl-9-oxo-4,9-dihydro-1H-naphthol[2,3-d] imidazol-3-onium, (E)-or (Z)-1-butyl-4-(hydroxylimino)-3-methyl-9-oxo-4,9-dihydro-1H-naphthol[2,3-d]imidazole-3 -onium, (E)-or (Z)-1-isopropyl-4-(hydroxylimino)-3-methyl-9-oxo-4,9-dihydro-1H-naphthol[2,3-d]imidaz ole-3-onium, (E)-or (Z)-1-isobutyl-4-(hydroxylimino)-3-methyl-9-oxo-4,9-dihydro-1H-naphthol[2,3-d]imidazol e-3-onium, (E)-or (Z)-1-(2-methylbutyl)-4-(hydroxylimino)-3-methyl-9-oxo-4,9-dihydro-1H-naphthol[2,3-d]i midazole-3-onium, (E)-or (Z)-1-tert-butyl-4-(hydroxylimino)-3-methyl-9-oxo-4,9-dihydro-1H-naphthol[2,3-d]imidaz ole-3-onium, (E)-or (Z)-1-cyclopropyl-4-(hydroxylimino)-3-methyl-9-oxo-4,9-dihydro-1H-naphthol[2,3-d]imid azole-3-onium, (E)-or (Z)-1-(2-methoxyethyl)-4-(hydroxylimino)-3-methyl-9-oxo-4,9-dihydro-1H-naphthol[2,3-d] imidazole-3-onium, (E)-or (Z)-1-(3-methyl-2-ene-1-yl)-4-(hydroxylimino)-3-methyl-9-oxo-4,9-dihydro-1H-naphthol[ 2,3-d]imidazole-3-onium, (E)-or (Z)-1-benzyl-4-(hydroxylimino)-3-methyl-9-oxo-4,9-dihydro-1H-naphthol[2,3-d]imidazole -3-onium, (E)-or (Z)-1-(2-ethoxy-2-oxyethyl)-4-(hydroxylimino)-3-methyl-9-oxo-4,9-dihydro-1H-naphthol[ 2,3-d]imidazole-3-onium, (E)-or (Z)-1-phenyl-4-(hydroxylimino)-3-methyl-9-oxo-4,9-dihydro-1H-naphthol[2,3-d]imidazole -3-onium, (E)-or (Z)-1-(2,4,6-trimethylphenyl)-4-(hydroxylimino)-3-methyl-9-oxo-4,9-dihydro-1H-naphthol [2,3-d]imidazole-3-onium, (E)-or (Z)-1-(thiophene-2-methyl)-4-(hydroxylimino)-3-methyl-9-oxo-4,9-dihydro-1H-naphthol[2, 3-d]imidazole-3-onium, (E)-or (Z)-1-((1,3-dioxoheterocycle-2-yl)methyl)-4-(hydroxylimino)-3-methyl-9-oxo-4,9-dihydro-1H-naphthol[2,3-d]imidazole-3-onium, (E)-or (Z)-1-(4-fluorobenzyl)-4-(hydroxylimino)-3-methyl-9-oxo-4,9-dihydro-1H-naphthol[2,3-d] imidazole-3-onium, (E)-or (Z)-1-(2-fluorophenyl)-4-(hydroxylimino)-3-methyl-9-oxo-4,9-dihydro-1H-naphthol[2,3-d] imidazole-3-onium, (E)-or (Z)-1-(3-fluorophenyl)-4-(hydroxylimino)-3-methyl-9-oxo-4,9-dihydro-1H-naphthol[2,3-d] imidazole-3-onium, (E)-or (Z)-1-(4-fluorophenyl)-4-(hydroxylimino)-3-methyl-9-oxo-4,9-dihydro-1H-naphthol[2,3-d] imidazole-3-onium, (E)-or (Z)-1-(2-fluorobenzyl)-4-(hydroxylimino)-3-methyl-9-oxo-4,9-dihydro-1H-naphthol[2,3-d] imidazole-3-onium, (E)-or (Z)-1-(3-fluorobenzyl)-4-(hydroxylimino)-3-methyl-9-oxo-4,9-dihydro-1H-naphthol[2,3-d] imidazole-3-onium, (E)-or (Z)-1-(4-methoxyphenyl)-4-(hydroxylimino)-3-methyl-9-oxo-4,9-dihydro-1H-naphthol[2,3 -d]imidazole-3-onium, (E)-or (Z)-1-(4-methoxybenzyl)-4-(hydroxylimino)-3-methyl-9-oxo-4,9-dihydro-1H-naphthol [2,3-d]imidazole-3-onium, (E)-or (Z)-1-(3-chloro-4-fluorophenyl)-4-(hydroxylimino)-3-methyl-9-oxo-4,9-dihydro-1H-napht hol[2,3-d]imidazole-3-onium, (E)-or (Z)-1-(3-bromo-4-fluorophenyl)-4-(hydroxylimino)-3-methyl-9-oxo-4,9-dihydro-1H-napht hol[2,3-d]imidazole-3-onium, (E)-or (Z)-1-butyl-4-(methoxyimino)-3-methyl-9-oxo-4,9-dihydro-1H-naphthol[2,3-d]imidazoliu m, (E)-or (Z)-1-butyl-4-(ethoxyimino)-3-methyl-9-oxo-4,9-dihydro-1H-naphthol [2,3-d]imidazolium,

(E)-or

(Z)-1-butyl-4-(benzyloxyimino)-3-methyl-9-oxo-4,9-dihydro-1H-naphthol[2,3-d]imidazoli um, (E)-or (Z)-1-butyl-4-(methoxyimino)-3-methyl-9-oxo-4,9-dihydro-1H-naphthol[2,3-d]imidazoliu m, (E)-or (Z)-1-butyl-4-(methoxyimino)-3-ethyl-9-oxo-4,9-dihydro-1H-naphthol[2,3-d]imidazolium,

(E)-or

(Z)-4-(methoxyimino)-1,3-dimethyl-9-oxo-4,9-dihydro-1H-naphthol[2,3-d]imidazolium,

(E)-or

(Z)-4-(ethoxyimino)-1,3-dimethyl-9-oxo-4,9-dihydro-1H-naphthol[2,3-d]imidazolium,

(E)-or

(Z)-4-(benzyloxyimino)-1,3-dimethyl-9-oxo-4,9-dihydro-1H-naphthol[2,3-d]imidazolium,

(E)-or

(Z)-3-ethyl-4-(methoxyimino)-1-methyl-9-oxo-4,9-dihydro-1H-naphthol[2,3-d]imidazoliu m, (E)-or (Z)-4-(isopropoxyimino)-1,3-dimethyl-9-oxo-4,9-dihydro-1H-naphthol[2,3-d]imidazolium,

(E)-or

(Z)-4-(butoxyimino)-1,3-dimethyl-9-oxo-4,9-dihydro-1H-naphthol[2,3-d]imidazolium,

(E)-or

(Z)-4-(isobutoxyimino)-1,3-dimethyl-9-oxo-4,9-dihydro-1H-naphthol[2,3-d]imidazolium,

(E)-or

(Z)-4-(tert-butoxyimino)-1,3-dimethyl-9-oxo-4,9-dihydro-1H-naphthol[2,3-d]imidazolium,

(E)-or

(Z)-4-(cyclopropylmethoxyimino)-1,3-dimethyl-9-oxo-4,9-dihydro-1H-naphthol[2,3-d]imi dazolium, (E)-or (Z)-4-(allyloxy)imino)-1,3-dimethyl-9-oxo-4,9-dihydro-1H-naphthol[2,3-d]imidazolium,

(E)-or

(Z)-4-((4-methoxybenzyloxy)imino)-1,3-dimethyl-9-oxo-4,9-dihydro-1H-naphthol[2,3-d]i midazolium, (E)-or (Z)-4-((4-benzyloxy)imino)-1,3-dimethyl-9-oxo-4,9-dihydro-1H-naphthol[2,3-d]imidazoliu m, (E)-or (Z)-4-((4-trifluoromethylbenzyloxy)imino)-1,3-dimethyl-9-oxo-4,9-dihydro-1H-naphthol[2, 3-d]imidazolium, (E)-or (Z)-4-((4-fluorobenzyloxy)imino)-1,3-dimethyl-9-oxo-4,9-dihydro-1H-naphthol[2,3-d]imid azolium, (E)-or (Z)-4-(perfluorobenzyloxyimino)-1,3-dimethyl-9-oxo-4,9-dihydro-1H-naphthol[2,3-d]imid azolium, (E)-or (Z)-4-((4-nitrobenzyloxy)imino)-1,3-dimethyl-9-oxo-4,9-dihydro-1H-naphthol[2,3-d]imida zolium, (E)-or

(Z)-4-((2-methoxyethoxy)imino)-1,3-dimethyl-9-oxo-4,9-dihydro-1H-naphthol[2,3-d]imida zolium, (E)-or (Z)-4-((2-(benzyloxy)ethoxy)imino)-1,3-dimethyl-9-oxo-4,9-dihydro-1H-naphthol[2,3-d]i midazolium, (E)-or (Z)-4-((2-(2-methoxyethoxy)ethoxy)imino)-1,3-dimethyl-9-oxo-4,9-dihydro-1H-naphthol[2, 3-d]imidazolium, (E)-or (Z)-4-((2-(2-(2-methoxyethoxy)ethoxy)ethoxy)imino)-1,3-dimethyl-9-oxo-4,9-dihydro-1H-naphthol[2,3-d]imidazolium, and (E)-or (Z)-4-((benzyloxy)imino)-1-butyl-3-methyl-9-oxo-2-(3,4,5-trimethoxyphenyl)-4,9-dihydro-1H-naphthol[2,3-d]imidazolium, etc.

In a second aspect, the present invention further relates to a pharmaceutical composition, including the one or more quinonoxime imidazolium derivatives above, and a pharmaceutically acceptable carrier.

In a third aspect, the present invention further relates to use of the pharmaceutical composition above in preparing an anti-cancer, anti-bacterial, or antiviral drug.

In a fourth aspect, the present invention further relates to a (Z) or (E)-fused quinonoxime imidazole derivative represented by the following Formula (I) and (II) and a hydrochloride thereof;
structural formulas are shown in:

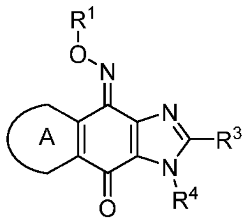 (III) and 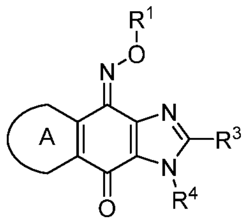 (IV)

In the structural formula,

the ring A is selected from a -benzene ring, a -naphthalene ring, or a $NO_2$-substituted benzene ring;
the $R^1$ is selected from -hydrogen atom, -lower alkyl, -lower alkynyl, -lower alkenyl, -lower linear/branched alkyl-O-lower linear/branched alkyl, -lower linear/branched alkyl-O-aryl, -lower linear/branched alkyl-O-lower linear/-branched alkyl-O-lower linear/branched alkyl, -lower linear/branched alkyl-O-lower linear/branched alkyl-O-lower linear/branched alkyl-O-lower linear/branched alkyl, -aryl, -(5 to 7-membered) saturated heterocycle, and -heteroaryl;
the $R^3$ is selected from -hydrogen atom, -lower linear/branched alkyl, -(3 to 7-membered) saturated cycloalkyl, -heterocyclyl, -aryl, -aryl substituted by lower linear/branched alkyl, -aryl substituted by halogen, -heteroaryl, -heteroaryl substituted by lower linear/branched alkyl, -O-lower linear/branched alkyl, -S-lower linear/branched alkyl, -acyl, and acyl substituted by lower alkyl; and
the $R^4$ is selected from -hydrogen atom, -lower linear/branched alkyl, -lower alkenyl, -lower alkynyl, -aryl, -CN, -(3 to 7-membered) saturated cycloalkyl, -aryl substituted by halogen, -aryl substituted by lower alkyl, -heteroaryl, -$CO_2R^a$, -saturated heterocyclyl, -lower linear/branched alkyl-$OR^a$, -lower linear/branched alkyl-O-lower linear/branched alkyl, -lower linear/branched alkyl-S-lower linear/branched alkyl, -lower linear/branched alkyl-O-lower linear/-branched alkyl-$OR^a$, wherein the $R^a$ is lower linear/branched alkyl.

As a further embodiment of the present invention, the derivative includes (E)-9-(hydroxylimino)-3-methyl-3,9-dihydro-4H-naphthol [2,3-d] imidazole-4-one, (E)-3-ethyl-9-(hydroxylimino)-3,9-dihydro-4H-naphthol [2,3-d] imidazole-4-one, (E)-3-propyl-9-(hydroxylimino)-3,9-dihydro-4H-naphthol [2,3-d] imidazole-4-one, (E)-3-butyl-9-(hydroxylimino)-3,9-dihydro-4H-naphthol [2,3-d] imidazole-4-one, (E)-3-isopropyl-9-(hydroxylimino)-3,9-dihydro-4H-naphthol [2,3-d] imidazole-4-one, (E)-3-isobutyl-9-(hydroxylimino)-3,9-dihydro-4H-naphthol [2,3-d] imidazole-4-one, (E)-9-(hydroxylimino)-3-(2-methylbutyl)-3,9-dihydro-4H-naphthol [2,3-d] imidazole-4-one, (E)-9-(hydroxylimino)-3-(4-methoxybenzyl)-3,9-dihydro-4H-naphthol [2,3-d] imidazole-4-one, (E)-9-(hydroxylimino)-3-(2-methoxyethyl)-3,9-dihydro-4H-naphthol [2,3-d] imidazole-4-one, (E)-9-(hydroxylimino)-3-(3-methyl-2-ene-1-yl)-3,9-dihydro-4H-naphthol [2,3-d] imidazole-4-one, (E)-9-(hydroxylimino)-3-(propyl-2-yne-1-yl)-3,9-dihydro-4H-naphthol [2,3-d] imidazole-4-one, (E)-9-(hydroxylimino)-3-(thiophen-2-yl-methyl)-3,9-dihydro-4H-naphthol [2,3-d] imidazole-4-one, (E)-9-(hydroxylimino)-3-benzyl-3,9-dihydro-4H-naphthol [2,3-d] imidazole-4-one, (E)-9-(hydroxylimino)-3-(2-fluorobenzyl)-3,9-dihydro-4H-naphthol [2,3-d] imidazole-4-one, (E)-9-(hydroxylimino)-3-(3-fluorobenzyl)-3,9-dihydro-4H-naphthol [2,3-d] imidazole-4-one, (E)-9-(hydroxylimino)-3-(4-fluorobenzyl)-3,9-dihydro-4H-naphthol [2,3-d] imidazole-4-one, (E)-2-(4-hydroxylimino)-9-oxo-4,9-dihydro-1H-naphthol [2,3-d] imidazole-1-yl) acetate, (E)-3-((1,3-dioxoheterocycle-2-yl)methyl)-9-(hydroxylimino)-3,9-dihydro-4H-naphthol [2,3-d] imidazole-4-one, (E)-11-(hydroxylimino)-3-methyl-3,11-dihydro-4H-anthraquinone [2,3-d] imidazole-4-one, (E)-11-(hydroxylimino)-3-ethyl-3,11-dihydro-4H-anthraquinone [2,3-d] imidazole-4-one, (E)-3-cyclopropyl-9-(hydroxylimino)-3,9-dihydro-4H-naphthol [2,3-d] imidazole-4-one, (E)-3-tert-butyl-9-(hydroxylimino)-3,9-dihydro-4H-naphthol [2,3-d] imidazole-4-one, (E)-9-(hydroxylimino)-3-(pyrazine-2-yl-methyl)-3,9-dihydro-4H-naphthol [2,3-d] imidazole-4-one, (E)-9-(hydroxylimino)-3-benzyl-3,9-dihydro-4H-naphthol [2,3-d] imidazole-4-one, (E)-9-(hydroxylimino)-3-(2,4,6-trimethylphenyl)-3,9-dihydro-4H-naphthol [2,3-d] imidazole-4-one, (E)-9-(hydroxylimino)-3-(2-fluorophenyl)-3,9-dihydro-4H-naphthol [2,3-d] imidazole-4-one, (E)-9-(hydroxylimino)-3-(3-fluorophenyl)-3,9-dihydro-4H-naphthol [2,3-d] imidazole-4-one, (E)-9-(hydroxylimino)-3-(4-fluorophenyl)-3,9-dihydro-4H-naphthol [2,3-d] imidazole-4-one, (E)-9-(hydroxylimino)-3-(4-methoxyphenyl)-3,9-dihydro-4H-naphthol [2,3-d] imidazole-4-one, (E)-9-(hydroxylimino)-3-(3-chloro-4-fluorophenyl)-3,9-dihydro-4H-naphthol [2,3-d] imidazole-4-one, (E)-9-(hydroxylimino)-3-(3-bromo-4-fluorophenyl)-3,9-dihydro-4H-naphthol [2,3-d] imidazole-4-one, (E)-9-(hydroxylimino)-3-methyl-8-nitro-3,9-dihydro-4H-naphthol [2,3-d] imidazole-4-one, (E)-9-(hydroxylimino)-2,3-dimethyl-3,9-dihydro-4H-naphthol [2,3-d] imidazole-4-one, (E)-3-butyl-9-(hydroxylimino)-2-methyl-3,9-dihydro-4H-naphthol [2,3-d] imidazole-4-one, (E)-11-(hydroxylimino)-2,3-dimethyl-3,11-dihydro-4H-anthraquinone [2,3-d] imidazole-4-one, (E)-3-ethyl-11-(hydroxylimino)-2-methyl-3,11-dihydro-4H-anthraquinone [2,3-d] imidazole-4-one, (E)-3-isobutyl-11-(hydroxylimino)-2-methyl-3,11-dihydro-4H-anthraquinone [2,3-d] imidazole-4-one, (E)-11-(hydroxylimino)-2-methyl-3-(3-methyl-2-ene-1-yl)-3,11-dihydro-4H-anthraquinone [2,3-d] imidazole-4-one, (E)-2-(4-(hydroxylimino)-2-methyl-11-oxo-4,11-dihydro-1H-anthracene [2,3-d] imidazole-1-yl) acetonitrile, (E)-3-benzyl-11-(hydroxylimino)-2-methyl-3,11-dihydro-4H-anthraquinone [2,3-d] imidazole-4-one, (E)-3-(4-fluorobenzyl)-11-(hydroxylimino)-2-methyl-3,11-dihydro-4H-anthraquinone [2,3-d] imidazole-4-one, (E)-3-butyl-9-(hydroxylimino)-2-ethyl-3,9-dihydro-4H-naphthol [2,3-d] imidazole-4-one, (E)-3-butyl-9-(hydroxylimino)-2-propyl-3,9-dihydro-4H-naphthol [2,3-d] imidazole-4-one, (E)-2-(sec-butyl)-3-butyl-9-(hydroxylimino)-3,9-dihydro-4H-naphthol [2,3-d] imidazole-4-one, (E)-3-butyl-9-(hydroxylimino)-2-isobutyl-3,9-dihydro-4H-naphthol [2,3-d] imidazole-4-one, (E)-3-butyl-9-(hydroxylimino)-2-(pentane-3-yl)-3,9-dihydro-4H-naphthol [2,3-d] imidazole-4-one, (E)-3-butyl-9-(hydroxylimino)-2-tert-butyl-3,9-dihydro-4H-naphthol [2,3-d] imidazole-4-one, (E)-3-butyl-9-(hydroxylimino)-2-(2-(5-methylfuran-2-yl)-ethyl)-3,9-dihydro-4H-naphthol [2,3-d] imidazole-4-one, (E)-3-butyl-9-(hydroxylimino)-2-cyclobutyl-3,9-dihydro-4H-naphthol [2,3-d] imidazole-4-one, (E)-3-butyl-9-(hydroxylimino)-2-phenyl-3,9-dihydro-4H-naphthol [2,3-d] imidazole-4-one, (E)-3-butyl-9-(hydroxylimino)-2-(4-isopropylphenyl)-3,9-dihydro-4H-naphthol [2,3-d] imidazole-4-one, (E)-3-butyl-9-(hydroxylimino)-2-(3,4,5-trimethoxyphenyl)-3,9-dihydro-4H-naphthol [2,3-d] imidazole-4-one, (E)-3-butyl-2-(3-chloro-4-methoxyphenyl)-9-(hydroxylimino)-3,9-dihydro-4H-naphthol [2,3-d] imidazole-4-one, (E)-3 -butyl-9-(hydroxylimino)-2-naphthyl-3,9-dihydro-4H-naphthol [2,3-d] imidazole-4-one, (E)-3-butyl-9-(hydroxylimino)-2-(trifluoromethyl)-3,9-dihydro-4H-naphthol [2,3-d] imidazole-4-one, (E)-3-butyl-9-(hydroxylimino)-2-(furan-2-yl)-3,9-dihydro-4H-naphthol [2,3-d] imidazole-4-one, (E)-3-butyl-9-(hydroxylimino)-2-(thiophene-2-yl)-3,9-dihydro-4H-naphthol [2,3-d] imidazole-4-one, (E)-2-ethyl-11-(hydroxylimino)-3-methyl-3,11-dihydro-4H-anthraquinone [2,3-d] imidazole-4-one, (E)-2-(furan-2-yl)-11-(hydroxylimino)-3-methyl-3,11-dihydro-4H-anthraquinone [2,3-d] imidazole-4-one, (E) and (Z)-9-(methoxyimino)-3-methyl-3,9-dihydro-4H-naphthol [2,3-d] imidazole-4-one, (E) and (Z)-9-(ethoxyimino)-3-methyl-3,9-dihydro-4H-naphthol [2,3-d] imidazole-4-one, (E) and (Z)-9-(benzyloxyimino)-3-methyl-3,9-dihydro-4H-naphthol [2,3-d] imidazole-4-one, (E) and (Z)-9-(isopropoxyimino)-3-methyl-3,9-dihydro-4H-naphthol [2,3-d] imidazole-4-one, (E) and (Z)-9-(butoxyimino)-3-methyl-3,9-dihydro-4H-naphthol [2,3-d] imidazole-4-one, (E) and (Z)-9-(isobutoxyimino)-3-methyl-3,9-dihydro-4H-naphthol [2,3-d] imidazole-4-one, (E) and (Z)-9-(tert-butoxyimino)-3-methyl-3,9-dihydro-4H-naphthol [2,3-d] imidazole-4-one, (E) and (Z)-9-((cyclopropoxy)imino)-3-methyl-3,9-dihydro-4H-naphthol [2,3-d] imidazole-4-one, (E) and (Z)-9-((allyloxy)imino)-3-methyl-3,9-dihydro-4H-naphthol [2,3-d] imidazole-4-one, (E) and (Z)-3-methyl-9-(((4-methoxybenzyl)oxy)imino)-3,9-dihydro-4H-naphthol [2,3-d] imidazole-4-one, (E) and (Z)-3-methyl-9-(((4-chlorobenzyl)oxy)imino)-3,9-dihydro-4H-naphthol [2,3-d] imidazole-4-one, (E) and (Z)-3-methyl-9-(((4-trifluoromethylbenzyl)oxy)imino)-3,9-dihydro-4H-naphthol [2,3-d] imidazole-4-one, (E) and (Z)-3-methyl-9-(((4-fluorobenzyl)oxy)imino)-3,9-dihydro-4H-naphthol [2,3-d] imidazole-4-one, (E) and (Z)-3 -methyl-9-(((perfluorobenzyl)oxy)imino)-3,9-dihydro-4H-naphthol [2,3-d] imidazole-4-one, (E) and (Z)-3 -methyl-9-(((4-nitrobenzyl)oxy)imino)-3,9-dihydro-4H-naphthol [2,3-d] imidazole-4-one, (E) and (Z)-11-(benzyloxyimino)-3-methyl-3,11-dihydro-4H-anthraquinone [2,3-d] imidazole-4-one, (E) and (Z)-11-(butoxyimino)-3-methyl-3,11-dihydro-4H-anthraquinone [2,3-d] imidazole-4-one, (E) and (Z)-11-(tert-butoxyimino)-3-methyl-3,11-dihydro-4H-anthraquinone [2,3-d] imidazole-4-one, (E)-9-((2-methoxyethoxy)imino)-3-methyl-3,9-dihydro-4H-naphthol [2,3-d] imidazole-4-one, (E) and (Z)-9-((2-benzyloxy)ethoxy)imino)-3-methyl-3,9-dihydro-4H-naphthol [2,3-d] imidazole-4-one, (E)-9-((2-(2-methoxyethoxy)ethoxy)imino)-3-methyl-3,9-dihydro-4H-naphthol [2,3-d] imidazole-4-one, (E) and (Z)-9-((2-(2-(2-methoxyethoxy)ethoxy)ethoxy)imino)-3-methyl-3,9-dihydro-4H-naphthol [2,3-d] imidazole-4-one, (E)-9-((3-aminopropoxy)imino)-3-methyl-3,9-dihydro-4H-naphthol [2,3-d] imidazole-4-one, (E) and (Z)-3-butyl-9-(methoxyimino)-3,9-dihydro-4H-naphthol [2,3-d] imidazole-4-one, (E) and (Z)-3-butyl-9-(ethoxyimino)-3,9-dihydro-4H-naphthol [2,3-d] imidazole-4-one, (E) and (Z)-9-((benzyloxy)imino)-3-butyl-3,9-dihydro-4H-naphthol [2,3-d] imidazole-4-one, (E) and (Z)-11-((methoxy)imino)-3-(2-(2-methoxyethoxy)ethyl)-3,11-dihydro-4H-anthraquinone [2,3-d] imidazole-4-one, (E) and (Z)-9-((benzyloxy)imino)-2,3-dimethyl-3,9-dihydro-4H-naphthol [2,3-d] imidazole-4-one, (E) and (Z)-11-((benzyloxy)imino)-2,3-dimethyl-3,11-dihydro-4H-anthraquinone [2,3-d] imidazole-4-one, (E) and (Z)-9-((benzyloxy)imino)-3-butyl-2-(3,4,5-trimethoxyphenyl)-3,9-dihydro-4H-naphthol [2,3-d] imidazole-4-one, and (E) and (Z)-2-cyclopropyl-11-(methoxyimino)-3-methyl-3,11-dihydro-4H-anthraquinone [2,3-d] imidazole-4-one.

The (Z) or (E)-fused quinonoxime imidazole derivative represented by Formula (III) or (IV) and a hydrochloride thereof are synthetic intermediates of the above Formulas (I) and (II); and the synthetic intermediates have good antitumor activity. Therefore, in a fifth aspect, the present invention further relates to a pharmaceutical composition, including the compound or hydrochloride thereof, and a pharmaceutically acceptable carrier. The pharmaceutical composition has anticancer, antibacterial and antiviral effects.

In a fifth aspect, the present invention further relates to use of the above pharmaceutical composition above in preparing an anti-cancer, anti-bacterial, or antiviral drug.

Compounds of Formulas (I), (II), (III), and (IV) will be further described below.

Term "lower" refers to a linear or branched hydrocarbon having 1 to 6 carbon atoms. "Lower alkyl" generally refers to alkyl having 1 to 4 carbon atoms, particularly preferably, methyl, ethyl, propyl, isopropyl, butyl, and isobutyl. "Lower alkenyl" generally refers to vinyl, allyl, 1-propenyl, isopropenyl, 1-butenyl, 2-butenyl, 3-butenyl, and 3-methyl-2-butenyl. "Lower alkynyl" generally refers to acetenyl, 1-propinyl, 2-propinyl, 1-butynyl, 2-butynyl, 3-butynyl, and 1-methyl-2-propinyl. "Aryl" refers to aromatic radicals, more preferably, aryl having 6 and 14 carbon atoms, and most preferably, phenyl, naphthyl, and fluorenyl. In addition, ring A refers to a ring forming the above aryl, most preferably, phenyl and naphthyl. "Heteroaromatic ring" refers to 5-6 membered monocyclic heteroaryl having 1-4 heteroatoms selected from one or more of N, S, and O, and bicyclic heteroaryl obtained by fusing a benzene ring or 5-6 membered monocyclic heteroaryl therewith. In this case, the 5-6 membered monocyclic heteroaryl is preferably furyl, thienyl, pyrryl, imidazolyl, thiazolyl, pyrazolyl, isothiazolyl, oxazolyl, isoxazolyl, oxadiazolyl, thiadiazolyl, triazolyl, tetrazolyl, pyridyl, pyrimidyl, pyrazinyl, pyridazinyl and triazinyl; the bicyclic heteroaryl is preferably benzofuryl, benzothiophenyl, benzothiadiazolyl, benzothiazolyl, benzoxazolyl, benzoxadiazolyl, benzimidazolyl, indolyl, isoindolyl, indazolyl, quinolyl, isoquinolyl, quinazolinyl, quinoxalinyl, benzodioxoly, indolizinyl, imidazolpyridyl, and cinnolinyl. Some of the saturated heteroaryl groups are, for example, 1,2,3,4-tetrahydroquinolyl, preferably, furyl and thienyl.

"Lower heteroalkyl" refers to C1-C4 alkyl having 1 to 4 heteroatoms selected from one or more of N, S, and O.

"Cycloalkyl" refers to cycloalkyl group having 3 to 10 carbon atoms, particularly preferably, cyclopropyl, cyclobutyl, cyclopentyl, and cyclohexyl. "Cycloalkenyl" is preferably cycloalkenyl having 3 to 8 carbon atoms.

"Coanion" is not limited particularly as long as it is a pharmaceutically acceptable anion as a coanion against imidazolium cation, preferably, univalent or bivalent anion, e.g., halide ions, organic sulfonate ions, acetate ions, trifluoroacetate ions, carbonate ions, and sulfate ions, most preferably, halide ions.

"Heterocyclyl" refers to 5 to 7-membered monocyclic saturated heterocycle having 1-4 heteroatoms selected from N, S, and O, or a bridge ring thereof, preferably, tetrahydrofuryl, tetrahydrofuryl, pyrrolidyl, piperazinyl, azacycloheptyl, diazacycloheptyl, quinuclidinyl, piperidyl, and morpholinyl.

The compound of the present invention has tautomers of the following formula due to the non-localized effect of cation, and the present invention also includes isolated tautomers or a mixture thereof. Therefore, in the present invention, the compound represented by 1H-imidazole-3-onium derivative also includes a mixture of the tautomer 3H-imidazole-1-onium derivative and two isomers.

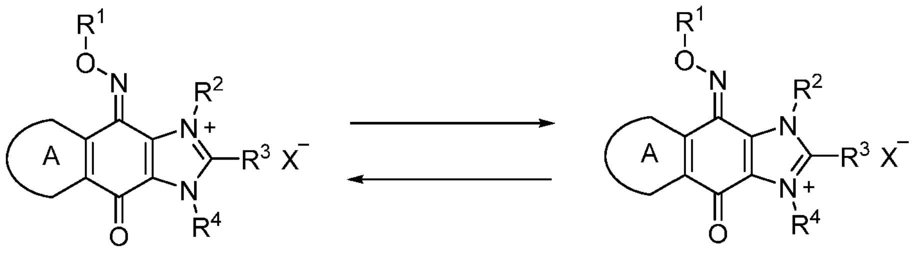

I I'

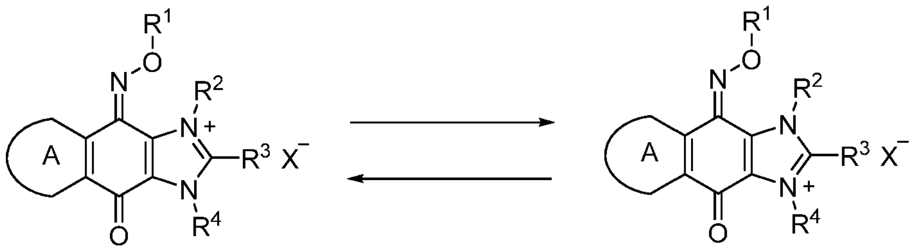

II II'

Additionally, hydrochlorides of Formulas (III) and (IV) in the present invention also fall within the present invention.

In some cases, the compounds (I), (II), (III), and (IV) of the present invention have geometrical isomers and tautomers according to different types of substituents. Isolated forms of these isomers or a mixture thereof are included in the present invention. Moreover, due to the existence of asymmetric carbon atoms in the compounds of the present invention, there are isomers to these asymmetric carbon atoms; the present invention includes mixed and isolated forms of these optical isomers. Moreover, in some cases, the compounds of the present invention form N-oxides due to different types of substituents; these substances are also included in the present invention; the present invention further includes

various hydrates, solvates, and polymorphic substances of the compounds (I), (II), (III), and (IV) herein.

In a sixth aspect, the present invention further relates to a method for preparing the preceding quinonoxime imidazolium derivative and synthetic intermediates thereof; the method includes the following steps:

A, serving a protic solvent as a solvent, and subjecting a substituted quinone imidazole derivative

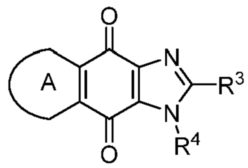

(V)

to an oximation reaction with $R^1ONH_2$•HCl under the catalytic action of a weak base or with corresponding $R^1ONH_2$ under the action of a weak-base hydrochloride, to obtain quinonoxime imidazole derivatives

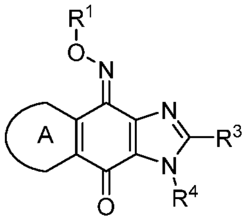

(III) and

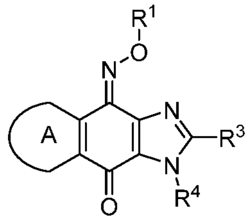

(IV);

and

B, performing N-alkylation reaction on the quinonoxime imidazole derivative (III) or (IV) and a corresponding halide reagent $R^2X$ in an organic solvent, to obtain a quinonoxime imidazolium derivative (I) or (II).

Preferably, in step A,
a molar ratio of the substituted naphthoquinone imidazole to the substituted oxamine hydrochloride R1ONH2•HCl, and to the weak base is 1:1.2-5:0.001-0.01.

Preferably, the substituted oxamine hydrochloride is selected from hydroxylamine hydrochloride, methoxyamine hydrochloride, phenoxyamine hydrochloride, and benzyloxyamine hydrochloride.

As an embodiment, in step A, the weak base is selected from potassium carbonate, triethylamine, pyridine, etc.

As an embodiment, in step A, the protic solvent is selected from methanol, ethanol, and isopropanol.

As an embodiment, in step A, reaction temperature is 90-125°C, and reaction time is 12-48 h.

As an embodiment, in step B, a molar ratio of the quinonoxime imidazole derivative (III) or (IV) to the halide reagent R2X is 1:1-1:100.

As an embodiment, in step B, the halohydrocarbon is selected from iodomethane, iodoethane, etc.

As an embodiment, in step B, the organic solvent is selected from acetonitrile, ethyl acetate, tetrahydrofuran, etc.

As an embodiment, in step B, the reaction temperature is 50-120°C, and reaction time is 8-48 h.

The compounds (III) and (IV) of the present invention may be used as a broad-spectrum anticancer, antibacterial and antiviral drug, and has low toxicity and good safety. Therefore, the compounds of the present invention have inhibition activity against proliferation of all solid tumors and lymphomas, in particular to breast cancer, cervical cancer, non-small cell lung cancer, rectal cancer, liver cancer, leukemia, prostate cancer, ovarian cancer, myeloma, esophageal cancer, pancreatic cancer, bladder cancer, melanoma, stomach cancer and other tumors.

The pharmaceutical composition of the present invention may be prepared by one or two compounds of Formulas (III) and (IV) above, and a conventional pharmaceutically acceptable carrier (a drug carrier, an excipient, etc.) in the art according to a conventional method. Administration may be conducted via any one of oral means of troches, pills, capsules, granules, powders, liquids, inhalants, etc. and para-oral means such as intravenous injection and intramuscular injection of injections, suppositories, eye drops, eye ointments, percutaneous liquids, ointments, percutaneous patches, transmucosal liquids, transmucosal patches, etc.

As solid compositions for oral administration of the present invention, troches, powders, granules, etc. are available. In these solid compositions, one or more active substances are mixed with at least one of inert excipients (e.g., lactose, mannitol, glucose, hydroxypropyl cellulose, microcrystalline cellulose, starch, polyvinylpyrrolidone, aluminum/-magnesium metasilicate, etc.). According to conventional steps, the compound may contain inert additives, e.g., lubricants (magnesium stearate, etc.), disintegrating agents (sodium carboxymethyl starch, etc.), and cosolvents. According to the need, troches or pills may be coated with icing, gastric-soluble or enteric soluble coating.

Liquid compositions for oral administration include pharmaceutically acceptable emulsions, liquids, suspending agents, syrups, elixirs, etc., and contain common inert solvents such as purified water or ethanol. Besides inert solvents, the composition may further contain a solubilizer, a wetting agent, a suspending agent, a sweetener, a flavoring agent, an aromatic, a preservative, and other additives.

Injections for para-oral administration contain sterile aqueous or aqueous solvents, suspending agents, and emulsions. Examples of aqueous solvents include distilled water and normal saline for injection. Insoluble agents include propylene glycol, polyethylene glycol, olive oil and other vegetable oil, ethanol and other alcohols, polysorbate 80, etc. These compositions may further contain isotonic agents, preservatives, wetting agents, emulsifiers, dispersants, stabilizers and co-solvents. These compositions are sterilized by, for example, filtering with a sterilizing membrane, mixing with a bactericide, or irradiation, etc. In addition, it may be prepared into sterile solid compositions may be further prepared, dissolved with sterile water or sterile solvent for injection or suspended for use.

## SPECIFIC IMPLEMENTATIONS

The following examples will help those skilled in the art further understand the present invention, but are not construed as limiting the present invention. It should be indicated that those skilled in the art may make further adjustments and improvements in the premise of not departing from the inventive concept herein. These all fall within the protection scope of the present invention. Compounds of the present invention are not limited to the compounds recited in the following examples at all. Experimental methods not marked with specific conditions in the following examples are generally subjected to conventional conditions, or conditions recommended by manufacturers. Moreover, the examples show the synthesis of raw materials of the compounds of the present invention.

## Example 1

2,3-Diamine-1,4-benzoquinone (20 g) was dissolved into 100 mL of formic acid, subjected to reflux reaction for 6 h, and cooled to room temperature, then poured into an ice-water mixture with the addition of ammonium hydroxide to regulate pH to 9-10, and the obtained solution was subjected to suction filtration and vacuum dried to obtain yellow solid 1,4-naphthoquinone imidazole (22 g). 1,4-naphthoquinone imidazole (0.3 g) and potassium hydroxide (0.15 g) were added to dimethyl sulfoxide solution (3 mL); the mixture was stirred at 50°C for 30 min and added with iodomethane (0.2 mL), after reaction for 2, water was added to separate out solid; the solid was subjected to suction filtration, washed with water, and vacuum dried to obtain yellow solid 2-methyl-1,4-naphthoquinone imidazole (0.31 g). 2-methyl-naphthoquinone imidazole (0.21 g), hydroxylamine hydrochloride (0.35 g), and pyridine (0.8 mL) were dissolved into ethanol (70 mL) and reacted for 48 h at 90°C; at the end of reaction, solid was separated out from solution, directly subjected to suction filtration, and vacuum dried to obtain light yellow solid (0.15 g), i.e., Compound 1.

Compound 1:

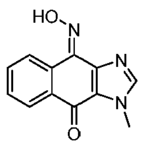

(E)-9-(hydroxylimino)-3-methyl-3,9-dihydro-4H-naphthol [2,3-d] imidazole-4-one

$^{1}$H NMR (400 MHz, DMSO-$d_6$) δ 13.01 (s, 1H), 8.26 (d, J = 0.8 Hz, 2H), 8.07 (dd, J = 8.0, 4.0 Hz, 1H), 7.72-7.64 (m, 1H), 7.61-7.52 (m, 1H), 4.00 (s, 3H).

$^{13}$C NMR (101 MHz, DMSO-$d_6$) δ 175.27, 145.22, 140.18, 139.62, 133.71, 133.06, 131.04, 129.58, 127.17, 126.12, 123.92, 34.14.

High-resolution mass spectrometry (electrospray ionization) (HRMS (ESI)): m/z theoretical value: $C_{12}H_9N_3O_2$ $[M+H]^+$ 228.0768, measured value: 228.0771.

1,4-Naphthoquinone imidazole was reacted with halohydrocarbons having different substitutes to prepare Compounds 2-20 according to the method in Example 1; other materials and reaction conditions for the compounds were kept the same and raw materials could be purchased commercially:

Compound 2:

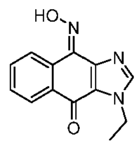

(E)-3-ethyl-9-(hydroxylimino)-3,9-dihydro-4H-naphthol [2,3-d] imidazole-4-one

$^{1}H$ NMR (400 MHz, DMSO-$d_6$) δ 13.04 (s, 1H), 8.36 (s, 1H), 8.26 (d, J = 8.0 Hz, 1H), 8.10 (d, J = 8.0 Hz, 1H), 7.72-7.64 (m, 1H), 7.60 (t, J = 7.2 Hz, 1H), 4.46 (q, J = 7.2 Hz, 2H), 1.39 (t, J = 8.0 Hz, 3H).

$^{13}C$ NMR (101 MHz, DMSO-$d_6$) δ 174.94, 144.31, 140.21, 140.08, 133.66, 133.06, 131.10, 129.59, 126.48, 126.22, 123.91, 42.17, 16.64.

HRMS (ESI): m/z theoretical value: $C_{13}H_{11}N_3O_2[M+H]^+$ 242.0924, measured value: 242.0927.

Compound 3:

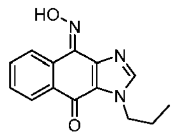

(E)-3-propyl-9-(hydroxyimino)-3,9-dihydro-4H-naphtho[2,3-d]imidazol-4-one

$^{1}H$ NMR (400 MHz, DMSO-$d_6$) δ 13.05 (s, 1H), 8.34 (s, 1H), 8.26 (d, J = 8.0 Hz, 1H), 8.09 (dd, J = 8.0, 1.0 Hz, 1H), 7.72-7.65 (m, 1H), 7.59 (td, J = 8.0, 1.0 Hz, 1H), 4.39 (t, J = 8.0 Hz, 2H), 1.85-1.72 (m, 2H), 0.83 (t, J = 7.4 Hz, 3H).

$^{13}C$ NMR (101 MHz, DMSO-$d_6$) δ 174.99, 144.84, 140.20, 140.05, 133.64, 133.05, 131.11, 129.58, 126.57, 126.21, 123.89, 48.30, 23.97, 11.02.

HRMS (ESI): m/z theoretical value: $C_{14}H_{13}N_3O_2[M+H]^+$ 256.1081, measured value: 256.1084.

Compound 4:

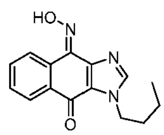

(E)-3-butyl-9-(hydroxyimino)-3,9-dihydro-4H-naphtho[2,3-d]imidazol-4-one

$^{1}H$ NMR (400 MHz, DMSO-$d_6$) δ 13.05 (s, 1H), 8.34 (s, 1H), 8.26 (dd, J = 8.0, 1.2 Hz, 1H), 8.09 (dd, J = 8.0, 1.2 Hz, 1H), 7.67 (td, J = 4.0, 1.6 Hz, 1H), 7.59 (td, J = 8.0, 1.2 Hz, 1H), 4.42 (t, J = 8.0 Hz, 2H), 1.81-1.66 (m, 2H), 1.30-1.19 (m, 2H), 0.86 (t, J = 8.0 Hz, 3H).

$^{13}C$ NMR (101 MHz, DMSO-$d_6$) δ 174.97, 144.77, 140.20, 140.03, 133.63, 133.04, 131.11, 129.57, 126.54, 126.22, 123.89, 46.55, 32.70, 19.48, 13.84.

HRMS (ESI): m/z theoretical value: $C_{15}H_{15}N_3O_2[M+H]^+$ 270.1237, measured value: 270.1240.

Compound 5:

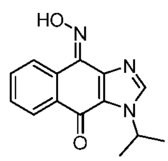

(E)-3-isopropyl-9-(hydroxylimino)-3,9-dihydro-4H-naphthol [2,3-d] imidazole-4-one

$^1H$ NMR (400 MHz, DMSO-$d_6$) δ 13.12 (s, 1H), 8.52 (s, 1H), 8.26 (d, J = 8.0 Hz, 1H), 8.11 (dd, J = 8.0, 1.6 Hz, 1H), 7.73-7.63 (m, 1H), 7.59 (td, J = 8.0, 1.2 Hz, 1H), 5.39-5.25 (m, 1H), 1.51 (d, J = 8.0 Hz, 6H).

$^{13}C$ NMR (101 MHz, DMSO-$d_6$) δ 174.95, 141.88, 140.23, 140.16, 133.33, 133.04, 131.27, 129.61, 126.36, 126.16, 123.78, 49.64, 23.06.

Compound 6:

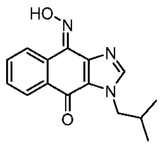

(E)-3-ethyl-isobutyl-9-(hydroxylimino)-3,9-dihydro-4H-naphthol [2,3-d] imidazole-4-one

$^1H$ NMR (400 MHz, DMSO-$d_6$) δ 13.05 (s, 1H), 8.33 (s, 1H), 8.27 (dd, J = 8.0, 1.2 Hz, 1H), 8.10 (dd, J = 8.0, 1.4 Hz, 1H), 7.68 (td, J = 8.0, 1.4 Hz, 1H), 7.59 (td, J = 8.0, 1.2 Hz, 1H), 4.26 (d, J = 8.0 Hz, 2H), 2.17-2.02 (m, 1H), 0.84 (d, J = 4.0 Hz, 6H).

$^{13}C$ NMR (101 MHz, DMSO-$d_6$) δ 175.08, 145.17, 140.19, 140.03, 133.64, 133.07, 131.13, 129.59, 126.69, 126.22, 123.90, 53.53, 29.52, 19.75.

HRMS (ESI): m/z theoretical value: $C_{15}H_{15}N_3O_2[M+H]^+$ 270.1237, measured value: 270.1237.

Compound 7:

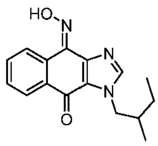

(E)-9-(hydroxylimino)-3-(2-methylbutyl)-3,9-dihydro-4H-naphthol [2,3-d] imidazole-4-one

$^1H$ NMR (400 MHz, DMSO-$d_6$) δ 13.05 (s, 1H), 8.33 (s, 1H), 8.27 (dd, J = 8.0, 1.2 Hz, 1H), 8.10 (dd, J = 8.0, 1.4 Hz, 1H), 7.71-7.64 (m, 1H), 7.60 (td, J = 8.0, 1.2 Hz, 1H), 4.43-4.35 (m, 1H), 4.24-4.16 (m, 1H), 1.99-1.83 (m, 1H), 1.37-1.20 (m, 1H), 1.17-1.07 (m, 1H), 0.85 (t, J = 8.0 Hz, 3H), 0.77 (d, J = 4.0 Hz, 3H).

$^{13}C$ NMR (101 MHz, DMSO-$d_6$) δ 175.08, 159.51, 145.25, 140.19, 140.05, 133.65, 133.08, 131.14, 129.60, 126.72, 126.25, 123.90, 52.24, 35.68, 26.51, 16.57, 11.31.

HRMS (ESI): m/z theoretical value: $C_{16}H_{17}N_3O_2[M+H]^+$ 284.1394, measured value: 284.1390.

Compound 8:

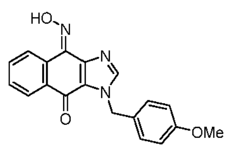

(E)-9-(hydroxylimino)-3-(4-methoxybenzyl)-3,9-dihydro-4H-naphthol [2,3-d] imidazole-4-one

$^1H$ NMR (400 MHz, DMSO-$d_6$) δ 13.03 (s, 1H), 8.48 (s, 1H), 8.27 (d, J = 8.0 Hz, 1H), 8.09 (dd, J = 8.0, 1.2 Hz, 1H), 7.68 (td, J = 8.0, 1.2 Hz, 1H), 7.62-7.55 (m, 1H), 7.28 (d, J = 8.0 Hz, 2H), 6.86 (d, J = 8.0 Hz, 2H), 5.62 (s, 2H).

$^{13}C$ NMR (101 MHz, DMSO-$d_6$) δ 175.10, 159.52, 159.38, 144.87, 140.11, 133.77, 133.14, 131.03, 129.59, 129.50, 129.35, 126.36, 126.22, 123.96, 114.49, 55.52, 49.10.

HRMS (ESI): m/z theoretical value: $C_{19}H_{15}FN_3O_3[M+H]^+$ 334.1186, measured value: 334.1182.

Compound 9:

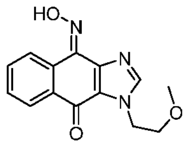

(E)-9-(hydroxylimino)-3-(2-methoxyethyl)-3,9-dihydro-4H-naphthol [2,3-d] imidazole-4-one

$^1$H NMR (400 MHz, DMSO-$d_6$) δ 13.04 (s, 1H), 8.30-8.23 (m, 2H), 8.09 (dd, J = 8.0, 1.4 Hz, 1H), 7.68 (td, J = 8.0, 1.4 Hz, 1H), 7.59 (td, J = 8.0, 1.2 Hz, 1H), 4.61 (t, J = 5.2 Hz, 2H), 3.68 (t, J = 4.0 Hz, 2H), 3.20 (s, 3H).

$^{13}$C NMR (101 MHz, DMSO-$d_6$) δ 175.16, 145.37, 140.16, 139.96, 133.69, 133.09, 131.05, 129.57, 126.48, 126.20, 123.93, 70.72, 58.48, 46.46.

HRMS (ESI): m/z theoretical value: $C_{14}H_{13}N_3O_3[M+H]^+$ 272.1030, measured value: 272.1032.

Compound 10:

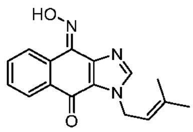

(E)-9-(hydroxylimino)-3-(3-methyl-2-ene-1-yl)-3,9-dihydro-4H-naphthol [2,3-d] imidazole-4-one

$^1$H NMR (400 MHz, DMSO-$d_6$) δ 13.03 (s, 1H), 8.32 (s, 1H), 8.26 (dd, J = 8.0, 1.2 Hz, 1H), 8.10 (dd, J = 8.0, 1.4 Hz, 1H), 7.68 (td, J = 8.0, 1.4 Hz, 1H), 7.59 (td, J = 8.0, 1.2 Hz, 1H), 5.44-5.35 (m, 1H), 5.06 (d, J = 8.0 Hz, 2H), 1.77 (s, 3H), 1.67 (s, 3H).

$^{13}$C NMR (101 MHz, DMSO-$d_6$) δ 175.05, 144.24, 140.17, 139.91, 137.77, 133.66, 133.07, 131.08, 129.58, 126.45, 126.23, 123.92, 119.76, 44.64, 25.77, 18.42.

HRMS (ESI): m/z theoretical value: $C_{16}H_{15}N_3O_2[M+H]^+$ 282.1237, measured value: 282.1239.

Compound 11:

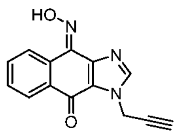

(E)-9-(hydroxylimino)-3-(propyl-2-yne-1-yl)-3,9-dihydro-4H-naphthol [2,3-d] imidazole-4-one

$^1$H NMR (400 MHz, DMSO-$d_6$) δ 13.02 (s, 1H), 8.41 (s, 1H), 8.28 (d, J = 8.0 Hz, 1H), 8.11 (d, J = 8.0 Hz, 1H), 7.73-7.64 (m, 1H), 7.64-7.54 (m, 1H), 5.35 (s, 2H), 3.53 (s, 1H).

$^{13}$C NMR (101 MHz, DMSO-$d_6$) δ 175.02, 144.29, 139.99, 133.91, 133.22, 130.86, 129.61, 129.58, 126.61, 126.12, 124.05, 78.38, 77.22, 36.50.

HRMS (ESI): m/z theoretical value: $C_{14}H_9N_3O_2[M+H]^+$ 252.0768, measured value: 252.0769.

Compound 12:

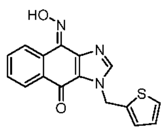

(E)-9-(hydroxylimino)-3-(thiophene-2-yl-methyl)-3,9-dihydro-4H-naphthol [2,3-d] imidazole-4-one

$^1$H NMR (400 MHz, DMSO-$d_6$) δ 13.01 (s, 1H), 8.49 (s, 1H), 8.27 (d, J = 8.0, 1H), 8.12 (dd, J = 7.9, 1.5 Hz, 1H), 7.69

(t, J = 8.0, 1H), 7.60 (t, J = 8.0 Hz, 1H), 7.43 (d, J = 8.0 Hz, 1H), 7.19 (d, J = 4.0 Hz, 1H), 6.95 (dd, J = 8.0, 4.0 Hz, 1H), 5.88 (s, 2H).

$^{13}C$ NMR (101 MHz, DMSO-$d_6$) δ 175.20, 144.72, 140.04, 139.91, 139.42, 133.87, 133.20, 130.93, 129.60, 127.89, 127.49, 127.25, 126.19,126.18, 124.02, 44.36.

HRMS (ESI): m/z theoretical value: $C_{16}H_{11}N_3O_2S[M+H]^+$ 310.0645, measured value: 310.0642.

Compound 13:

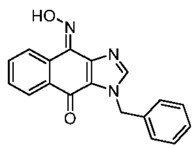

(E)-9-(hydroxylimino)-3-benzyl-3,9-dihydro-4H-naphthol [2,3-d] imidazole-4-one

$^1H$ NMR (400 MHz, DMSO-$d_6$) δ 13.05 (s, 1H), 8.51 (s, 1H), 8.27 (dd, J = 8.0, 1.2 Hz, 1H), 8.07 (dd, J = 8.0, 1.4 Hz, 1H), 7.67 (td, J = 8.0 1.4 Hz, 1H), 7.57 (td, J = 8.0, 1.2 Hz, 1H), 7.39-7.10 (m, 5H), 5.70 (s, 2H).

$^{13}C$ NMR (101 MHz, DMSO-$d_6$) δ 175.08, 145.10, 140.10, 140.06, 137.44, 133.81, 133.14, 130.98, 129.56, 129.12, 128.25, 127.72, 126.48, 126.18, 123.97, 49.58.

HRMS (ESI): m/z theoretical value: $C_{18}H_{13}N_3O_2[M+H]^+$ 304.1081, measured value: 304.1082.

Compound 14:

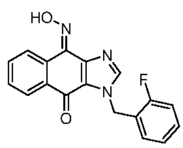

(E)-9-(hydroxylimino)-3-(2-fluorobenzyl)-3,9-dihydro-4H-naphthol [2,3-d] imidazole-4-one

$^1H$ NMR (400 MHz, DMSO-$d_6$) δ 13.04 (s, 1H), 8.41 (s, 1H), 8.31-8.22 (m, 1H), 8.04 (dd, J = 8.0, 1.4 Hz, 1H), 7.70-7.64 (m, 1H), 7.62-7.54 (m, 1H), 7.35-7.27 (m, 1H), 7.25-7.18 (m, 1H), 7.10 (td, J = 7.5, 1.2 Hz, 1H), 7.04 (td, J = 8.0, 1.8 Hz, 1H), 5.77 (s, 2H).

$^{13}C$ NMR (101 MHz, DMSO-$d_6$) δ 175.00, 163.05 (160.71, J = 234.0 Hz), 145.45, 140.08, 139.99, 133.88, 133.16, 130.90, 130.45 (130.37, J = 8.0 Hz), 129.56, 129.35 (129.31, J = 4.0 Hz), 126.66, 126.15, 125.19 (125.15, J = 4.0 Hz), 124.45 (124.31, J = 14.0 Hz), 123.99, 115.92 (115.71, J = 21.0 Hz), 44.17 (44.12, J = 5.0 Hz).

HRMS (ESI): m/z theoretical value: $C_{18}H_{12}FN_3O_2[M+H]^+$ 322.0986, measured value: 322.0984.

Compound 15:

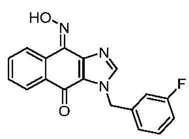

(E)-9-(hydroxylimino)-3-(3-fluorobenzyl)-3,9-dihydro-4H-naphthol [2,3-d] imidazole-4-one

$^1H$ NMR (400 MHz, DMSO-$d_6$) δ 8.51 (s, 1H), 8.27 (d, J = 8.0 Hz, 1H), 8.07 (d, J = 8.0 Hz, 1H), 7.68 (t, J = 8.0 Hz, 1H), 7.58 (t, J = 8.0 Hz, 1H), 7.40-7.30 (m, 1H), 7.17-7.06 (m, 3H), 5.71 (s, 2H).

$^{13}C$ NMR (101 MHz, DMSO-$d_6$) δ 175.12, 163.82 (161.39, J = 243.0 Hz), 145.17, 140.22 (140.15, J = 7.0 Hz), 140.10, 133.87, 133.17, 131.22 (131.14, J = 8.0 Hz), 130.95, 129.56, 126.63, 126.47, 126.18, 123.99, 123.75 (123.72, J = 3.0 Hz), 115.21 (115.00, J = 21.0 Hz), 114.78 (114.56, J = 22.0 Hz), 49.06 (49.05, J = 1.0 Hz).

Compound 16:

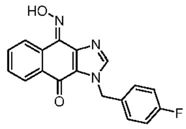

(E)-9-(hydroxylimino)-3-(4-fluorobenzyl)-3,9-dihydro-4H-naphthol [2,3-d] imidazole-4-one

$^1H$ NMR (400 MHz, DMSO-$d_6$) δ 13.04 (s, 1H), 8.51 (s, 1H), 8.27 (dd, J = 8.0, 1.2 Hz, 1H), 8.07 (dd, J = 8.0, 1.4 Hz, 1H), 7.68 (td, J = 8.0, 1.4 Hz, 1H), 7.58 (td, J = 8.0, 1.2 Hz, 1H), 7.39-7.31 (m, 2H), 7.18 -7.07 (m, 2H), 5.68 (s, 2H).
$^{13}C$ NMR (101 MHz, DMSO-$d_6$) δ 175.11, 163.34 (160.91, J = 243.0 Hz), 145.01, 140.13, 140.09, 133.83, 133.65 (133.62, J = 3.0 Hz), 133.15, 130.96, 130.15 (130.07, J = 8.0 Hz), 129.56, 126.40, 126.19, 123.97, 116.03 (115.81, J = 22.0 Hz), 48.87.
HRMS (ESI): m/z theoretical value: $C_{18}H_{12}FN_3O_2[M+H]^+$ 322.0986, measured value: 322.0986.

Compound 17:

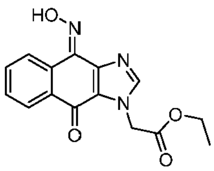

(E)-2-(4-(hydroxylimino)-9-oxo-4,9-dihydro-1H-naphthol [2,3-d] imidazole-1-yl) acetate

$^1H$ NMR (400 MHz, DMSO-$d_6$) δ 13.01 (s, 1H), 8.31-8.25 (m, 2H), 8.06 (dd, J = 8.0, 0.8 Hz, 1H), 7.70 (td, J = 8.0, 1.2 Hz, 1H), 7.60 (td, J = 8.0, 0.8 Hz, 1H), 5.32 (s, 2H), 4.15 (q, J = 7.2 Hz, 2H), 1.19 (t, J = 7.2 Hz, 3H).
$^{13}C$ NMR (101 MHz, DMSO-$d_6$) δ 175.27, 168.10, 145.42, 134.05, 133.27, 130.71, 129.61, 127.16, 126.61, 126.05, 124.13, 61.78, 48.16, 14.47.
HRMS (ESI): m/z theoretical value: $C_{15}H_{13}N_3O_4[M+H]^+$ 300.0979, measured value: 300.0981.

Compound 18:

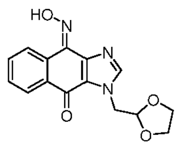

(E) -3-((1,3-ethyl-dioxoheterocycle-2-yl) methyl)-9-(hydroxylimino)-3,9-dihydro-4H-naphthol [2,3-d] imidazole-4-one

$^1H$ NMR (400 MHz, DMSO-$d_6$) δ 13.03 (s, 1H), 8.30-8.20 (m, 2H), 8.09 (dd, J = 8.0, 1.4 Hz, 1H), 7.68 (td, J = 8.0, 1.4 Hz, 1H), 7.59 (td, J = 8.0, 1.2 Hz, 1H), 5.20 (t, J = 4.0 Hz, 1H), 4.67 (d, J = 4.0 Hz, 2H), 3.82-3.73 (m, 4H).
$^{13}C$ NMR (101 MHz, DMSO-$d_6$) δ 175.26, 145.49, 140.06, 139.55, 133.72, 133.12, 131.03, 129.58, 126.88, 126.19, 123.95, 101.10, 65.05, 48.12.
HRMS (ESI): m/z theoretical value: $C_{15}H_{13}N_3O_4[M+H]^+$ 300.0979, measured value: 300.0978.

Compound 19:

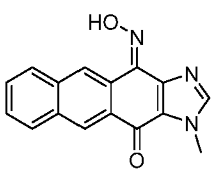

(E)-11-(hydroxylimino)-3-methyl-3,11-dihydro-4H-anthraquinone [2,3-d] imidazole-4-one

$^1$H NMR (400 MHz, DMSO-$d_6$) δ 13.09 (s, 1H), 9.04 (s, 1H), 8.77 (d, J = 12.4 Hz, 2H), 8.15 (dd, J = 26.4, 7.7 Hz, 2H), 7.64 (d, J = 20.0 Hz, 2H), 4.03 (s, 3H).

HRMS (ESI): m/z theoretical value: $C_{15}H_{13}N_3O_4$[M+H]$^+$ 278.0924, measured value: 278.0922.

Compound 20:

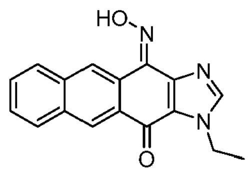

(E)-11-(hydroxylimino)-3-ethyl-3,11-dihydro-4H-anthraquinone [2,3-d] imidazole-4-one

$^1$H NMR (400 MHz, DMSO-$d_6$) δ 13.89 (s, 1H), 8.93 (s, 1H), 8.84 (s, 2H), 8.21 (dd, J = 22.4, 8.2 Hz, 2H), 7.82-7.64 (m, 2H), 4.60 (d, J = 7.8 Hz, 2H), 1.41 (t, J = 8.0 Hz, 3H).

HRMS (ESI): m/z theoretical value: $C_{15}H_{13}N_3O_4$[M+H]$^+$ 292.1081, measured value: 292.1078.

**Example 2**

2,2'-(1,4-dioxy-1,4,6,7-tetrahydronaphthalene-2,3-diyl)bis(isoindolin-1,3-dione) (13.3 g) was dissolved into 200 mL of dichloromethane and 20 mL of methanol with the addition of cyclopropylamine, and subjected to reaction for 2h, then solvent was removed, the obtained product was added with 100 mL of water, pulped for 10 min, and subjected to suction filtration to obtain red solid (10 g). 10 g solid was directly added to 150 mL of ethanol; 8 mL hydrazine hydrate (80) was dropwisely added, and the mixture was stirred at room temperature for half an hour; organic phase was removed; 200 mL of water was added, and the obtained product was pulped for 10 min, filtered and washed with water until colorless, and then vacuum dried to obtain blue solid 2-amino-3-cyclopropyl-6,7-dihydronaphthalene-1,4-dione (6.2 g). 2-amino-3-cyclopropyl-6,7-dihydronaphthalene-1,4-dione (1 g) was subjected to reflux for 2.5 h in an orthoformic acid medium (25 mL), and solvent was removed, and the remaining material was isolated by silica gel column chromatography to obtain white solid 1-cyclopropyl 6,7-dihydro-1H-naphthol [2,3-d] imidazole-4,9-dione (0.46 g). 3-Cyclopropylnaphthoquinone imidazole (0.21 g), hydroxylamine hydrochloride (0.35 g), and pyridine (0.8 mL) were dissolved into ethanol (70 mL) and reacted for 36 h at 100°C; at the end of reaction, solid was separated out from solution, directly subjected to suction filtration, and vacuum dried to obtain light yellow solid (0.15 g).

Compound 21 could be prepared using the method in Example 2:

Compound 21:

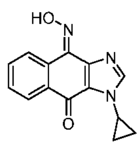

(E)-3-cyclopropyl-9-(hydroxylimino)-3,9-dihydro-4H-naphthol [2,3-d] imidazole-4-one

$^1$H NMR (400 MHz, DMSO-$d_6$) δ 13.00 (s, 1H), 8.31 (s, 1H), 8.25 (dd, J = 8.0, 1.2 Hz, 1H), 8.11 (dd, J = 8.0, 1.4 Hz, 1H), 7.67 (td, J = 8.0, 1.4 Hz, 1H), 7.59 (td, J = 8.0, 1.2 Hz, 1H), 3.93-3.86 (m, 1H), 1.13-1.03 (m, 4H).

$^{13}$C NMR (101 MHz, DMSO-$d_6$) δ 174.50, 143.32, 140.18, 139.97, 133.46, 132.97, 131.25, 129.58, 128.36, 126.23, 123.86, 29.61, 7.44.

HRMS (ESI): m/z theoretical value: $C_{14}H_{11}N_3O_2$[M+H]$^+$ 254.0924, measured value: 254.0927.

**Example 3**

Nitrogen-(3-chloro-1,4-dioxy-1,4,6,7-tetrahydronaphthalene-2-yl) acetamide (0.25 g) was dissolved into 20 mL of tetrahydrofuran; reaction liquid was added with triethylamine and tert-butylamine in turn, subjected to reflux reaction for

26 h under nitrogen protection, and cooled to room temperature, and then solvent was removed, the remaining solution was added with 10 mL of ultrapure water, and pulped for half an hour to obtain nitrogen-(3-(tert-butylamino)-1,4-dioxy-1,4,6,7-tetrahydronaphthalene-2-yl) acetamide. The obtained product was dispersed into 85 mL of hydrazine hydrate (50%) for reaction for 2 h at 80°C and extracted with dichloromethane; organic phase was collected, washed with saturated salt solution, dried with anhydrous sodium sulfate, filtered, concentrated, and isolated by silica gel column chromatography to obtain a product 2-amino-3-(tert-butylamino)-6,7-dihydronaphthalene-1,4-dione. 2-Amino-3-(tert-butylamino)-6,7-dihydronaphthalene-1,4-dione (1.63 g) was added with 50 mL of triethyl orthoformate for reflux reaction for 3 h, and then cooled to room temperature; solvent was removed, isolated and purified by silica gel column chromatography to obtain a product 3-tert-butylnaphthoquinone imidazole. 3-tert-butylnaphthoquinone imidazole (0.21 g), hydroxylamine hydrochloride (0.17 g), and pyridine (0.4 mL) were dissolved into ethanol (50 mL) and reacted for 24 h at 110°C; at the end of reaction, solid was separated out from solution, directly subjected to suction filtration, and vacuum dried to obtain light yellow solid (0.16 g).

Amines having different substitutes were added to prepare Compounds 22-32 according to the method in Example 3; other materials and reaction conditions for the compounds were kept the same and raw materials could be purchased commercially:

Compound 22:

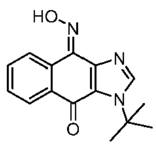

(E)-3-tert-butyl-9-(hydroxylimino)-3,9-dihydro-4H-naphthol [2,3-d] imidazole-4-one

$^1$H NMR (400 MHz, DMSO-$d_6$) δ 13.55 (s, 1H), 8.49 (s, 1H), 8.23 (d, J = 8.0 Hz, 1H), 8.17 (d, J = 7.4 Hz, 1H), 7.68 (t, J = 8.0 Hz, 1H), 7.60 (t, J = 8.0 Hz, 1H), 1.72 (s, 9H).

$^{13}$C NMR (101 MHz, DMSO-$d_6$) δ 173.70, 142.87, 142.71, 140.17, 132.99, 132.43, 131.66, 129.80, 127.11, 127.08, 123.26, 59.59, 29.32.

HRMS (ESI): m/z theoretical value: $C_{15}H_{15}N_3O_2$[M+H]$^+$ 270.1237, measured value: 270.1241.

Compound 23:

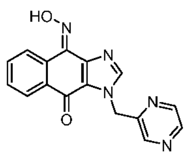

(E)-9-(hydroxylimino)-3-(pyrazine-2-yl-methyl)-3,9-dihydro-4H-naphthol [2,3-d] imidazole-4-one

$^1$H NMR (400 MHz, DMSO-$d_6$) δ 13.06 (s, 1H), 8.71 (s, 1H), 8.59-8.41 (m, 3H), 8.27 (d, J = 8.0 Hz, 1H), 7.99 (d, J = 8.0 Hz, 1H), 7.72-7.51 (m, 2H), 5.87 (s, 2H).

$^{13}$C NMR (101 MHz, DMSO-$d_6$) δ 174.99, 151.72, 145.78, 144.60, 144.34, 143.98, 140.12, 139.84, 133.89, 133.16, 130.80, 129.55, 126.79, 126.08, 124.01, 49.11.

HRMS (ESI): m/z theoretical value: $C_{16}H_{11}N_5O_2$[M+H]$^+$ 306.0986, measured value: 306.0985.

Compound 24:

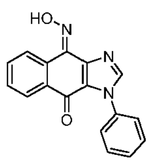

(E)-9-(hydroxylimino)-3-benzyl-3,9-dihydro-4H-naphthol [2,3-d] imidazole-4-one

$^1H$ NMR (400 MHz, DMSO-$d_6$) δ 13.10 (s, 1H), 8.47 (s, 1H), 8.30 (dd, J = 8.0, 1.2 Hz, 1H), 8.02 (dd, J = 8.0, 1.4 Hz, 1H), 7.69 (td, J = 8.0, 1.4 Hz, 1H), 7.63-7.48 (m, 6H).

$^{13}C$ NMR (101 MHz, DMSO-$d_6$) δ 174.10, 145.03, 140.19, 140.10, 135.90, 133.66, 133.07, 131.24, 129.59, 129.36, 129.31, 127.14, 126.57, 126.27, 123.91.

HRMS (ESI): m/z theoretical value: $C_{17}H_{11}N_3O_2[M+H]^+$ 290.0924, measured value: 290.0922.

Compound 25:

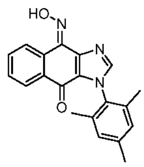

(E)-9-(hydroxylimino)-3-(2,4,6-trimethylphenyl)-3,9-dihydro-4H-naphthol [2,3-d] imidazole-4-one

$^1H$ NMR (400 MHz, DMSO-$d_6$) δ 13.09 (s, 1H), 8.35-8.26 (m, 2H), 8.00-7.95 (m, 1H), 7.73-7.65 (m, 1H), 7.62-7.54 (m, 1H), 7.03 (s, 2H), 3.34 (s, 9H).

$^{13}C$ NMR (101 MHz, DMSO-$d_6$) δ 174.25, 144.93, 140.19, 139.83, 138.97, 135.02, 133.96, 133.14, 132.50, 130.95, 129.58, 129.12, 127.34, 126.10, 124.05, 21.06, 17.48.

HRMS (ESI): m/z theoretical value: $C_{20}H_{17}N_3O_2[M+H]^+$ 322.1394, measured value: 322.1392.

Compound 26:

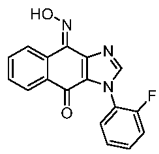

(E)-9-(hydroxylimino)-3-(2-fluorophenyl)-3,9-dihydro-4H-naphthol [2,3-d] imidazole-4-one

$^1H$ NMR (400 MHz, DMSO-$d_6$) δ 13.09 (s, 1H), 8.50 (s, 1H), 8.32 (dd, J = 8.0, 1.2 Hz, 1H), 8.01 (dd, J = 8.0, 1.4 Hz, 1H), 7.75-7.65 (m, 2H), 7.64-7.56 (m, 2H), 7.51-7.44 (m, 1H), 7.38 (td, J = 8.0, 1.2 Hz, 1H).

$^{13}C$ NMR (101 MHz, DMSO-$d_6$) δ 174.23, 158.18 (155.70, J = 248.0 Hz), 145.23, 139.94 (139.68, J = 26.0 Hz), 133.93, 133.26, 131.86 (131.78, J = 8.0 Hz), 130.84, 129.64, 129.18, 127.94, 127.80, 126.10, 125.33 (125.29, J = 4.0 Hz), 124.21, 124.10, 116.75 (116.55, J = 20.0 Hz).

HRMS (ESI): m/z theoretical value: $C_{17}H_{10}FN_3O_2[M+H]^+$ 308.0830, measured value: 308.0831.

Compound 27:

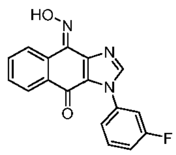

(E)-9-(hydroxylimino)-3-(3-fluorophenyl)-3,9-dihydro-4H-naphthol [2,3-d] imidazole-4-one

$^1H$ NMR (400 MHz, DMSO-$d_6$) δ 13.08 (s, 1H), 8.49 (s, 1H), 8.30 (dd, J = 8.0, 1.2 Hz, 1H), 8.03 (dd, J = 8.0, 1.4 Hz, 1H), 7.70 (td, J = 8.0, 1.4 Hz, 1H), 7.62-7.54 (m, 3H), 7.48-7.43 (m, 1H), 7.42-7.35 (m, 1H).

$^{13}C$ NMR (101 MHz, DMSO-$d_6$) δ 174.15, 163.26 (160.83, J = 243.0 Hz), 145.03, 140.17 (140.02, J = 15.0 Hz), 137.30, 137.19, 133.70, 133.11, 131.17, 130.96 (130.87, J = 9.0 Hz), 129.58, 127.19, 126.28, 123.92, 122.87 (122.84, J =

3.0 Hz), 116.42 (116.21, J = 21.0 Hz), 114.56 (114.31, J = 25.0 Hz).

HRMS (ESI): m/z theoretical value: $C_{17}H_{10}FN_3O_2[M+H]^+$ 308.0830, measured value: 308.0832.

Compound 28:

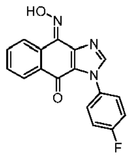

(E)-9-(hydroxylimino)-3-(4-fluorophenyl)-3,9-dihydro-4H-naphthol [2,3-d] imidazole-4-one

$^1H$ NMR (400 MHz, DMSO-$d_6$) δ 13.07 (s, 1H), 8.45 (s, 1H), 8.32-8.27 (m, 1H), 8.02 (dd, J = 8.0, 1.4 Hz, 1H), 7.72-7.62 (m, 3H), 7.61-7.55 (m, 1H), 7.41-7.35 (m, 2H).

$^{13}C$ NMR (101 MHz, DMSO-$d_6$) δ 174.18, 163.67 (161.22, J = 245.0 Hz), 145.09, 140.08, 133.72, 133.10, 132.30 (132.27, J = 3.0 Hz), 131.19, 129.59, 128.95 (128.86, J = 9.0 Hz), 127.28, 126.60, 126.24, 123.93, 116.22 (115.99, J = 23.0 Hz).

HRMS (ESI): m/z theoretical value: $C_{17}H_{10}FN_3O_2[M+H]^+$ 308.0830, measured value: 308.0829.

Compound 29:

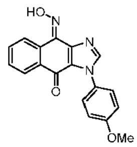

(E)-9-(hydroxylimino)-3-(4-methoxyphenyl)-3,9-dihydro-4H-naphthol [2,3-d] imidazole-4-one

$^1H$ NMR (400 MHz, DMSO-$d_6$) δ 13.07 (s, 1H), 8.40 (s, 1H), 8.32-8.28 (m, 1H), 8.02 (dd, J = 8.0, 1.4 Hz, 1H), 7.70 (td, J = 8.0, 1.4 Hz, 1H), 7.59 (td, J = 8.0, 1.2 Hz, 1H), 7.51-7.47 (m, 2H), 7.08-7.04 (m, 2H), 3.82 (s, 3H).

$^{13}C$ NMR (101 MHz, DMSO-$d_6$) δ 174.10, 159.95, 159.53, 145.06, 140.16, 133.63, 133.05, 131.28, 129.59, 128.73, 127.82, 127.23, 126.25, 123.90, 114.40, 56.00.

HRMS (ESI): m/z theoretical value: $C_{18}H_{13}FN_3O_3[M+H]^+$ 320.1030, measured value: 320.1026.

Compound 30:

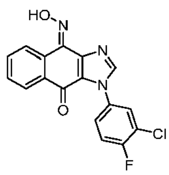

(E)-9-(hydroxylimino)-3-(3-chloro-4-fluorophenyl)-3,9-dihydro-4H-naphthol [2,3-d] imidazole-4-one

$^1H$ NMR (400 MHz, DMSO-$d_6$) δ 13.13 (s, 1H), 8.52 (s, 1H), 8.30 (dd, J = 8.0, 1.2 Hz, 1H), 8.05-7.97 (m, 2H), 7.72-7.64 (m, 2H), 7.62-7.55 (m, 2H).

$^{13}C$ NMR (101 MHz, DMSO-$d_6$) δ 174.25, 158.99 (156.52, J = 247.0 Hz), 145.02, 139.91, 133.71, 133.19, 132.90 (132.87, J = 3.0 Hz), 131.06, 129.61, 129.20, 127.83, 127.75, 127.28, 126.26, 123.95, 120.02 (119.83, J = 19.0 Hz), 117.46 (117.24 J = 22.0 Hz).

HRMS (ESI): m/z theoretical value: $C_{17}H_9ClFN_3O_2[M+H]^+$ 342.0440, measured value: 342.0438.

Compound 31:

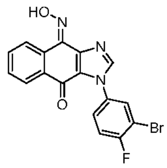

(E)-9-(hydroxylimino)-3-(3-bromo-4-fluorophenyl)-3,9-dihydro-4H-naphthol [2,3-d] imidazole-4-one

$^1$H NMR (400 MHz, DMSO-$d_6$) δ 13.06 (s, 1H), 8.47 (s, 1H), 8.30 (dd, J = 8.0, 1.2 Hz, 1H), 8.10-8.00 (m, 2H), 7.72 - 7.66 (m, 2H), 7.61-7.53 (m, 2H).

$^{13}$C NMR (101 MHz, DMSO-$d_6$) δ 174.27, 159.55, 145.12, 140.03 (139.98, J = 5.0 Hz), 133.77, 133.16, 133.15, 131.83, 131.11, 129.59, 128.45, 128.37, 127.34, 126.25, 123.96, 117.20 (116.96, J = 24.0 Hz), 108.33 (108.11, J = 22.0 Hz).

HRMS (ESI): m/z theoretical value: $C_{17}H_9BrFN_3O_2[M+H]^+$ 385.9935, measured value: 385.9930.

Compound 32:

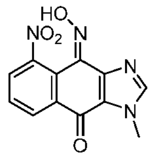

(E)-9-(hydroxylimino)-3-methyl -8-nitro-3,9-dihydro-4H-naphthol [2,3-d] imidazole-4-one

1H NMR (400 MHz, DMSO-d6) δ 14.67 (s, 1H), 9.66 (s, 1H), 8.37 (d, J = 7.8 Hz, 1H), 8.25 (d, J = 7.9 Hz, 1H), 8.09 (t, J = 7.8 Hz, 1H), 4.14 (s, 3H).

HRMS (ESI): m/z theoretical value: $C_{17}H_9BrFN_3O_2[M+H]^+$ 273.0619, measured value: 273.0620.

**Example 4**

2,3-Diamine-1,4-benzoquinone (20 g) was dissolved into 100 mL of acetic acid, subjected to reflux reaction for 6 h, and cooled to room temperature, then poured into an ice-water mixture with the addition of ammonium hydroxide to regulate pH to 9-10, and the obtained solution was subjected to suction filtration and vacuum dried to obtain yellow solid 1,4-naphthoquinone imidazole (22 g). 1,4-naphthoquinone imidazole (0.3 g) and potassium hydroxide (0.15 g) were added to dimethyl sulfoxide solution (3 mL); the mixture was stirred at 50°C for 30 min and added with iodomethane (0.2 mL), after reaction for 2, water was added to separate out solid; the solid was subjected to suction filtration, washed with water, and vacuum dried to obtain yellow solid 2-methyl-1,4-naphthoquinone imidazole (0.31 g). 2-methylnaphthoquinone imidazole (0.2 g), hydroxylamine hydrochloride (0.28 g), and pyridine (0.7 mL) were dissolved into ethanol (70 mL) and reacted for 36 h at 105°C; at the end of reaction, solid was separated out from solution, directly subjected to suction filtration, and vacuum dried to obtain light yellow solid (0.16 g).

2,3,-Diamine-1,4-naphthoquinone (20 g) was dissolved into 100 mL of acetic acid and subjected to reflux reaction for 6 h to obtain anthraquinone imidazole; or halohydrocarbons having different substitutes were added to prepare Compounds 33-41 according to the method in Example 4; other materials and reaction conditions for the compounds were kept the same and raw materials could be purchased commercially:

Compound 33:

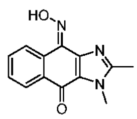

(E)-9-(hydroxylimino)-2,3-dimethyl-3,9-dihydro-4H-naphthol [2,3-d] imidazole-4-one

$^{1}$H NMR (400 MHz, DMSO-$d_6$) δ 13.01 (s, 1H), 8.26 (d, J = 0.8 Hz, 2H), 8.07 (dd, J = 8.0, 4.0 Hz, 1H), 7.72-7.64 (m, 1H), 7.61-7.52 (m, 1H), 4.00 (s, 3H), 2.81 (s, 3H).

$^{13}$C NMR (101 MHz, DMSO-$d_6$) δ 175.27, 145.22, 140.18, 139.62, 133.71, 133.06, 131.04, 129.58, 127.17, 126.12, 123.92, 34.14, 20.1.

HRMS (ESI): m/z theoretical value: $C_{12}H_9N_3O_2$[M+H]$^+$ 241.0851, measured value: 241.0854.

Compound 34:

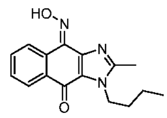

(E)-3-butyl-9-(hydroxylimino)-2-methyl-3,9-dihydro-4H-naphthol [2,3-d] imidazole-4-one

$^{1}$H NMR (400 MHz, DMSO-$d_6$) δ 13.14 (s, 1H), 8.27 (d, J = 7.9 Hz, 1H), 8.12 (dd, J = 7.8, 1.5 Hz, 1H), 7.73 - 7.65 (m, 1H), 7.65 - 7.57 (m, 1H), 4.41 (d, J = 15.1 Hz, 2H), 1.73 - 1.65 (m, 2H), 1.32 (q, J = 7.4 Hz, 2H), 0.90 (t, J = 7.3 Hz, 3H).

Compound 35:

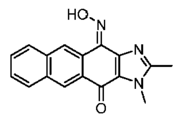

(E)-11-(hydroxylimino)-2,3-dimethyl-3,11-dihydro-4H-anthraquinone [2,3-d] imidazole-4-one

$^{1}$H NMR (400 MHz, DMSO-$d_6$) δ 13.09 (s, 1H), 8.77 (d, J = 12.4 Hz, 2H), 8.15 (dd, J = 26.4, 7.8 Hz, 2H), 7.64 (d, J = 20.0 Hz, 2H), 4.03 (s, 3H), 2.54 (s, 3H).

HRMS (ESI): m/z theoretical value: $C_{12}H_9N_3O_2$[M+H]$^+$ 292.1081, measured value: 292.1082.

Compound 36:

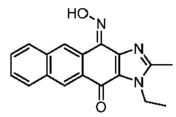

(E)-3-ethyl-11-(hydroxylimino)-2-methyl-3,11-dihydro-4H-anthraquinone [2,3-d] imidazole-4-one

$^{1}$H NMR (400 MHz, DMSO-$d_6$) δ 13.87 (s, 1H), 8.84 (s, 2H), 8.21 (dd, J = 22.4, 8.2 Hz, 2H), 7.82-7.64 (m, 2H), 4.60 (d, J = 7.8 Hz, 2H), 2.79 (s, 3H), 1.40 (d, J = 8.0 Hz, 3H).

HRMS (ESI): m/z theoretical value: $C_{12}H_9N_3O_2$[M+H]$^+$ 306.1237, measured value: 306.1240.

Compound 37:

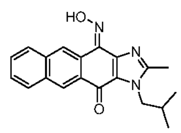

(E)-3-isobutyl-11-(hydroxylimino)-2-methyl-3,11-dihydro-4H-anthraquinone [2,3-d] imidazole-4-one

$^{1}$H NMR (400 MHz, DMSO-$d_6$) δ 13.90 (s, 1H), 8.84 (s, 2H), 8.20 (dd, J = 15.0, 8.4 Hz, 2H), 7.81-7.58 (m, 2H), 4.41

(d, J = 7.0 Hz, 2H), 2.78 (s, 3H), 2.22-2.14 (m, 1H), 0.95 (d, J = 6.8 Hz, 6H).

HRMS (ESI): m/z theoretical value: $C_{12}H_9N_3O_2[M+H]^+$ 344.1550, measured value: 344.1554.

Compound 38:

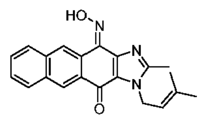

(E)-11-(hydroxylimino)-2-methyl-3-(3-methyl-2-ene-1-yl)-3,11-dihydro-4H-anthraqu inone [2,3-d] imidazole-4-one

$^1$H NMR (400 MHz, DMSO-$d_6$) δ 13.44 (s, 1H), 8.81 (s, 2H), 8.24-8.13 (m, 2H), 7.74-7.66 (m, 2H), 5.30-5.22 (m, 2H), 5.14-5.10 (m, 1H), 2.65 (s, 3H), 1.84 (s, 3H), 1.70 (s, 3H).

HRMS (ESI): m/z theoretical value: $C_{12}H_9N_3O_2[M+H]^+$ 346.1550, measured value: 346.1555.

Compound 39:

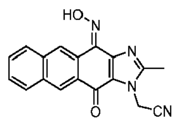

(E)-2-(4-(hydroxylimino)-2- methyl-11-oxo-4,11-dihydro-1H-anthracene [2,3-d] imidazole-1-yl) acetonitrile

$^1$H NMR (400 MHz, DMSO-$d_6$) δ 13.46 (s, 1H), 8.69 (s, 2H), 8.26 (s, 2H), 7.76 (d, J = 6.4 Hz, 2H), 5.70 (s, 2H), 2.59 (s, 3H).

HRMS (ESI): m/z theoretical value: $C_{12}H_9N_3O_2[M+H]^+$ 317.1033, measured value: 317.1035.

Compound 40:

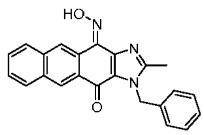

(E)-3-benzyl-11-(hydroxylimino)-2-methyl-3,11-dihydro-4H-anthraquinone [2,3-d] imidazole-4-one

$^1$H NMR (400 MHz, DMSO-$d_6$) δ 13.01 (s, 1H), 8.78 (d, J = 33.2 Hz, 2H), 8.15 (dd, J = 16.8, 7.8 Hz, 2H), 7.65 (d, J = 19.9 Hz, 2H), 7.43-7.12 (m, 5H), 5.83 (s, 2H), 2.65 (s, 3H).

HRMS (ESI): m/z theoretical value: $C_{12}H_9N_3O_2[M+H]^+$ 368.1394, measured value: 368.1395.

Compound 41:

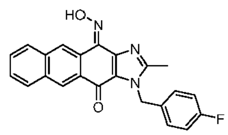

(E)-3-(4-fluorobenzyl)-11-(hydroxylimino)-2-methyl-3,11-dihydro-4H-anthraquinone [2,3-d] imidazole-4-one

$^1$H NMR (400 MHz, DMSO-$d_6$) δ 13.50 (s, 1H), 8.81 (d, J = 16.8 Hz, 2H), 8.24 - 8.13 (m, 2H), 7.75 - 7.60 (m, 2H), 7.16 (d, J = 8.8 Hz, 4H), 5.86 (s, 2H), 2.62 (s, 3H).

HRMS (ESI): m/z theoretical value: $C_{12}H_9N_3O_2[M+H]^+$ 386.1300, measured value: 386.1327.

**Example 5**

2,3-Diamine-1,4-benzoquinone (4 g) was dissolved into 13 mL of dimethyl sulfoxide, added with butyraldehyde (2.12 mL), and subjected to open-ended reaction at 80°C for 13 h, and cooled to room temperature, then slowly added with water, solid was separated out, subjected to suction filtration and vacuum dried to obtain yellow solid 1,4-naphthoquinone imidazole (0.15 g). 1,4-naphthoquinone imidazole (0.3 g) and potassium hydroxide (0.15 g) were added to dimethyl sulfoxide solution (3 mL), respectively; the mixture was stirred at 50°C for 30 min and added with iodobutane (0.2 mL), after reaction for 2, water was added to separate out solid; the solid was subjected to suction filtration, washed with water, and vacuum dried to obtain yellow solid 2-methyl-1,4-naphthoquinone imidazole (0.31 g). 2-methylnaphthoquinone imidazole (0.2 g), hydroxylamine hydrochloride (0.11 g), and pyridine (0.35 mL) were dissolved into ethanol (35 mL) and reacted for 36 h at 105°C; at the end of reaction, solid was separated out from solution, directly subjected to suction filtration, and vacuum dried to obtain light yellow solid (0.15 g).

2,3,-Diamine-1,4-benzoquinone (4 g) was dissolved into 13 mL of dimethyl sulfoxide and reacted with different aldehydes; or 2,3,-diamine-1,4-naphthoquinone (20 g) was dissolved into dimethyl sulfoxide and reacted with different aldehydes; or halohydrocarbons having different substitutes were added to prepare Compounds 42-59 according to the method in Example 5; other materials and reaction conditions for the compounds were kept the same and raw materials could be purchased commercially:

Compound 42:

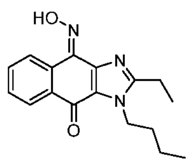

(E)-3-butyl-9-(hydroxylimino)-2-ethyl-3,9-dihydro-4H-naphthol [2,3-d] imidazole-4-one

$^1H$ NMR (400 MHz, DMSO-$d_6$) δ 13.26 (s, 1H), 8.25 (d, J = 7.9 Hz, 1H), 8.10 (d, J = 8.0 Hz, 1H), 7.75 - 7.65 (m, 1H), 7.60 (t, J = 7.5 Hz, 1H), 4.38 (t, J = 7.5 Hz, 2H), 2.86 (q, J = 7.5 Hz, 2H), 1.67 (ddd, J = 12.6, 10.2, 6.5 Hz, 2H), 1.31 (td, J = 7.5, 3.7 Hz, 5H), 0.89 (t, J = 7.4 Hz, 3H).

HRMS (ESI): m/z theoretical value: $C_{12}H_9N_3O_2[M+H]^+$ 298.1550, measured value: 298.1557.

Compound 43:

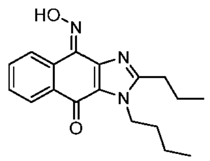

(E)-3-butyl-9-(hydroxylimino)-2-propyl-3,9-dihydro-4H-naphthol [2,3-d] imidazole-4-one

$^1H$ NMR (400 MHz, Chloroform-d) δ 13.95 (s, 1H), 8.42 (d, J = 7.8 Hz, 1H), 8.28 - 8.21 (m, 1H), 7.65 (t, J = 7.3 Hz, 1H), 7.59 - 7.55 (m, 1H), 4.46 (t, J = 7.6 Hz, 2H), 2.80 (t, J = 7.6 Hz, 2H), 1.93 (h, J = 7.7 Hz, 2H), 1.79 (q, J = 7.7 Hz, 2H), 1.44 (q, J = 7.6 Hz, 2H), 1.08 (t, J = 7.4 Hz, 3H), 0.99 (t, J = 7.4 Hz, 3H).

HRMS (ESI): m/z theoretical value: $C_{12}H_9N_3O_2[M+H]^+$ 312.1707, measured value: 312.1709.

Compound 44:

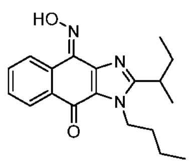

(E)-2-(sec-butyl)-3-butyl-9-(hydroxylimino)-3,9-dihydro-4H-naphthol [2,3-d] imidazole-4-one

$^1$H NMR (400 MHz, DMSO-$d_6$) δ 13.32 (s, 1H), 8.26 (d, J = 8.0 Hz, 1H), 8.13 (d, J = 7.8 Hz, 1H), 7.70 (t, J = 7.7 Hz, 1H), 7.62 (t, J = 7.5 Hz, 1H), 4.52-4.40 (m, 2H), 3.08 (q, J = 6.8 Hz, 1H), 1.80 (dt, J = 14.0, 7.4 Hz, 1H), 1.67 (q, J = 7.3 Hz, 3H), 1.39 - 1.32 (m, 2H), 1.28 (d, J = 6.8 Hz, 3H), 0.90 (t, J = 7.4 Hz, 3H), 0.83 (t, J = 7.2 Hz, 3H).
HRMS (ESI): m/z theoretical value: $C_{12}H_9N_3O_2[M+H]^+$ 326.1863, measured value: 326.1865.

Compound 45:

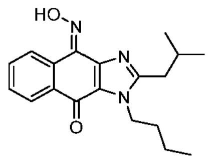

(E)-3-butyl-9-(hydroxylimino)-2-isobutyl-3,9-dihydro-4H-naphthol [2,3-d] imidazole-4-one

$^1$H NMR (400 MHz, Chloroform-d) δ 13.91 (s, 1H), 8.38 (d, J = 7.8 Hz, 1H), 8.21 (d, J = 7.8 Hz, 1H), 7.62 (t, J = 7.6 Hz, 1H), 7.54 (t, J = 7.6 Hz, 1H), 4.44 (t, J = 7.8 Hz, 2H), 2.68 (d, J = 7.0 Hz, 2H), 2.32 (dp, J = 13.8, 6.8 Hz, 1H), 1.78 (p, J = 7.6 Hz, 2H), 1.44 (p, J = 7.4 Hz, 2H), 1.11 - 0.91 (m, 9H).
HRMS (ESI): m/z theoretical value: $C_{12}H_9N_3O_2[M+H]^+$ 326.1863, measured value: 326.1862.

Compound 46:

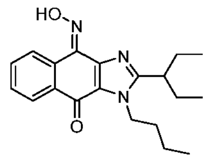

(E)-3-butyl-9-(hydroxylimino)-2-(pentane-3-yl)-3,9-dihydro-4H-naphthol [2,3-d] imidazole-4-one

$^1$H NMR (400 MHz, Chloroform-d) δ 14.05 (s, 1H), 8.44 (dd, J = 8.0, 1.2 Hz, 1H), 8.29 - 8.20 (m, 1H), 7.67 (td, J = 7.6, 1.6 Hz, 1H), 7.59 (td, J = 7.6, 1.4 Hz, 1H), 4.57 - 4.46 (m, 2H), 2.79 (tt, J = 8.4, 5.6 Hz, 1H), 1.92 - 1.80 (m, 6H), 1.49 (h, J = 7.4 Hz, 2H), 1.02 (t, J = 7.4 Hz, 3H), 0.90 (t, J = 7.4 Hz, 6H).
HRMS (ESI): m/z theoretical value: $C_{12}H_9N_3O_2[M+H]^+$ 340.2020, measured value: 340.2025.

Compound 47:

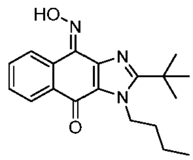

(E)-3-butyl-9-(hydroxylimino)-2-tert-butyl-3,9-dihydro-4H-naphthol [2,3-d] imidazole-4-one

$^1$H NMR (400 MHz, Chloroform-d) δ 14.09 (s, 1H), 8.40 (d, J = 7.8 Hz, 1H), 8.25 (d, J = 7.8 Hz, 1H), 7.64 (t, J = 7.2 Hz, 1H), 7.56 (t, J = 7.8 Hz, 1H), 4.62-4.49 (m, 2H), 1.95-1.85 (m, 2H), 1.60-1.53 (m, 11H), 1.03 (t, J = 7.2 Hz, 3H).
HRMS (ESI): m/z theoretical value: $C_{12}H_9N_3O_2[M+H]^+$ 326.1863, measured value: 326.1864.

Compound 48:

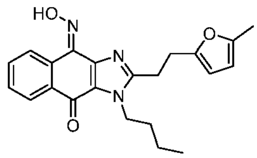

(E)-3-butyl-9-(hydroxylimino)-2-(2-(5-methylfuran-2-yl)ethyl)-3,9-dihydro-4H-napht hol [2,3-d] imidazole-4-one

$^1$H NMR (400 MHz, Chloroform-d) δ 13.87 (s, 1H), 8.42 (d, J = 7.8 Hz, 1H), 8.24 (d, J = 7.8 Hz, 1H), 7.67 - 7.62 (m, 1H), 7.60 - 7.53 (m, 1H), 5.91 (d, J = 3.0 Hz, 1H), 5.88 - 5.81 (m, 1H), 4.38 (t, J = 7.6 Hz, 2H), 3.24 - 3.07 (m, 3H), 2.25 (s, 3H), 1.90-1.83 (m, 1H), 1.78-1.67 (m, 2H), 1.41 (q, J = 7.6 Hz, 2H), 0.97 (t, J = 7.2 Hz, 3H).
HRMS (ESI): m/z theoretical value: $C_{12}H_9N_3O_2[M+H]^+$ 378.1812, measured value: 378.1813.

Compound 49:

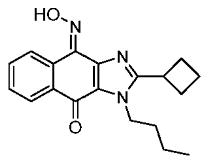

(E)-3-butyl-9-(hydroxylimino)-2-cyclobutyl-3,9-dihydro-4H-naphthol [2,3-d] imidazole-4-one

$^1$H NMR (400 MHz, Chloroform-d) δ 14.08 (s, 1H), 8.42 (d, J = 7.8 Hz, 1H), 8.29 - 8.19 (m, 1H), 7.65 (td, J = 8.2, 7.8, 1.6 Hz, 1H), 7.60 - 7.51 (m, 1H), 4.44 - 4.28 (m, 2H), 3.68 (q, J = 8.5 Hz, 1H), 2.62 - 2.52 (m, 2H), 2.44 (q, J = 8.9 Hz, 2H), 2.17 (q, J = 9.5 Hz, 1H), 2.09-2.04 (m, 1H), 1.76 (p, J = 7.8 Hz, 2H), 1.48-1.35 (m, 2H), 0.98 (t, J = 7.3 Hz, 3H).
HRMS (ESI): m/z theoretical value: $C_{12}H_9N_3O_2[M+H]^+$ 324.1707, measured value: 324.1709.

Compound 50:

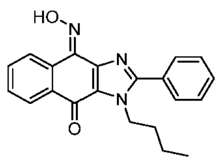

(E)-3-butyl-9-(hydroxylimino)-2-phenyl-3,9-dihydro-4H-naphthol [2,3-d] imidazole-4-one

$^1$H NMR (400 MHz, Chloroform-d) δ 13.92 (s, 1H), 8.46 (d, J = 7.8 Hz, 1H), 8.29 (d, J = 7.8 Hz, 1H), 7.69 (d, J = 7.4 Hz, 2H), 7.63-7.58 (m, 5H), 4.58 (t, J = 7.8 Hz, 2H), 1.86 (q, J = 7.6 Hz, 2H), 1.37 - 1.31 (m, 2H), 0.89 (t, J = 7.2 Hz, 3H).
HRMS (ESI): m/z theoretical value: $C_{12}H_9N_3O_2[M+H]^+$ 346.1550, measured value: 346.1554.

Compound 51:

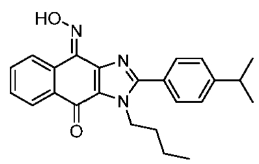

(E)-3-butyl-9-(hydroxylimino) -2-(4-isopropylphenyl)-3,9-dihydro-4H-naphthol [2,3-d] imidazole-4-one

$^1$H NMR (400 MHz, Chloroform-d) δ 13.96 (s, 1H), 8.44 (d, J = 7.8 Hz, 1H), 8.28 (dd, J = 78, 1.6 Hz, 1H), 7.93 - 7.54 (m, 4H), 7.52 - 7.31 (m, 2H), 4.57 (t, J = 7.8 Hz, 2H), 1.92-1.83 (m, 2H), 1.42-1.25 (m, 8H), 0.90 (t, J = 7.4 Hz, 3H).
HRMS (ESI): m/z theoretical value: $C_{12}H_9N_3O_2[M+H]^+$ 388.2020, measured value: 388.2024.

Compound 52:

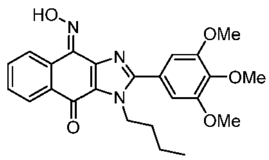

(E)-3-butyl-9-(hydroxylimino)-2-(3,4,5-trimethoxyphenyl)-3,9-dihydro-4H-naphthol [2,3-d] imidazole-4-one

$^1$H NMR (400 MHz, Chloroform-d) δ 13.85 (s, 1H), 8.45 (d, J = 7.8 Hz, 1H), 8.29 (d, J = 7.8 Hz, 1H), 7.68 (t, J = 8.0 Hz, 1H), 7.60 (t, J = 7.4 Hz, 1H), 6.88 (s, 2H), 4.63 - 4.52 (m, 2H), 3.94 (s, 9H), 1.92-1.86 (m, 2H), 1.40-1.34 (m, 2H), 0.93 (t, J = 7.4 Hz, 3H).

HRMS (ESI): m/z theoretical value: $C_{12}H_9N_3O_2[M+H]^+$ 436.1867, measured value: 436.1868.

Compound 53:

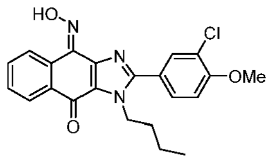

(E)-3-butyl-2-(3-chloro-4-methoxyphenyl)-9-(hydroxylimino)-3,9-dihydro-4H-napht hol [2,3-d] imidazole-4-one

$^1$H NMR (400 MHz, DMSO-$d_6$) δ 13.10 (s, 1H), 8.30 (d, J = 8.0 Hz, 1H), 8.16 (d, J = 7.6 Hz, 1H), 7.81 (s, 1H), 7.70 (d, J = 8.4 Hz, 2H), 7.64 (d, J = 7.6 Hz, 1H), 7.34 (d, J = 8.5 Hz, 1H), 4.48 (t, J = 8.4 Hz, 2H), 3.94 (s, 3H), 1.72-1.67 (m, 2H), 1.19-1.12 (m, 2H), 0.76 (t, J = 7.4 Hz, 3H).

HRMS (ESI): m/z theoretical value: $C_{12}H_9N_3O_2[M+H]^+$ 410.1266, measured value: 410.1266.

Compound 54:

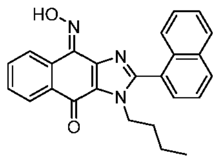

(E)-3-butyl-9-(hydroxylimino)-2-naphthyl-3,9-dihydro-4H-naphthol [2,3-d] imidazole-4-one

$^1$H NMR (400 MHz, Chloroform-d) δ 13.83 (s, 1H), 8.50 (dd, J = 7.8, 1.2 Hz, 1H), 8.34 (dd, J = 7.8, 1.6 Hz, 1H), 8.12 (dd, J = 7.2, 2.2 Hz, 1H), 8.01 (dd, J = 8.0, 1.8 Hz, 1H), 7.78 - 7.51 (m, 7H), 4.38 (t, J = 7.4 Hz, 2H), 1.67 (p, J = 7.6 Hz, 2H), 1.13 (h, J = 7.4 Hz, 2H), 0.69 (t, J = 7.4 Hz, 3H).

HRMS (ESI): m/z theoretical value: $C_{12}H_9N_3O_2[M+H]^+$ 396.1707, measured value: 396.1709.

Compound 55:

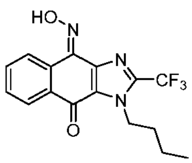

(E)-3-butyl-9-(hydroxylimino)-2-(trifluoromethyl)-3,9-dihydro-4H-naphthol [2,3-d] imidazole-4-one

$^1$H NMR (400 MHz, Chloroform-d) δ 12.85 (s, 1H), 8.41 (dd, J = 8.0, 1.2 Hz, 1H), 8.24 (dd, J = 7.8, 1.6 Hz, 1H), 7.69 (td, J = 7.8, 1.6 Hz, 1H), 7.59 (td, J = 7.6, 1.2 Hz, 1H), 4.72 - 4.54 (m, 2H), 1.88 (p, J = 8.0 Hz, 2H), 1.50 (h, J = 7.4 Hz, 2H), 1.01 (t, J = 7.4 Hz, 3H).

HRMS (ESI): m/z theoretical value: $C_{12}H_9N_3O_2[M+H]^+$ 338.1111, measured value: 338.1115.

Compound 56:

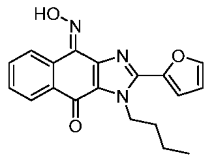

(E)-3-butyl-9-(hydroxylimino)-2-(furan-2-yl)-3,9-dihydro-4H-naphthol [2,3-d] imidazole-4-one

$^1$H NMR (400 MHz, Chloroform-d) δ 13.80 (s, 1H), 8.43 (d, J = 8.0 Hz, 1H), 8.28 (d, J = 7.8 Hz, 1H), 7.66 (d, J = 10.2 Hz, 2H), 7.61 - 7.55 (m, 1H), 7.24 (d, J = 4.8 Hz, 1H), 4.96 - 4.89 (m, 2H), 1.89 (q, J = 7.6 Hz, 2H), 1.47 (q, J = 7.6 Hz, 2H), 0.99 (t, J = 7.2 Hz, 3H).

HRMS (ESI): m/z theoretical value: $C_{12}H_9N_3O_2[M+H]^+$ 336.1343, measured value: 336.1344.

Compound 57:

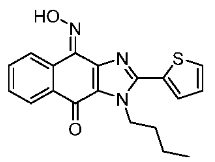

(E)-3-butyl-9-(hydroxylimino)-2-(thiophene-2-yl)-3,9-dihydro-4H-naphthol [2,3-d] imidazole-4-one

$^1$H NMR (400 MHz, Chloroform-d) δ 13.78 (s, 1H), 8.44 (d, J = 8.0 Hz, 1H), 8.34 - 8.13 (m, 1H), 7.70 - 7.52 (m, 4H), 7.24 (s, 1H), 4.82 - 4.71 (m, 2H), 1.95 (p, J = 8.2 Hz, 2H), 1.53 - 1.45 (m, 2H), 1.01 (t, J = 7.4 Hz, 3H).

HRMS (ESI): m/z theoretical value: $C_{12}H_9N_3O_2[M+H]^+$ 352.1114, measured value: 352.1116.

Compound 58:

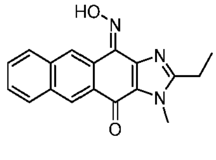

(E)-2-ethyl-11-(hydroxylimino)-3-methyl-3,11-dihydro-4H-anthraquinone [2,3-d] imidazole-4-one

$^1$H NMR (400 MHz, DMSO-$d_6$) δ 13.71 (s, 1H), 8.60 (d, J = 5.6 Hz, 2H), 8.21-8.15 (m, 2H), 7.71-7.65 (m, 2H), 2.76 (q, J = 7.6 Hz, 2H), 1.27 (t, J = 7.6 Hz, 2H), 4.09 (s, 1H).

HRMS (ESI): m/z theoretical value: $C_{12}H_9N_3O_2[M+H]^+$ 306.1237, measured value: 306.1236.

Compound 59:

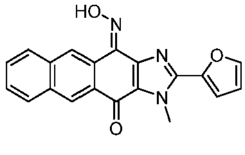

(E)-2-(furan-2-yl)-11-(hydroxylimino)-3-methyl-3,11-dihydro-4H-anthraquinone [2,3-d] imidazole-4-one

$^1$H NMR (400 MHz, DMSO-$d_6$) δ 13.85 (s, 1H), 8.69 (s, 2H), 8.27-8.22 (m, 2H), 7.95 (d, J = 1.2 Hz, 1H), 7.76-7.70 (m, 2H), 7.35 (d, J = 3.4 Hz, 1H), 6.75-6.70 (m, 1H), 4.25 (s, 1H).

HRMS (ESI): m/z theoretical value: $C_{12}H_9N_3O_2[M+H]^+$ 344.1030, measured value: 344.1031.

**Example 6**

2,3-Diamine-1,4-benzoquinone (20 g) was dissolved into 100 mL of formic acid, subjected to reflux reaction for 6 h, and cooled to room temperature, then poured into an ice-water mixture with the addition of ammonium hydroxide to regulate pH to 9-10, and the obtained solution was subjected to suction filtration and vacuum dried to obtain yellow solid 1,4-naphthoquinone imidazole (22 g). 1,4-naphthoquinone imidazole (0.3 g) and potassium hydroxide (0.15 g) were added to dimethyl sulfoxide solution (3 mL); the mixture was stirred at 50°C for 30 min and added with iodomethane (0.2 mL), after reaction for 2, water was added to separate out solid; the solid was subjected to suction filtration, washed with water, and vacuum dried to obtain yellow solid 2-methyl-1,4-naphthoquinone imidazole (0.31 g). 2-methylnaphthoquinone imidazole (0.2 g), methoxyamine hydrochloride (0.1 g), and pyridine (0.08 mL) were dissolved into ethanol (30 mL) and reacted for 12 h at 125°C; at the end of reaction, solid was separated out from solution, directly subjected to suction filtration, and vacuum dried to obtain light yellow solid (0.15 g).

2,3-Diamine-1,4-naphthoquinone (20 g) was dissolved into 100 mL of formic acid and subjected to reflux reaction for 6 h to obtain anthraquinone imidazole; or aminoxyhydrochlorides having different substitutes were added to prepare Compounds 60-77 according to the method in Example 6; other materials and reaction conditions for the compounds were kept the same and raw materials could be purchased commercially:

Compound 60:

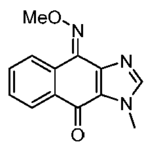

(E) and (Z)-9-(methoxyimino)-3-methyl-3,9-dihydro-4H-naphthol [2,3-d] imidazole-4-one

$^1$H NMR (400 MHz, DMSO-$d_6$) δ 8.99 (dd, J = 8.0, 1.2 Hz, 1H), 8.31-8.20 (m, 4H), 8.16-8.07 (m, 2H), 7.82-7.60 (m, 5H), 4.24 (s, 3H), 4.20 (s, 4H), 4.03 (s, 4H), 3.99 (s, 3H).

$^{13}$C NMR (101 MHz, DMSO) δ 175.40, 174.52, 145.91, 145.85, 145.58, 140.34, 139.95, 139.03, 133.69, 133.20, 133.12, 132.39, 131.62, 131.20, 131.09, 129.94, 128.10, 128.03, 127.04, 126.43, 126.16, 124.33, 64.73, 64.47, 34.20, 33.92.

HRMS (ESI): m/z theoretical value: $C_{13}H_{11}N_3O_2[M+H]^+$ 242.0924, measured value: 242.0928.

Compound 61:

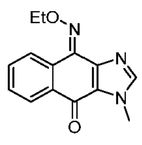

(E) and (Z)-9-(ethoxyimino)-3-methyl-3,9-dihydro-4H-naphthol [2,3-d] imidazole-4-one

$^1$H NMR (400 MHz, DMSO-$d_6$) δ 9.04 (dd, J = 8.0, 1.2 Hz, 1H), 8.30 (dd, J = 8.0, 1.2 Hz, 2H), 8.26-8.20 (m, 3H), 8.14-8.07 (m, 3H), 7.79 (td, J = 8.0, 1.6 Hz, 1H), 7.75-7.68 (m, 3H), 7.63 (td, J = 8.0, 1.2 Hz, 2H), 4.53-4.42 (m, 6H), 4.04 (s, 6H), 4.00 (s, 3H), 1.46-1.32 (m, 9H).

$^{13}$C NMR (101 MHz, DMSO) δ 175.42, 174.53, 146.10, 145.90, 145.64, 140.16, 139.75, 139.33, 133.66, 133.50, 133.04, 132.38, 131.51, 131.07, 129.79, 128.08, 128.04, 127.00, 126.37, 126.10, 124.33, 124.09, 72.72, 72.20, 34.16, 33.90, 15.29, 15.04.

HRMS (ESI): m/z theoretical value: $C_{14}H_{13}N_3O_2[M+H]^+$ 256.1081, measured value: 256.1085.

Compound 62:

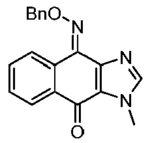

(E) and (Z)-9-(benzyloxyimino)-3-methyl-3,9-dihydro-4H-naphthol [2,3-d] imidazole-4-one

$^1H$ NMR (400 MHz, DMSO-$d_6$) δ 8.30-8.22 (m, 2H), 8.11 (dd, J = 8.0, 1.2 Hz, 1H), 7.71 (td, J = 8.0, 1.6 Hz, 1H), 7.64 (td, J = 8.0, 1.2 Hz, 1H), 7.55-7.49 (m, 2H), 7.45-7.31 (m, 3H), 5.50 (s, 2H), 4.03 (s, 3H).

Compound 63:

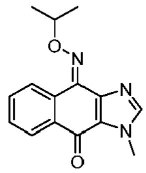

(E) and (Z)-9-(isopropoxyimino)-3-methyl-3,9-dihydro-4H-naphthol [2,3-d] imidazole-4-one

$^1H$ NMR (400 MHz, DMSO-$d_6$) δ 9.07 (dd, J = 8.0, 1.2 Hz, 1H), 8.33 (dd, J = 8.0, 1.2 Hz, 1H), 8.28-8.21 (m, 2H), 8.16-8.10 (m, 2H), 7.80 (td, J = 8.0, 1.6 Hz, 1H), 7.72 (td, J = 8.0, 1.4 Hz, 2H), 7.63 (td, J = 8.0, 1.2 Hz, 1H), 4.72-4.60 (m, 2H), 4.04 (s, 3H), 4.00 (s, 3H), 1.43 (dd, J = 12.0, 8.0 Hz, 12H).

$^{13}C$ NMR (101 MHz, DMSO) δ 175.44, 174.53, 146.27, 145.91, 145.69, 139.80, 139.47, 139.28, 133.77, 133.66, 133.03, 132.39, 131.42, 131.07, 130.99, 129.68, 128.14, 128.00, 127.00, 126.32, 126.07, 124.33, 79.35, 78.63, 34.16, 33.91, 22.10, 21.97.

HRMS (ESI): m/z theoretical value: $C_{15}H_{15}N_3O_2[M+H]^+$ 270.1237, measured value: 270.1238.

Compound 64:

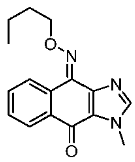

(E) and (Z)-9-(butoxyimino)-3-methyl-3,9-dihydro-4H-naphthol [2,3-d] imidazole-4-one

$^1H$ NMR (400 MHz, DMSO-$d_6$) δ 9.03 (dd, J = 8.0, 1.2 Hz, 2H), 8.30 (dd, J = 8.0, 1.2 Hz, 1H), 8.28- 8.21 (m, 4H), 8.14 (s, 3H), 7.87-7.78 (m, 3H), 7.76-7.69 (m, 3H), 7.64 (td, J = 8.0, 1.2 Hz, 1H), 4.48-4.38 (m, 7H), 4.04 (s, 3H), 4.00 (s, 7H), 1.85-1.72 (m, 7H), 1.53-1.39 (m, 7H), 1.00-0.92 (m, 10H).

$^{13}C$ NMR (101 MHz, DMSO) δ 175.45, 174.54, 146.12, 145.96, 145.75, 140.23, 139.79, 139.45, 133.74, 133.53, 133.09, 132.41, 131.42, 131.14, 129.82, 128.11, 128.07, 127.07, 126.92, 126.40, 126.14, 124.34, 76.88, 76.36, 34.17, 33.92, 31.53, 31.20, 19.23, 19.08, 14.25, 14.23.

HRMS (ESI): m/z theoretical value: $C_{16}H_{17}N_3O_2[M+H]^+$ 284.1394, measured value: 284.1398.

Compound 65:

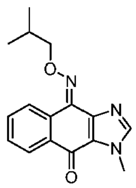

(E) and (Z)-9-(isobutoxyimino)-3-methyl-3,9-dihydro-4H-naphthol [2,3-d] imidazole-4-one

$^1$H NMR (400 MHz, DMSO-$d_6$) δ 9.04 (dd, J = 8.1, 1.2 Hz, 1H), 8.33-8.23 (m, 4H), 8.15-8.09 (m, 3H), 7.82 (td, J = 8.0, 1.6 Hz, 1H), 7.75-7.69 (m, 2H), 7.64 (td, J = 8.0, 1.2 Hz, 2H), 4.25 (d, J = 8.0 Hz, 2H), 4.21 (d, J = 8.0 Hz, 3H), 4.04 (s, 5H), 4.00 (s, 3H), 2.20-2.09 (m, 3H), 1.01 (t, J = 6.0 Hz, 16H).

$^{13}$C NMR (101 MHz, DMSO) δ 175.44, 174.52, 146.10, 145.95, 145.77, 140.24, 139.84, 139.48, 133.73, 133.50, 133.07, 132.42, 131.34, 131.14, 131.10, 129.80, 128.08, 127.09, 126.40, 126.13, 124.32, 83.43, 82.87, 34.16, 33.92, 28.50, 28.21, 19.45, 19.42.

HRMS (ESI): m/z theoretical value: $C_{16}H_{17}N_3O_2[M+H]^+$ 284.1394, measured value: 284.1397.

Compound 66:

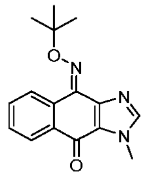

(E) and (Z)-9-(tert-butoxyimino)-3-methyl-3,9-dihydro-4H-naphthol [2,3-d] imidazole-4-one

$^1$H NMR (400 MHz, DMSO-$d_6$) δ 9.10 (dd, J = 8.0, 1.2 Hz, 1H), 8.38 (dd, J = 8.0, 1.2 Hz, 1H), 8.27- 8.21 (m, 2H), 8.15-8.10 (m, 2H), 7.83-7.60 (m, 5H), 4.05 (s, 3H), 4.01 (s, 4H), 1.50 (s, 12H), 1.48 (s, 9H).

$^{13}$C NMR (101 MHz, DMSO) δ 175.47, 174.56, 146.58, 145.91, 145.70, 139.61, 139.39, 138.81, 134.17, 133.61, 133.01, 132.40, 131.32, 131.05, 130.83, 129.52, 128.22, 127.95, 126.95, 126.23, 126.02, 124.29, 82.90, 82.08, 34.15, 33.90, 28.02, 27.74.

HRMS (ESI): m/z theoretical value: $C_{16}H_{17}N_3O_2[M+H]^+$ 284.1394, measured value: 284.1393.

Compound 67:

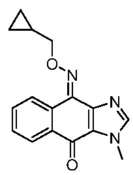

(E) and (Z)-9-((cyclopropoxy)imino)-3-methyl-3,9-dihydro-4H-naphthol [2,3-d] imidazole-4-one

$^1$H NMR (400 MHz, DMSO-$d_6$) δ 9.10 (dd, J = 8.0, 1.2 Hz, 1H), 8.33-8.24 (m, 4H), 8.16- 8.09 (m, 3H), 7.84-7.77 (m, 1H), 7.75-7.70 (m, 2H), 7.64 (td, J = 8.0, 1.2 Hz, 2H), 4.32-4.24 (m, 5H), 4.04 (s, 5H), 4.00 (s, 3H), 1.36-1.26 (m, 3H), 0.65-0.55 (m, 6H), 0.48-0.35 (m, 5H).

$^{13}$C NMR (101 MHz, DMSO) δ 175.45, 174.54, 146.15, 145.95, 145.72, 140.08, 139.62, 139.41, 133.69, 133.58, 133.07, 132.40, 131.50, 131.11, 131.09, 129.77, 128.17, 128.06, 127.04, 126.39, 126.12, 124.35, 81.62, 80.98, 34.16, 33.91, 11.04, 10.65, 3.64, 3.59.

HRMS (ESI): m/z theoretical value: $C_{16}H_{15}N_3O_2[M+H]^+$ 282.1237, measured value: 282.1238.

Compound 68:

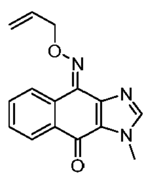

(E) and (Z)-9-((allyloxy)imino)-3-methyl-3,9-dihydro-4H-naphthol [2,3-d] imidazole-4-one

$^1$H NMR (400 MHz, DMSO-$d_6$) δ 9.03 (dd, J = 8.0, 1.2 Hz, 1H), 8.30-8.21 (m, 6H), 8.15-8.08 (m, 4H), 7.80 (td, J = 8.0, 1.6 Hz, 1H), 7.77-7.65 (m, 4H), 7.64 (td, J = 8.0, 1.2 Hz, 3H), 6.22-6.08 (m, 4H), 5.55-5.40 (m, 4H), 5.36-5.27 (m, 4H), 5.00-4.92 (m, 7H), 4.04 (s, 8H), 3.99 (s, 3H).

$^{13}$C NMR (101 MHz, DMSO) δ 175.43, 174.51, 145.94, 145.72, 140.65, 140.24, 139.31, 135.14, 134.58, 134.13, 133.71, 133.34, 133.09, 132.42, 131.59, 131.22, 131.12, 129.93, 128.15, 128.05, 127.08, 126.48, 126.15, 124.37, 119.04, 118.54, 77.72, 77.22, 34.16, 33.92.

HRMS (ESI): m/z theoretical value: $C_{15}H_{13}N_3O_2[M+H]^+$ 268.1081, measured value: 268.1083.

Compound 69:

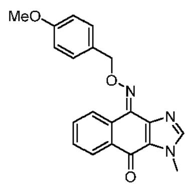

(E) and (Z)-3-methyl-9-(((4-methoxybenzyl)oxy)imino)-3,9-dihydro-4H-naphthol [2,3-d] imidazole-4-one

$^1$H NMR (400 MHz, DMSO-$d_6$) δ 9.05-8.98 (m, 1H), 8.34-8.27 (m, 3H), 8.27-8.20 (m, 4H), 8.15-8.09 (m, 4H), 7.76-7.61 (m, 8H), 7.49-7.43 (m, 8H), 6.99-6.93 (m, 8H), 5.44 (s, 2H), 5.41 (s, 6H), 4.03 (s, 9H), 3.99 (s, 3H), 3.76 (s, 3H), 3.75 (s, 9H).

$^{13}$C NMR (101 MHz, DMSO) δ 175.43, 174.52, 159.81, 159.60, 145.98, 145.75, 140.52, 140.16, 139.34, 133.72, 133.41, 133.13, 132.42, 131.50, 131.19, 131.13, 130.65, 130.54, 129.93, 129.82, 129.17, 128.15, 128.09, 127.08, 126.48, 126.16, 124.38, 114.40, 114.24, 78.57, 78.04, 55.58, 55.55, 34.16, 33.92.

HRMS (ESI): m/z theoretical value: $C_{20}H_{17}N_3O_3[M+H]^+$ 348.1343, measured value: 348.1339.

Compound 70:

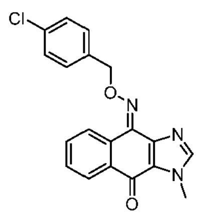

(E) and (Z)-3-methyl-9-(((4-chlorobenzyl)oxy)imino)-3,9-dihydro-4H-naphthol [2,3-d] imidazole-4-one

$^1$H NMR (400 MHz, DMSO-$d_6$) δ 9.03 (dd, J = 8.0, 1.2 Hz, 1H), 8.28-8.22 (m, 5H), 8.14-8.08 (m, 3H), 7.82-7.60 (m, 7H), 7.57-7.43 (m, 13H), 5.51 (s, 2H), 5.49 (s, 4H), 4.03 (s, 6H), 3.99 (s, 3H).

$^{13}$C NMR (101 MHz, DMSO) δ 175.42, 174.50, 145.97, 145.80, 140.99, 140.68, 139.26, 137.10, 136.54, 133.77, 133.30, 133.21, 133.16, 132.98, 132.47, 131.63, 131.33, 131.31, 131.16, 130.52, 130.32, 130.06, 129.03, 128.87, 128.27, 128.03, 127.13, 126.55, 126.20, 124.40, 77.67, 77.17, 34.17, 33.92.

HRMS (ESI): m/z theoretical value: $C_{19}H_{14}ClN_3O_2[M+H]^+$ 352.0847, measured value: 352.0843.

Compound 71:

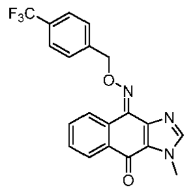

(E) and (Z)-3-methyl-9-(((4-trifluoromethyl benzyl)oxy)imino)-3,9-dihydro-4H-naphthol [2,3-d] imidazole-4-one

yellow solid, 100mg, % yield, m. p. 136-138 °C.

$^{1}H$ NMR (400 MHz, DMSO-$d_6$) δ 9.07 (dd, J = 8.0, 1.2 Hz, 1H), 8.28-8.21 (m, 4H), 8.14-8.07 (m, 3H), 7.82-7.60 (m, 17H), 5.63 (s, 2H), 5.60 (s, 3H), 4.04 (s, 5H), 3.99 (s, 3H).

$^{13}C$ NMR (101 MHz, DMSO) δ 175.40, 174.48, 145.95, 145.80, 145.73, 142.98, 142.48, 141.24, 140.95, 139.23, 133.78, 133.15, 133.11, 132.48, 131.70, 131.35, 131.16, 130.09, 128.94, 128.74, 128.62, 128.31, 128.00, 127.13, 126.57, 126.19, 126.07, 125.95, 125.91, 125.87, 125.83, 125.79, 125.76, 125.72, 125.68, 124.39, 123.37, 77.49, 77.04, 34.17, 33.90.

HRMS (ESI): m/z theoretical value: $C_{20}H_{14}F_3N_3O_2[M+H]^+$ 386.1111, measured value: 386.1107.

Compound 72:

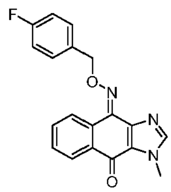

(E) and (Z)-3-methyl-9-(((4-fluorobenzyl)oxy)imino)-3,9-dihydro-4H-naphthol [2,3-d] imidazole-4-one

$^{1}H$ NMR (400 MHz, DMSO-$d_6$) δ 9.03 (dd, J = 8.0, 1.4 Hz, 1H), 8.33-8.20 (m, 5H), 8.18-8.09 (m, 3H), 7.79-7.53 (m, 12H), 7.30-7.17 (m, 7H), 5.50 (s, 2H), 5.48 (s, 3H), 4.03 (s, 5H), 3.99 (s, 3H).

$^{13}C$ NMR (101 MHz, DMSO) δ 175.43, 174.51, 163.71, 163.54, 161.12, 145.98, 145.88, 145.79, 140.85, 140.53, 139.28, 134.26, 134.23, 133.76, 133.72, 133.69, 133.27, 133.16, 132.46, 131.59, 131.28, 131.16, 131.06, 130.98, 130.84, 130.76, 130.03, 129.51, 128.24, 128.04, 127.12, 126.54, 126.19, 124.40, 115.96, 115.79, 115.75, 115.57, 77.83, 77.35, 34.17, 33.92.

HRMS (ESI): m/z theoretical value: $C_{19}H_{14}FN_3O_2[M+H]^+$ 336.1143, measured value: 336.1140.

Compound 73:

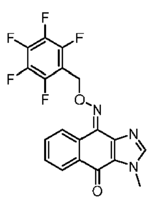

(E) and (Z)-3-methyl-9-(((perfluorobenzyl)oxy)imino)-3,9-dihydro-4H-naphthol [2,3-d] imidazole-4-one

yellow solid, 100mg, % yield, m. p. 224-226 °C.

$^{1}H$ NMR (400 MHz, DMSO-$d_6$) δ 8.92 (dd, J = 8.0, 1.2 Hz, 1H), 8.26-8.07 (m, 7H), 7.83-7.71 (m, 4H), 7.65 (td, J = 8.0, 1.2 Hz, 2H), 5.61 (s, 2H), 5.53 (s, 3H), 4.02 (s, 5H), 3.98 (s, 3H).

$^{13}C$ NMR (101 MHz, DMSO) δ 175.39, 174.49, 146.06, 145.82, 145.43, 144.63, 141.75, 141.24, 138.94, 133.86, 133.81, 133.32, 132.90, 132.55, 131.67, 131.67, 131.59, 131.23, 130.34, 130.11, 128.49, 128.46, 127.93, 127.87, 127.20, 126.26, 124.69, 124.25, 65.48, 64.75, 34.17, 33.93.

HRMS (ESI): m/z theoretical value: $C_{19}H_{10}F_5N_3O_2[M+H]^+$ 408.0766, measured value: 408.0767.

Compound 74:

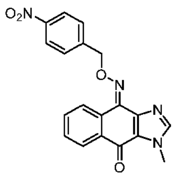

(E) and (Z)-3-methyl-9-(((4-nitrobenzyl)oxy)imino)-3,9-dihydro-4H-naphthol [2,3-d] imidazole-4-one

$^1H$ NMR (400 MHz, DMSO-$d_6$) δ 9.09 (dd, J = 8.0, 1.2 Hz, 1H), 8.31-8.17 (m, 14H), 8.13-8.09 (m, 3H), 7.84-7.62 (m, 15H), 5.67 (s, 2H), 5.65 (s, 5H), 4.08-4.02 (m, 8H), 3.99-3.97 (m, 3H).

$^{13}C$ NMR (101 MHz, DMSO) δ 175.42, 174.50, 147.64, 147.47, 146.09, 145.97, 145.85, 145.66, 145.55, 141.45, 141.20, 139.20, 133.85, 133.21, 133.02, 132.51, 131.79, 131.46, 131.18, 130.20, 129.18, 128.95, 128.37, 127.98, 127.19, 126.62, 126.24, 124.43, 124.15, 124.03, 77.09, 76.65, 34.20, 33.93.

HRMS (ESI): m/z theoretical value: $C_{19}H_{14}N_4O_4[M+H]^+$ 363.1088, measured value: 363.1084.

Compound 75:

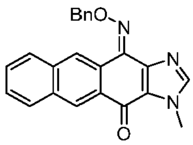

(E) and (Z)-11-(benzyloxyimino)-3-methyl-3,11-dihydro-4H-anthraquinone [2,3-d] imidazole-4-one

$^1H$ NMR (400 MHz, DMSO-$d_6$) δ 9.67 (s, 1H), 8.79 (d, J = 6.8 Hz, 2H), 8.73 (s, 1H), 8.27 (s, 1H), 8.23 - 8.08 (m, 6H), 8.00 - 7.95 (m, 1H), 7.73 - 7.55 (m, 11H), 7.46 - 7.33 (m, 8H), 5.57 (s, 2H), 5.53 (s, 3H), 4.06 (s, 5H), 4.01 (s, 3H).

HRMS (ESI): m/z theoretical value: $C_{19}H_{14}N_4O_4[M+H]^+$ 368.1394, measured value: 368.1397.

Compound 76:

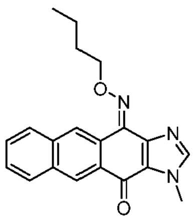

(E) and (Z)-11-(butoxyimino)-3-methyl-3,11-dihydro-4H-anthraquinone [2,3-d] imidazole-4-one

$^1H$ NMR (400 MHz, DMSO-$d_6$) δ 9.58 (s, 1H), 8.75 (d, J = 2.1 Hz, 2H), 8.69 (s, 1H), 8.24 (s, 1H), 8.21 - 8.09 (m, 5H), 8.02 - 7.98 (m, 1H), 7.73 - 7.59 (m, 5H), 4.45 (dt, J = 20.6, 6.6 Hz, 5H), 4.05 (s, 3H), 4.01 (s, 4H), 1.90-1.75 (m, 5H), 1.57-1.45 (m, 5H), 1.05-0.96 (m, 7H).

HRMS (ESI): m/z theoretical value: $C_{19}H_{14}N_4O_4[M+H]^+$ 334.2210, measured value: 334.2214.

Compound 77:

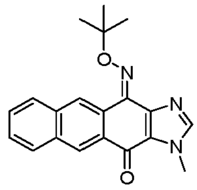

(E) and (Z)-11-(tert-butoxyimino)-3-methyl-3,11-dihydro-4H-anthraquinone[2,3-d] imidazole-4-one

$^1$H NMR (400 MHz, DMSO-$d_6$) δ 9.70 (s, 1H), 8.87 (s, 1H), 8.81 (s, 1H), 8.74 (s, 1H), 8.28 - 8.13 (m, 5H), 8.07 - 8.02 (m, 1H), 7.75 - 7.60 (m, 4H), 4.08 (s, 3H), 4.04 (s, 3H), 1.55 (s, 9H), 1.52 (s, 9H).
HRMS (ESI): m/z theoretical value: $C_{19}H_{14}N_4O_4[M+H]^+$ 334.2210, measured value: 334.2213.

## Example 7

2,3-Diamine-1,4-benzoquinone (20 g) was dissolved into 100 mL of formic acid, subjected to reflux reaction for 6 h, and cooled to room temperature, then poured into an ice-water mixture with the addition of ammonium hydroxide to regulate pH to 9-10, and the obtained solution was subjected to suction filtration and vacuum dried to obtain yellow solid 1,4-naphthoquinone imidazole (22 g). 1,4-naphthoquinone imidazole (0.3 g) and potassium hydroxide (0.15 g) were added to dimethyl sulfoxide solution (3 mL); the mixture was stirred at 50°C for 30 min and added with iodomethane (0.2 mL), after reaction for 2, water was added to separate out solid; the solid was subjected to suction filtration, washed with water, and vacuum dried to obtain yellow solid 2-methyl-1,4-naphthoquinone imidazole (0.31 g). 2-methylnaphthoquinone imidazole (0.2 g), hydroxylamine hydrochloride (0.3 g), and pyridine (0.7 mL) were dissolved into ethanol (70 mL) and reacted for 36 h at 105°C; at the end of reaction, solid was separated out from solution, directly subjected to suction filtration, and vacuum dried to obtain light yellow solid (E)-9-(hydroxylimino)-3-methyl-3,6,7,9-tetrahydro-4H-naphthol [2,3-d] imidazole-4-one (0.15 g). 106 mg of oximate product was added to 2 mL of a dimethyl sulfoxide solution; 56 μL of bromoethyl methyl ether and 104 mg of potassium hydroxide were added successively, and these materials were reacted for 2 h at room temperature. At the end of the reaction, water was added for quenching and dichloromethane was used for extraction; organic phase was washed with a saturated salt solution, dried with anhydrous magnesium sulfate, and concentrated, then isolated by silica gel column chromatography.
The oximated product was reacted with halohydrocarbons having different substitutes to prepare Compounds 78-82 according to the method in Example 7; other materials and reaction conditions for the compounds were kept the same and raw materials could be purchased commercially:

Compound 78:

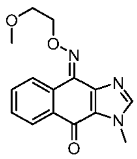

(E)-9-((2-methoxyethoxy)imino)-3-methyl-3,9-dihydro-4H-naphthol [2,3-d] imidazole-4-one

$^1$H NMR (400 MHz, DMSO-$d_6$) δ 8.34-8.24 (m, 2H), 8.16-8.09 (m, 1H), 7.76-7.70 (m, 1H), 7.65 (td, J = 8.0, 1.2 Hz, 1H), 4.53 (t, J = 4.0 Hz, 2H), 4.04 (s, 3H), 3.76 (t, J = 4.0 Hz, 2H), 3.32 (s, 3H).
$^{13}$C NMR (101 MHz, DMSO) δ 175.46, 145.74, 140.04, 139.32, 133.40, 133.14, 131.13, 129.95, 128.15, 126.17, 124.43, 76.07, 70.75, 58.76, 34.18.
HRMS (ESI): m/z theoretical value: $C_{15}H_{15}N_3O_3[M+H]^+$ 286.1186, measured value: 286.1188.

Compound 79:

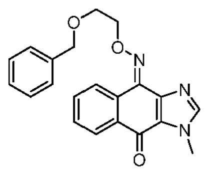

(E) and (Z)-9-((2-(benzyloxy)ethoxy)imino)-3-methyl-3,9-dihydro-4H-naphthol [2,3-d] imidazole-4-one

$^1$H NMR (400 MHz, DMSO-$d_6$) δ 8.30-8.25 (m, 2H), 8.15-8.10 (m, 1H), 7.75-7.61 (m, 2H), 7.38-7.31 (m, 2H), 7.30-7.21 (m, 3H), 4.63-4.54 (m, 4H), 4.04 (s, 3H), 3.89-3.82 (m, 2H).
$^{13}$C NMR (101 MHz, DMSO-$d_6$) δ 175.45, 145.72, 140.12, 139.36, 138.95, 133.39, 133.08, 131.13, 129.92, 128.60, 128.15, 127.86, 127.74, 126.14, 124.43, 76.28, 72.40, 68.47, 34.18.
HRMS (ESI): m/z theoretical value: $C_{21}H_{19}N_3O_3[M+H]^+$ 362.1499, measured value: 362.1495.

Compound 80:

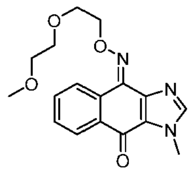

(E)-9-((2-(2-methoxyethoxy)ethoxy)imino)-3-methyl-3,9-dihydro-4H-naphthol [2,3-d] imidazole-4-one

$^1$H NMR (400 MHz, DMSO-$d_6$) δ 8.31 (dd, J = 8.0, 1.2 Hz, 1H), 8.24 (s, 1H), 8.13 (dd, J = 8.0, 1.6 Hz, 1H), 7.72 (td, J = 8.0, 1.6 Hz, 1H), 7.65 (td, J = 8.0, 1.4 Hz, 1H), 4.55-4.49 (m, 2H), 4.04 (s, 3H), 3.86 -3.80 (m, 2H), 3.64-3.59 (m, 2H), 3.46-3.42 (m, 2H), 3.22 (s, 3H).
$^{13}$C NMR (101 MHz, DMSO-$d_6$) δ 175.45, 145.72, 140.07, 139.31, 133.40, 133.13, 131.13, 129.95, 128.15, 126.16, 124.45, 76.25, 71.78, 70.26, 69.26, 58.52, 34.18.
HRMS (ESI): m/z theoretical value: $C_{17}H_{19}N_3O_4[M+H]^+$ 330.1448, measured value: 330.1447.

Compound 81:

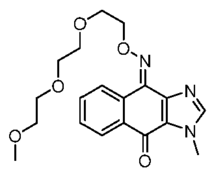

(E) and (Z)-9-((2-(2-(2-methoxyethoxy)ethoxy)ethoxy)imino)-3-methyl-3,9-dihydro-4H-naphthol [2,3-d] imidazole-4-one

$^1$H NMR (400 MHz, DMSO-$d_6$) δ 8.30 (dd, J = 8.0, 1.2 Hz, 1H), 8.23 (s, 1H), 8.14-8.10 (m, 1H), 7.74-7.69 (m, 1H), 7.65 (td, J = 8.0, 1.2 Hz, 1H), 4.55-4.51 (m, 2H), 4.04 (s, 3H), 3.87-3.82 (m, 2H), 3.65-3.59 (m, 2H), 3.54-3.47 (m, 4H), 3.39-3.35 (m, 2H), 3.19 (s, 3H).
$^{13}$C NMR (101 MHz, DMSO-$d_6$) δ 175.44, 145.70, 140.04, 139.29, 133.39, 133.11, 131.10, 129.93, 128.12, 126.14, 124.43, 76.23, 71.70, 70.48, 70.24, 70.03, 69.26, 58.46, 34.16.
HRMS (ESI): m/z theoretical value: $C_{19}H_{23}N_3O_5[M+H]^+$ 374.1710, measured value: 374.1709.

Compound 82:

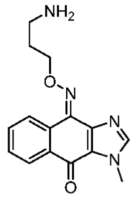

(E)-9-((3-aminopropoxy)imino)-3-methyl-3,9-dihydro-4H-naphthol [2,3-d] imidazole-4-one

$^1$H NMR (400 MHz, DMSO-$d_6$) δ 8.30-8.25 (m, 2H), 8.15-8.10 (m, 1H), 7.75-7.61 (m, 2H), 4.04 (s, 3H), 3.51 (t, J = 8.0 Hz, 2H), 2.68 (t, J = 8.0 Hz, 2H),1.79-1.70 (m, 2H).

HRMS (ESI): m/z theoretical value: $C_{19}H_{23}N_3O_5$[M+H]$^+$ 285.1346, measured value: 285.1347.

**Example 8**

2,3-Diamine-1,4-benzoquinone (20 g) was dissolved into 100 mL of formic acid, subjected to reflux reaction for 6 h, and cooled to room temperature, then poured into an ice-water mixture with the addition of ammonium hydroxide to regulate pH to 9-10, and the obtained solution was subjected to suction filtration and vacuum dried to obtain yellow solid 1,4-naphthoquinone imidazole (22 g). 1,4-naphthoquinone imidazole (0.3 g) and potassium hydroxide (0.15 g) were added to dimethyl sulfoxide solution (3 mL), respectively; the mixture was stirred at 50°C for 30 min and added with iodobutane (0.18 mL), after reaction for 5 h, water was added to separate out solid; the solid was subjected to suction filtration, washed with water, and vacuum dried to obtain yellow solid 2-butyl-1,4-naphthoquinone imidazole (0.31 g). 2-butylnaphthoquinone imidazole (0.21 g), methoxyamine hydrochloride (0.3 g), and pyridine (0.7 mL) were dissolved into ethanol (70 mL) and reacted for 36 h at 105°C; at the end of reaction, the obtained solution was concentrated and isolated by silica gel column chromatography to obtain light yellow product (0.18 g).

2,3-Diamine-1,4-naphthoquinone (20 g) was dissolved into 100 mL of formic acid and subjected to reflux reaction for 6 h to obtain anthraquinone imidazole; halohydrocarbons having different substitutes or aminoxyhydrochlorides having different substitutes were used to prepare Compounds 83-86 according to the method in Example 8; other materials and reaction conditions for the compounds were kept the same and raw materials could be purchased commercially:

Compound 83:

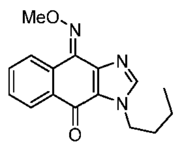

(E) and (Z) -3-butyl-9-(methoxyimino)-3,9-dihydro-4H-naphthol [2,3-d] imidazole-4-one

$^1$H NMR (400 MHz, DMSO-$d_6$) δ 9.00 (dd, J = 8.0, 1.2 Hz, 1H), 8.34-8.21 (m, 4H), 8.13 (dd, J = 8.0, 1.4 Hz, 1H), 7.82-7.62 (m, 4H), 4.46 (t, J = 7.2 Hz, 2H), 4.40 (t, J = 7.0 Hz, 2H), 4.24 (s, 3H), 4.21 (s, 3H), 1.84-1.72 (m, 4H), 1.37-1.18 (m, 4H), 0.91 (t, J = 8.0 Hz, 6H).

$^{13}$C NMR (101 MHz, DMSO) δ 175.14, 174.26, 146.33, 145.50, 145.23, 140.43, 140.02, 139.65, 133.69, 133.17, 133.10, 132.43, 131.59, 131.20, 131.15, 129.93, 127.94, 127.51, 127.14, 126.25, 125.85, 124.31, 64.75, 64.49, 46.53, 46.39, 32.75, 19.52, 19.50, 13.88.

HRMS (ESI): m/z theoretical value: $C_{16}H_{17}N_3O_2$[M+H]$^+$ 284.1394, measured value: 284.1394.

Compound 84:

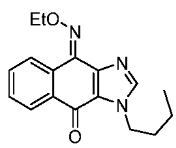

(E) and (Z) -3-butyl-9-(ethoxyimino)-3,9-dihydro-4H-naphthol [2,3-d] imidazole-4-one

$^1$H NMR (400 MHz, DMSO-$d_6$) δ 9.06 (d, J = 8.0 Hz, 1H), 8.37-8.19 (m, 6H), 8.14 (d, J = 8.0 Hz, 2H), 7.83-7.61 (m, 6H), 4.55-4.36 (m, 11H), 1.82-1.72 (m, 6H), 1.46-1.38 (m, 9H), 1.35-1.20 (m, 7H), 0.91 (t, J = 8.0 Hz, 9H).
$^{13}$C NMR (101 MHz, DMSO) δ 175.16, 174.27, 146.52, 145.50, 145.27, 140.23, 139.77, 133.69, 133.43, 133.07, 132.42, 131.50, 131.13, 129.82, 128.00, 127.43, 127.13, 126.21, 125.79, 124.31, 72.74, 72.25, 46.52, 46.38, 32.78, 19.52, 19.50, 15.29, 15.06, 13.89.
HRMS (ESI): m/z theoretical value: $C_{17}H_{19}N_3O_2[M+H]^+$ 298.1550, measured value: 298.1549.

Compound 85:

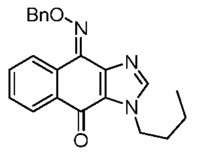

(E) and (Z)-9-((benzyloxy)imino)-3-butyl-3,9-dihydro-4H-naphthol [2,3-d] imidazole-4-one

$^1$H NMR (400 MHz, DMSO-$d_6$) δ 9.06 (dd, J = 8.0, 1.2 Hz, 1H), 8.36-8.24 (m, 6H), 8.22 (s, 1H), 8.13 (dd, J = 8.0, 1.6 Hz, 2H), 7.81-7.69 (m, 4H), 7.65 (dd, J = 8.0, 1.4 Hz, 2H), 7.63-7.59 (m, 1H), 7.56-7.49 (m, 6H), 7.45-7.31 (m, 16H), 5.53 (s, 2H), 5.50 (s, 5H), 4.45 (t, J = 7.2 Hz, 5H), 4.39 (t, J = 7.1 Hz, 2H), 1.82-1.72 (m, 7H), 1.34-1.22 (m, 7H), 0.90 (td, J = 8.0, 1.0 Hz, 11H).

Compound 86:

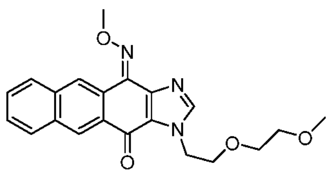

(E) and (Z)-11-((methoxy)imino) -3-(2-(2-methoxyethoxy)ethyl)-3,11-dihydro-4H-anthraquinone [2,3-d] imidazole-4-one

$^1$H NMR (400 MHz, DMSO-$d_6$) δ 9.55 (s, 2H), 8.74 (d, J = 9.3 Hz, 3H), 8.69 (s, 1H), 8.63 (s, 1H), 8.59 (s, 1H), 8.25 - 8.04 (m, 12H), 7.75 - 7.63 (m, 7H), 4.58 (dq, J = 16.0, 5.2 Hz, 7H), 4.25 (s, 5H), 4.18 (s, 3H), 3.83-3.75 (m, 7H), 3.55-3.10 (m, 7H), 3.14 - 3.12 (m, 8H).
HRMS (ESI): m/z theoretical value: $C_{17}H_{19}N_3O_2[M+H]^+$ 380.1605, measured value: 380.1609.

## Example 9

2,3-Diamine-1,4-benzoquinone (20 g) was dissolved into 100 mL of acetic acid, subjected to reflux reaction for 6 h, and cooled to room temperature, then poured into an ice-water mixture with the addition of ammonium hydroxide to regulate pH to 9-10, and the obtained solution was subjected to suction filtration and vacuum dried to obtain yellow solid 1,4-naphthoquinone imidazole (22 g). 1,4-naphthoquinone imidazole (0.3 g) and potassium hydroxide (0.15 g) were added to dimethyl sulfoxide solution (3 mL), respectively; the mixture was stirred at 50°C for 30 min and added with iodobutane (0.18 mL), after reaction for 5 h, water was added to separate out solid; the solid was subjected to suction filtration, washed with water, and vacuum dried to obtain yellow solid 2-butyl-1,4-naphthoquinone imidazole (0.31 g). 2-butylnaphthoquinone imidazole (0.21 g), methoxyamine hydrochloride (0.3 g), and pyridine (0.7 mL) were dissolved into ethanol (70 mL) and reacted for 36 h at 105°C; at the end of reaction, the obtained solution was concentrated and isolated by silica gel column chromatography to obtain light yellow product (0.16 g).
2,3-Diamine-1,4-naphthoquinone (20 g) was dissolved into 100 mL of formic acid and subjected to reflux reaction for 6 h to obtain anthraquinone imidazole; or Compounds 87-88 may be prepared according to the method in Example 9; other materials and reaction conditions for the compounds were kept the same and raw materials could be purchased commercially:

Compound 87:

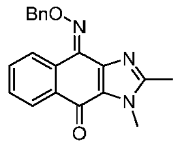

(E) and (Z)-9-((benzyloxy)imino)-2,3-dimethyl-3,9-dihydro-4H-naphthol [2,3-d] imidazole-4-one

$^1H$ NMR (400 MHz, DMSO-$d_6$) δ 8.99 (dd, J = 8.0, 1.4 Hz, 1H), 8.22-8.16 (m, 4H), 8.05 (dd, J = 7.8, 1.6 Hz, 3H), 7.73 - 7.68 (m, 1H), 7.66 - 7.62 (m, 3H), 7.58 (td, J = 7.6, 1.4 Hz, 3H), 7.51 - 7.45 (m, 7H), 7.39 - 7.31 (m, 8H), 7.31 - 7.26 (m, 3H), 5.48 (s, 2H), 5.46 (s, 5H), 3.93 (s, 8H), 3.87 (s, 3H), 2.45 (s, 8H), 2.40 (s, 3H).

HRMS (ESI): m/z theoretical value: $C_{17}H_{19}N_3O_2[M+H]^+$ 332.1394, measured value: 332.1398.

Compound 88:

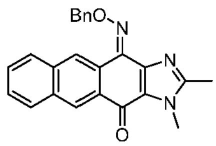

(E) and (Z)-11-((benzyloxy)imino)-2,3-dimethyl-3,11-dihydro-4H-anthraquinone [2,3-d] imidazole-4-one

$^1H$ NMR (400 MHz, DMSO-$d_6$) δ 8.69 (d, J = 10.2 Hz, 2H), 8.13 (d, J = 8.2 Hz, 1H), 8.05 (d, J = 8.0 Hz, 1H), 7.69 - 7.52 (m, 2H), 7.39 - 7.31 (m, 3H), 5.59 (s, 2H), 4.03 (s, 3H), 2.72 (s, 3H).

HRMS (ESI): m/z theoretical value: $C_{17}H_{19}N_3O_2[M+H]^+$ 382.1550, measured value: 382.1558.

**Example 10**

2,3-Diamine-1,4-benzoquinone (4 g) was dissolved into 13 mL of dimethyl sulfoxide, added with 3,4,5-trimethoxybenzaldehyde (2.5 mL), and subjected to open-ended reaction for 13 h at 80°C, and cooled to room temperature, then slowly added with water, solid was separated out, subjected to suction filtration and vacuum dried to obtain yellow solid 2-(3,4,5-trimethoxyphenyl)-1,4-naphthoquinone imidazole (0.15 g). Naphthoquinone imidazole (0.4 g) and potassium hydroxide (0.15 g) were added to dimethyl sulfoxide solution (3 mL), respectively; the mixture was stirred at 50°C for 30 min and added with iodobutane (0.17 mL), after reaction for 5 h, water was added to separate out solid; the solid was subjected to suction filtration, washed with water, and vacuum dried to obtain yellow solid 1-butyl-2-(3,4,5-trimethoxyphenyl)-1,4-naphthoquinone imidazole (0.35 g). Naphthoquinone imidazole (0.42 g), benzyloxyamine hydrochloride (0.64 g), and pyridine (0.7 mL) were dissolved into ethanol (70 mL) and reacted for 36 h at 105°C; at the end of reaction, the obtained solution was concentrated and isolated by silica gel column chromatography to obtain light yellow product (0.36 g).

2,3-Diamine-1,4-naphthoquinone (4 g) was dissolved into 13 mL of dimethyl sulfoxide and reacted with different aldehydes; or aminoxyhydrochlorides having different substitutes were used to prepare Compounds 89-90 according to the method in Example 10; other materials and reaction conditions for the compounds were kept the same and raw materials could be purchased commercially:

Compound 89:

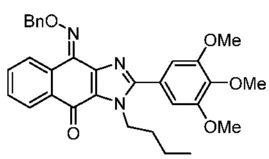

(E) and (Z)-9-((benzyloxy)imino)-3-butyl-2-(3,4,5-trimethoxyphenyl)-3,9-dihydro-4H-naphthol [2,3-d] imidazole-4-one

$^1H$ NMR (400 MHz, DMSO-$d_6$) δ 9.04 (d, J = 8.0 Hz, 1H), 8.27 (t, J = 7.0 Hz, 2H), 8.13 (dd, J = 7.8, 1.6 Hz, 2H), 7.72

(dt, J = 15.2, 7.4 Hz, 3H), 7.63 (t, J = 7.6 Hz, 2H), 7.57 - 7.50 (m, 3H), 7.47 (d, J = 7.4 Hz, 2H), 7.40-7.18 (m, 14H), 6.98 (s, 3H), 6.95 (s, 2H), 5.52 (s, 2H), 5.48 (s, 3H), 4.53 (s, 3H), 4.47 (t, J = 7.4 Hz, 3H), 4.41 (t, J = 7.6 Hz, 2H), 3.82 (s, 4H), 3.80 (s, 7H), 3.72 (d, J = 1.2 Hz, 5H), 1.70-1.65 (m, 5H), 1.21 - 1.15 (m, 5H), 0.85-0.70 (m, 7H).

HRMS (ESI): m/z theoretical value: $C_{17}H_{19}N_3O_2[M+H]^+$ 526.2337, measured value: 526.2337.

Compound 90:

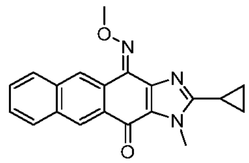

(E) and (Z)-2-cyclopropyl-11-(methoxyimino)-3-methyl-3,11-dihydro-4H-anthraquinone [2,3-d] imidazole-4-one

$^1$H NMR (400 MHz, DMSO-$d_6$) δ 8.55 (s, 2H), 8.18-8.12 (m, 2H), 7.73-7.60 (m, 2H), 2.15-2.01 (m, 1H), 1.12-0.97 (m, 4H), 4.05 (s, 3H), 3.84 (s, 3H).

HRMS (ESI): m/z theoretical value: $C_{17}H_{19}N_3O_2[M+H]^+$ 332.1394, measured value: 332.1395.

## Example 11

Compound 1 (0.05 mmol) and 31 μL of iodomethane were added to 3 mL acetonitrile for reaction for 24 h at 120°C. At the end of the reaction, solvent was removed to obtain a yellow solid, and the solid was pulped for half an hour and subjected to suction filtration to obtain the final product.

Different synthetic intermediate compounds above were used to prepare Compounds 91-118 according to the method in Example 11; other materials and reaction conditions for the compounds were kept the same and raw materials could be purchased commercially:

Compound 91:

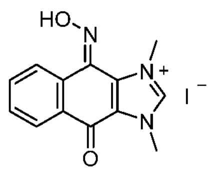

(E)-1-methyl-4-(hydroxylimino)-3-methyl -9-oxo-4,9-dihydro-1H-naphthol [2,3-d] imidazole-3-iodide

$^1$H NMR (500 MHz, DMSO-$d_6$) δ 14.50 (s, 1H), 9.57 (s, 1H), 9.25 (dd, J = 10.0, 0.5 Hz, 1H), 8.33 (dd, J = 10.0, 1.0 Hz, 1H), 8.01-7.93 (m, 1H), 7.85 (td, J = 5.0, 1.5 Hz, 1H), 4.19 (s, 3H), 4.10 (s, 3H).

$^{13}$C NMR (126 MHz, DMSO-$d_6$) δ 174.28, 143.85, 138.38, 135.34, 135.31, 131.90, 131.49, 130.83, 127.34, 127.16, 125.39, 38.98, 36.41.

HRMS(ESI-TOF) m/z theoretical value: $C_{13}H_{12}N_3O_2[M - I]^+$, 242.0924, measured value: 242.0928.

Compound 92:

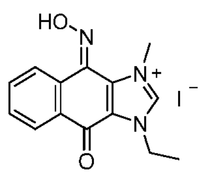

(E)-1-ethyl-4-(hydroxylimino)-3-methyl -9-oxo-4,9-dihydro-1H-naphthol [2,3-d] imidazole-3-iodide

$^1$H NMR (500 MHz, DMSO-$d_6$) δ 14.49 (s, 1H), 9.67 (s, 1H), 9.26 (dd, J = 10.0, 1.0 Hz, 1H), 8.34 (dd, J = 10.0, 1.0

Hz, 1H), 7.96 (td, J = 10.0, 1.5 Hz, 1H), 7.85 (td, J = 10.0, 1.0 Hz, 1H), 4.63 (q, J = 7.5 Hz, 2H), 4.11 (s, 3H), 1.53 (t, J = 5.0 Hz, 3H).

$^{13}$C NMR (126 MHz, DMSO-$d_6$) δ 174.01, 143.12, 138.52, 135.86, 135.28, 131.83, 131.37, 130.85, 127.39, 127.03, 124.77, 44.93, 39.09, 15.42.

HRMS(ESI-TOF) m/z theoretical value: $C_{14}H_{14}N_3O_2$[M - I]$^+$, 256.1081, measured value: 256.1078.

Compound 93:

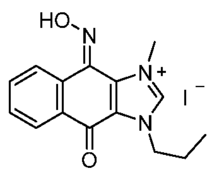

(E)-4-(hydroxylimino)-3-methyl -9-oxo-1-propyl-4,9-dihydro-1H-naphthol [2,3-d] imidazole-3-iodide

$^1$H NMR (500 MHz, DMSO-$d_6$) δ 14.51 (s, 1H), 9.67 (s, 1H), 9.25 (dd, J = 10.0, 1.0 Hz, 1H), 8.34 (dd, J = 10.0, 1.5 Hz, 1H), 8.00-7.93 (m, 1H), 7.85 (td, J = 10.0, 1.0 Hz, 1H), 4.57 (t, J = 7.0 Hz, 2H), 4.11 (s, 3H), 1.96-1.85 (m, 2H), 0.96 (t, J = 7.5 Hz, 3H).

$^{13}$C NMR (126 MHz, DMSO-$d_6$) δ 174.09, 143.40, 138.51, 135.95, 135.29, 131.88, 131.43, 130.91, 127.42, 127.08, 124.84, 50.68, 39.17, 23.13, 10.92.

HRMS(ESI-TOF) m/z theoretical value: $C_{15}H_{16}N_3O_2$[M - I]$^+$, 270.1237, measured value: 270.1244.

Compound 94:

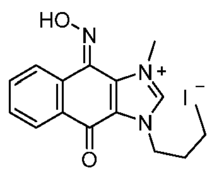

(E)-1-butyl-4-(hydroxylimino)-3-methyl -9-oxo-4,9-dihydro-1H-naphthol [2,3-d] imidazole-3-iodide

$^1$H NMR (500 MHz, DMSO-$d_6$) δ 14.50 (s, 1H), 9.67 (s, 1H), 9.25 (dd, J = 10.0, 0.5 Hz, 1H), 8.34 (dd, J = 10.0, 1.5 Hz, 1H), 7.96 (td, J = 10.0, 1.5 Hz, 1H), 7.85 (td, J = 10.0, 1.5 Hz, 1H), 4.60 (t, J = 7.0 Hz, 2H), 4.10 (s, 3H), 1.91-1.85 (m, 2H), 1.44-1.33 (m, 2H), 0.95 (t, J = 7.5 Hz, 3H).

$^{13}$C NMR (126 MHz, DMSO-$d_6$) δ 174.06, 143.37, 138.52, 135.93, 135.28, 131.85, 131.40, 130.89, 127.42, 127.06, 124.80, 49.00, 39.16, 31.69, 19.28, 13.86.

HRMS(ESI-TOF) m/z theoretical value: $C_{16}H_{18}N_3O_2$[M - I]$^+$, 284.1394, measured value: 284.1393.:

Compound 95:

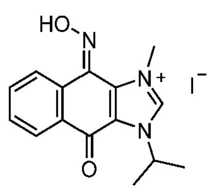

(E)-4-(hydroxylimino) -1-isopropyl-3-methyl-9-oxo-4,9-dihydro-1H-naphthol [2,3-d] imidazole-3-iodide

$^1$H NMR (400 MHz, DMSO-$d_6$) δ 14.50 (s, 1H), 9.81 (s, 1H), 9.24 (dd, J = 8.0, 1.2 Hz, 1H), 8.35 (dd, J = 8.0, 1.6 Hz, 1H), 8.04-7.90 (m, 1H), 7.85 (td, J = 8.0, 1.2 Hz, 1H), 5.49-5.40 (m, 1H), 4.11 (s, 3H), 1.61 (d, J = 8.0 Hz, 6H).

$^{13}$C NMR (101 MHz, DMSO-$d_6$) δ 174.04, 141.76, 138.52, 135.96, 135.23, 131.88, 131.36, 131.04, 127.51, 126.85, 124.69, 52.90, 39.23, 22.54.

HRMS(ESI-TOF) m/z theoretical value: $C_{15}H_{16}N_3O_2$[M - I]$^+$, 270.12374, measured value: 270.1237.:

Compound 96:

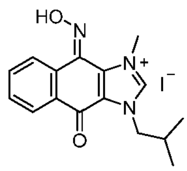

(E)-4-(hydroxylimino)-1-isobutyl-3-methyl-9-oxo-4,9-dihydro-1H-naphthol [2,3-d] imidazole-3-iodide

$^1$H NMR (500 MHz, DMSO-$d_6$) δ 14.51 (s, 1H), 9.68 (s, 1H), 9.25 (dd, J = 10.0, 0.5 Hz, 1H), 8.33 (dd, J = 10.0, 1.0 Hz, 1H), 7.99-7.93 (m, 1H), 7.85 (td, J = 10.0, 1.0 Hz, 1H), 4.44 (d, J = 10.0 Hz, 2H), 4.12 (s, 3H), 2.27-2.10 (m, 1H), 0.98 (d, J = 5.0 Hz, 6H).

$^{13}$C NMR (126 MHz, DMSO-$d_6$) δ 174.14, 143.52, 138.52, 136.06, 135.31, 131.87, 131.41, 130.92, 127.42, 127.08, 124.84, 55.59, 39.26, 29.14, 19.66.

HRMS(ESI-TOF) m/z theoretical value: $C_{16}H_{18}N_3O_2$[M - I]$^+$, 284.1394, measured value: 284.1387.:

Compound 97:

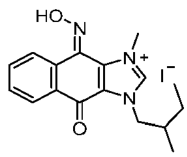

(E)-4-(hydroxylimino) -3-methyl-1-(2-methylbutyl)-9-oxo-4,9-dihydro-1H-naphthol [2,3-d] imidazole-3-iodide

$^1$H NMR (400 MHz, DMSO-$d_6$) δ 14.50 (s, 1H), 9.69 (s, 1H), 9.25 (dd, J = 8.0, 0.8 Hz, 1H), 8.33 (dd, J = 8.0, 1.4 Hz, 1H), 8.01-7.91 (m, 1H), 7.84 (td, J = 8.0, 1.2 Hz, 1H), 4.56 (dd, J = 12.0, 4.0 Hz, 1H), 4.42-4.35 (m, 1H), 4.12 (s, 3H), 2.07-1.90 (m, 1H), 1.53-1.40 (m, 1H), 1.33-1.19 (m, 1H), 1.00-0.87 (m, 6H).

$^{13}$C NMR (101 MHz, DMSO-$d_6$) δ 174.12, 143.58, 138.52, 136.06, 135.29, 131.86, 131.40, 130.91, 127.43, 127.07, 124.87, 54.38, 39.27, 35.19, 26.25, 16.46, 11.30.

HRMS(ESI-TOF) m/z theoretical value: $C_{17}H_{20}N_3O_2$[M - I]$^+$, 298.1550, measured value: 298.1551.

Compound 98:

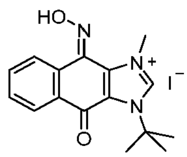

(E)-1-(tert-butyl)-4-(hydroxylimino)-3-methyl-9-oxo-4,9-dihydro-1H-naphthol [2,3-d] imidazole-3-iodide

$^1$H NMR (400 MHz, DMSO-$d_6$) δ 14.50 (s, 1H), 9.67 (s, 1H), 9.19 (dd, J = 8.0, 1.2 Hz, 1H), 8.38 (dd, J = 8.0, 1.4 Hz, 1H), 7.98-7.91 (m, 1H), 7.89-7.79 (m, 1H), 4.11 (s, 3H), 1.81 (s, 9H).

$^{13}$C NMR (101 MHz, DMSO-$d_6$) δ 173.08, 142.92, 138.84, 138.42, 134.98, 131.90, 131.45, 130.93, 1210.06, 126.24, 126.02, 63.89, 36.40, 28.72.

HRMS(ESI-TOF) m/z theoretical value: $C_{16}H_{18}N_3O_2$[M - I]$^+$, 284.1394, measured value: 284.1395.

Compound 99:

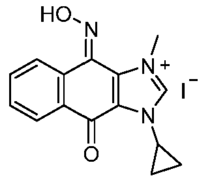

(E)-1-cyclopropyl-4-(hydroxylimino)-3-methyl-9-oxo-4,9-dihydro-1H-naphthol [2,3-d] imidazole-3-iodide

$^1H$ NMR (500 MHz, DMSO-$d_6$) δ 14.48 (s, 1H), 9.73 (s, 1H), 9.31-9.16 (m, 1H), 8.35 (dd, J = 10.0, 1.5 Hz, 1H), 7.96 (td, J = 10.0, 1.0 Hz, 1H), 7.86 (td, J = 10.0, 1.0 Hz, 1H), 4.16-4.05 (m, 1H), 4.06 (s, 3H), 1.31-1.18 (m, 4H).

$^{13}C$ NMR (126 MHz, DMSO-$d_6$) δ 173.42, 143.46, 138.43, 135.56, 135.19, 131.93, 131.43, 131.00, 127.40, 126.93, 126.42, 39.02, 31.83, 7.48.

HRMS(ESI-TOF) m/z theoretical value: $C_{15}H_{14}N_3O_2[M - I]^+$, 268.1081, measured value: 268.1081.

Compound 100:

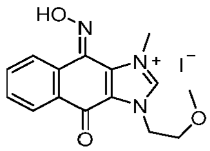

(E)-4-(hydroxylimino)-1-(2-methoxyethyl)-3-methyl-9-oxo-4,9-dihydro-1H-naphthol [2,3-d] imidazole-3-iodide

$^1H$ NMR (500 MHz, DMSO-$d_6$) δ 14.51 (s, 1H), 9.65 (s, 1H), 9.25 (d, J = 10.0, 1H), 8.33 (dd, J = 10.0, 1.5 Hz, 1H), 7.97 (td, J = 10.0, 1.5 Hz, 1H), 7.85 (td, J = 10.0, 1.0 Hz, 1H), 4.81 (t, J = 5.0 Hz, 2H), 4.14 (s, 3H), 3.81 (t, J = 5.0 Hz, 2H), 3.30 (s, 3H).

$^{13}C$ NMR (126 MHz, DMSO-$d_6$) δ 174.24, 143.67, 138.44, 135.78, 135.33, 131.86, 131.44, 130.88, 127.41, 127.09, 124.77, 69.37, 58.66, 48.91, 39.15.

HRMS(ESI-TOF) m/z theoretical value: $C_{15}H_{16}N_3O_3[M - I]^+$, 286.1186, measured value: 286.1187.:

Compound 101:

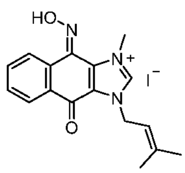

(E)-4-(hydroxylimino) -3-methyl-1-(3-methyl-2-ene-1-yl)-9-oxo-4,9-dihydro-1H-naphthol [2,3-d] imidazole-3-iodide

$^1H$ NMR (500 MHz, DMSO-$d_6$) δ 14.50 (s, 1H), 9.62 (s, 1H), 9.25 (dd, J = 10.0, 1.0 Hz, 1H), 8.34 (dd, J = 10.0, 1.5 Hz, 1H), 7.96 (td, J = 10.0, 1.5 Hz, 1H), 7.85 (td, J = 10.0, 1.0 Hz, 1H), 5.55-5.45 (m, 1H), 5.22 (d, J = 5.0 Hz, 2H), 4.11 (s, 3H), 1.84 (s, 3H), 1.80 (s, 3H).

$^{13}C$ NMR (126 MHz, DMSO-$d_6$) δ 174.14, 142.89, 141.39, 138.44, 135.86, 135.31, 131.89, 131.45, 130.86, 127.42, 127.07, 124.81, 116.94, 47.08, 39.11, 26.02, 18.66.

HRMS(ESI-TOF) m/z theoretical value: $C_{17}H_{18}N_3O_2[M - I]^+$, 296.1394, measured value: 296.1390.

Compound 102:

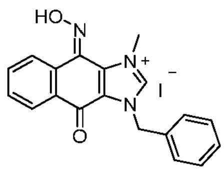

(E)-1-benzyl-4-(hydroxylimino)-3-methyl-9-oxo-4,9-dihydro-1H-naphthol [2,3-d] imidazole-3-iodide

$^1$H NMR (500 MHz, DMSO-$d_6$) δ 14.53 (s, 1H), 9.76 (s, 1H), 9.24 (d, J = 10.0 Hz, 1H), 8.30 (dd, J = 10.0, 1.5 Hz, 1H), 7.95 (td, J = 10.0, 1.5 Hz, 1H), 7.83 (td, J = 10.0, 1.0 Hz, 1H), 7.51-7.45 (m, 2H), 7.45-7.36 (m, 3H), 5.88 (s, 2H), 4.13 (s, 3H).

$^{13}$C NMR (126 MHz, DMSO-$d_6$) δ 174.00, 143.75, 138.46, 136.14, 135.37, 134.74, 131.87, 131.44, 130.76, 129.34, 129.11, 128.56, 127.39, 127.12, 124.57, 51.80, 39.35.

HRMS(ESI-TOF) m/z theoretical value: $C_{19}H_{16}N_3O_2$[M - I]$^+$, 318.1237, measured value: 318.1233.

Compound 103:

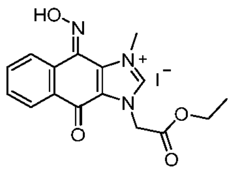

(E)-1-(2-ethoxy-2-ethoxy)-4-(hydroxylimino)-3-methyl -9-oxo-4,9-dihydro-1H-naphthol [2,3-d] imidazole-3-iodide

$^1$H NMR (500 MHz, DMSO-$d_6$) δ 14.62 (s, 1H), 9.61 (s, 1H), 9.27 (d, J = 10.0 Hz, 1H), 8.30 (dd, J = 10.0, 1.5 Hz, 1H), 7.99 (td, J = 10.0, 1.5 Hz, 1H), 7.88-7.82 (m, 1H), 5.58 (s, 2H), 4.26 (q, J = 7.0 Hz, 2H), 4.19 (s, 3H), 1.27 (t, J = 7.0 Hz, 3H).

$^{13}$C NMR (126 MHz, DMSO-$d_6$) δ 174.04, 166.57, 144.24, 138.22, 135.63, 135.54, 131.97, 131.59, 130.42, 127.36, 127.23, 124.83, 62.54, 49.97, 39.38, 14.51.

HRMS(ESI-TOF) m/z theoretical value: $C_{16}H_{16}N_3O_4$[M - I]$^+$, 314.1135, measured value: 314.1138.:

Compound 104:

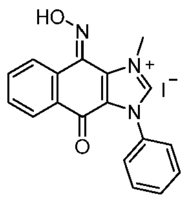

(E)-4-(hydroxylimino)-3-methyl-9-oxo-1-phenyl-4,9-dihydro-1H-naphthol [2,3-d] imidazole-3-iodide

$^1$H NMR (500 MHz, DMSO-$d_6$) δ 14.60 (s, 1H), 10.00 (s, 1H), 9.28 (d, J = 5.0, Hz, 1H), 8.23 (dd, J = 10.0, 1.5 Hz, 1H), 8.01-7.95 (m, 1H), 7.84 (td, J = 10.0, 1.0 Hz, 1H), 7.77-7.74 (m, 2H), 7.71-7.67 (m, 3H), 4.19 (s, 3H).

$^{13}$C NMR (126 MHz, DMSO-$d_6$) δ 172.91, 144.41, 138.47, 135.61, 135.26, 133.99, 131.95, 131.41, 131.18, 131.04, 129.83, 127.44, 126.91, 126.62, 125.20, 39.40.

HRMS(ESI-TOF) m/z theoretical value: $C_{18}H_{14}N_3O_2$[M - I]$^+$, 304.1081, measured value: 304.1085.

Compound 105:

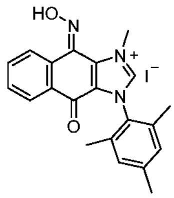

(E)-4-(hydroxylimino)-1-sym-trimethyl-3-methyl-9-oxo-4,9-dihydro-1H-naphthol [2,3-d] imidazole-3-iodide

$^1$H NMR (500 MHz, DMSO-$d_6$) δ 14.58 (s, 1H), 9.81 (s, 1H), 9.29 (dd, J = 10.0, 0.5 Hz, 1H), 8.18 (dd, J = 10.0, 1.0 Hz, 1H), 8.03-7.93 (m, 1H), 7.82 (td, J = 10.0, 1.0 Hz, 1H), 7.16 (s, 2H), 4.20 (s, 3H), 2.38 (s, 3H), 2.04 (s, 6H).

$^{13}$C NMR (126 MHz, DMSO-$d_6$) δ 173.08, 144.26, 140.75, 138.74, 136.36, 135.27, 135.12, 131.82, 131.44, 130.87, 130.41, 129.56, 127.35, 127.27, 124.97, 39.78, 21.15, 17.54.

HRMS(ESI-TOF) m/z theoretical value: $C_{21}H_{20}N_3O_2$[M - I]$^+$, 346.1550, measured value: 346.1555.

Compound 106:

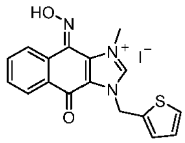

(E)-4-(hydroxylimino)-3-methyl-9-oxo-1-(thiophene-2-yl-methyl)-4,9-dihydro-1H-na phthol [2,3-d] imidazole-3-iodide

$^1$H NMR (400 MHz, DMSO-$d_6$) δ 13.01 (s, 1H), 8.49 (s, 1H), 8.27 (d, J = 7.8 Hz, 1H), 8.12 (dd, J = 8.0, 1.6 Hz, 1H), 7.69 (t, J = 7.6 Hz, 1H), 7.60 (t, J = 7.1 Hz, 1H), 7.43 (d, J = 5.9 Hz, 1H), 7.19 (d, J = 3.0 Hz, 1H), 6.95 (dd, J = 5.1, 3.5 Hz, 1H), 5.88 (s, 2H), 4.28 (s, 3H).

HRMS(ESI-TOF) m/z theoretical value: C17H14N302S[M-I]+, 324.0801, measured value: 324.0802.

Compound 107:

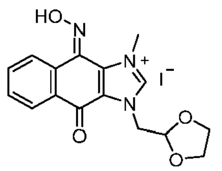

(E)-1-((1,3-dioxoheterocycle-2-yl)methyl)-4-(hydroxylimino)-3-methyl -9-oxo-4,9-dihydro-1H-naphthol [2,3-d] imidazole-3-iodide

$^1$H NMR (500 MHz, DMSO-$d_6$) δ 14.53 (s, 1H), 9.64 (s, 1H), 9.25 (dd, J = 10.0, 0.5 Hz, 1H), 8.33 (dd, J = 10.0, 1.5 Hz, 1H), 8.01-7.93 (m, 1H), 7.85 (td, J = 10.0, 1.0 Hz, 1H), 5.34 (t, J = 3.5 Hz, 1H), 4.91 (d, J = 5.0 Hz, 2H), 4.16 (s, 3H), 3.86 (s, 4H).

$^{13}$C NMR (126 MHz, DMSO-$d_6$) δ 174.18, 144.11, 138.36, 135.52, 135.39, 131.89, 131.45, 130.83, 127.43, 127.06, 125.01, 99.99, 65.37, 49.50, 39.28.

HRMS(ESI-TOF) m/z theoretical value: $C_{16}H_{15}N_3O_4$[M - I]$^+$, 314.1135, measured value: 314.1136.

Compound 108:

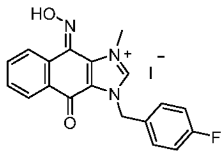

(E) -1-(4-fluorobenzyl)-4-(hydroxylimino)-3-methyl-9-oxo-4,9-dihydro-1H-naphthol [2,3-d] imidazole-3-iodide

$^1$H NMR (500 MHz, DMSO-$d_6$) δ 14.54 (s, 1H), 9.71 (s, 1H), 9.24 (dd, J = 10.0, 0.5 Hz, 1H), 8.31 (dd, J = 10.0, 1.5 Hz, 1H), 7.96 (td, J = 10.0, 1.5 Hz, 1H), 7.83 (td, J = 10.0, 1.0 Hz, 1H), 7.57 (dd, J = 10.0, 5.0 Hz, 2H), 7.28 (t, J = 10.0 Hz, 2H), 5.85 (s, 2H), 4.12 (s, 3H).
$^{13}$C NMR (126 MHz, DMSO-$d_6$) δ 174.02, 163.66 (161.71, J = 243.8 Hz), 143.70, 138.45, 136.13, 135.39, 131.89, 131.45, 131.20 (131.14, J = 7.5 Hz), 130.89 (130.87, J = 2.5 Hz), 130.76, 127.40, 127.12, 124.57, 116.29 (116.12, J = 21.3 Hz), 51.15, 39.33.
HRMS(ESI-TOF) m/z theoretical value: $C_{19}H_{15}FN_3O_2$[M-I]$^+$, 336.1143, measured value: 336.1138.

Compound 109:

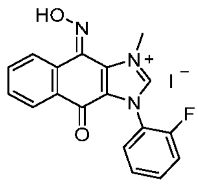

(E)-1-(2-fluorophenyl)-4-(hydroxylimino)-3-methyl-9-oxo-4,9-dihydro-1H-naphthol [2,3-d] imidazole-3-iodide

$^1$H NMR (500 MHz, DMSO-$d_6$) δ 14.68 (s, 1H), 10.08 (s, 1H), 9.29 (dd, J = 10.0, 1.0 Hz, 1H), 8.24 (dd, J = 10.0, 1.5 Hz, 1H), 8.03-7.95 (m, 1H), 7.89-7.77 (m, 3H), 7.68-7.61 (m, 1H), 7.54 (td, J = 10.0, 1.0 Hz, 1H), 4.21 (s, 3H).
$^{13}$C NMR (126 MHz, DMSO) δ 172.95, 157.58 (155.57, J = 251.3 Hz), 145.03, 138.34, 135.74, 135.52, 133.83 (133.77, J = 7.5 Hz), 132.01, 131.52, 130.65, 128.91, 127.34, 127.06, 125.88 (125.85, J = 3.8 Hz), 125.17, 121.97 (121.87, J = 12.5 Hz), 117.28 (117.13, J = 18.8 Hz), 39.62.
HRMS(ESI-TOF) m/z theoretical value: $C_{18}H_{13}FN_3O_2$[M-I]$^+$, 322.0986, measured value: 322.0986.

Compound 110:

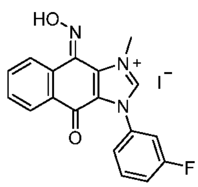

(E)-1-(3-fluorophenyl)-4-(hydroxylimino)-3-methyl-9-oxo-4,9-dihydro-1H-naphthol [2,3-d] imidazole-3-iodide

$^1$H NMR (400 MHz, DMSO-$d_6$) δ 14.64 (s, 1H), 10.04 (d, J = 0.4 Hz, 1H), 9.28 (dd, J = 8.0, 0.8 Hz, 1H), 8.25 (dd, J = 8.0, 1.2 Hz, 1H), 8.03-7.95 (m, 1H), 7.85 (td, J = 8.0, 1.2 Hz, 1H), 7.80-7.71 (m, 2H), 7.66-7.54 (m, 2H), 4.20 (s, 3H).
$^{13}$C NMR (101 MHz, DMSO) δ 172.90, 163.16 (160.72, J = 244.0 Hz), 144.62, 138.38, 135.43, 135.33, 135.07 (134.96, J = 11.0 Hz), 131.98, 131.76 (131.67, J = 9.0 Hz), 131.41, 130.99, 127.49, 126.87, 125.21, 123.05 (123.02, J = 3.0 Hz), 118.47 (118.26, J = 21.0 Hz), 114.78 (114.53, J = 25.0 Hz), 39.45.
HRMS(ESI-TOF) m/z theoretical value: $C_{18}H_{13}FN_3O_2$ [M-I]$^+$, 322.0986, measured value: 322.0982.:

Compound 111:

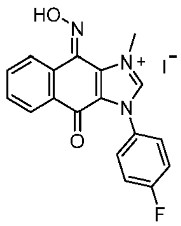

(E)-1-(4-fluorophenyl)-4-(hydroxylimino)-3-methyl-9-oxo-4,9-dihydro-1H-naphthol [2,3-d] imidazole-3-iodide

$^{1}H$ NMR (400 MHz, DMSO-$d_6$) δ 14.60 (s, 1H), 9.96 (s, 1H), 9.26 (d, J = 8.0 Hz, 1H), 8.22 (d, J = 8.0 Hz, 1H), 7.95 (t, J = 8.0 Hz, 1H), 7.86-7.72 (m, 3H), 7.54 (t, J = 8.0 Hz, 2H), 4.17 (s, 3H).

$^{13}C$ NMR (126 MHz, DMSO) δ 172.97, 164.38 (162.40, J = 1910.0 Hz), 144.56, 138.40, 135.41 (135.31, J = 10.0 Hz), 131.97, 131.44, 130.99, 130.26, 130.24, 129.22 (129.14, J = 10.0 Hz), 127.44, 126.91, 125.32, 116.93 (116.74, J = 19.0 Hz), 39.39.

HRMS(ESI-TOF) m/z theoretical value: $C_{18}H_{13}FN_3O_2[M-I]^+$, 322.0986, measured value: 322.0991.

## Compound 112:

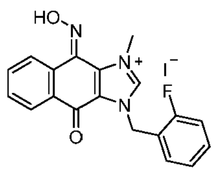

(E) -1-(2-fluorobenzyl)-4-(hydroxylimino)-3-methyl-9-oxo-4,9-dihydro-1H-naphthol [2,3-d] imidazole-3-iodide

$^{1}H$ NMR (500 MHz, DMSO-$d_6$) δ 14.55 (s, 1H), 9.71 (s, 1H), 9.25 (dd, J = 10.0, 1.0 Hz, 1H), 8.29 (dd, J = 10.0, 1.5 Hz, 1H), 7.96 (td, J = 10.0, 1.5 Hz, 1H), 7.83 (td, J = 10.0, 1.0 Hz, 1H), 7.52-7.43 (m, 2H), 7.37-7.30 (m, 1H), 7.24 (td, J = 10.0, 1.0 Hz, 1H), 5.94 (s, 2H), 4.14 (s, 3H).

$^{13}C$ NMR (126 MHz, DMSO-$d_6$) δ 173.93, 161.62 (159.66, J = 245.0 Hz), 144.04, 138.44, 136.14, 135.42, 131.90, 131.61 (131.55, J = 7.5 Hz), 131.46, 130.70, 130.67, 127.40, 127.13, 125.35 (125.32, J = 3.8 Hz), 124.67, 121.80 (121.69, J = 13.8 Hz), 116.23 (116.07, J = 20.0 Hz), 46.44 (46.41, J = 3.8 Hz), 39.32.

HRMS(ESI-TOF) m/z theoretical value: $C_{19}H_{15}FN_3O_2[M-I]^+$, 336.1143, measured value: 336.1145.

## Compound 113:

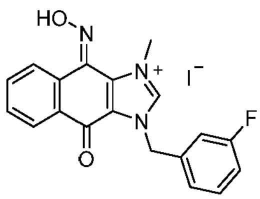

(E) -1-(3-fluorobenzyl)-4-(hydroxylimino)-3-methyl-9-oxo-4,9-dihydro-1H-naphthol [2,3-d] imidazole-3-iodide

$^{1}H$ NMR (500 MHz, DMSO-$d_6$) δ 14.55 (s, 1H), 9.74 (s, 1H), 9.25 (dd, J = 10.0, 1.0 Hz, 1H), 8.30 (dd, J = 10.0, 2.0 Hz, 1H), 7.96 (td, J = 10.0, 1.5 Hz, 1H), 7.83 (td, J = 10.0, 1.0 Hz, 1H), 7.50-7.45 (m, 1H), 7.36-7.29 (m, 2H), 7.26-7.18 (m, 1H), 5.89 (s, 2H), 4.13 (s, 3H).

$^{13}C$ NMR (126 MHz, DMSO-$d_6$) δ 173.96, 163.70 (161.76, J = 242.5 Hz), 143.95, 138.48, 137.47 (137.41, J = 7.5 Hz), 136.15, 135.39, 131.88, 131.45, 131.39 (131.32, J = 8.8 Hz), 130.75, 127.40, 127.15, 124.61, 124.59, 115.98 (115.82, J = 20.0 Hz), 115.41 (115.23, J = 22.5 Hz), 51.21, 39.38.

HRMS(ESI-TOF) m/z theoretical value: $C_{19}H_{15}FN_3O_2[M-I]^+$, 336.1143, measured value: 336.1146.

Compound 114:

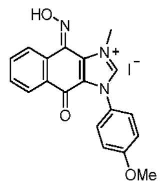

(E)-4-(hydroxylimino)-1-(4-methoxyphenyl)-3-methyl-9-oxo-4,9-dihydro-1H-naphth ol [2,3-d] imidazole-3-iodide

$^1$H NMR (500 MHz, DMSO-$d_6$) δ 14.58 (s, 1H), 9.92 (s, 1H), 9.27 (dd, J = 10.0, 1.0 Hz, 1H), 8.24 (dd, J = 10.0, 1.5 Hz, 1H), 8.01-7.93 (m, 1H), 7.84 (td, J = 10.0, 1.5 Hz, 1H), 7.66 (d, J = 10.0 Hz, 2H), 7.21 (d, J = 10.0 Hz, 2H), 4.17 (s, 3H), 3.89 (s, 3H).

$^{13}$C NMR (126 MHz, DMSO-$d_6$) δ 172.93, 161.12, 144.32, 138.47, 135.44, 135.22, 131.94, 131.40, 131.08, 127.96, 127.42, 126.91, 126.64, 125.31, 114.85, 56.23, 39.30.

HRMS(ESI-TOF) m/z theoretical value: $C_{19}H_{16}N_3O_3$[M - I]$^+$, 334.1186, measured value: 334.1184.

Compound 115:

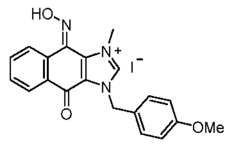

(E)-4-(hydroxylimino)-1-(4-methoxybenzyl)-3-methyl-9-oxo-4,9-dihydro-1H-naphth ol [2,3-d] imidazole-3-iodide

$^1$H NMR (500 MHz, DMSO-$d_6$) δ 14.51 (s, 1H), 9.68 (s, 1H), 9.23 (dd, J = 10.0, 1.0 Hz, 1H), 8.32 (dd, J = 10.0, 1.0 Hz, 1H), 7.99-7.91 (m, 1H), 7.83 (td, J = 10.0, 1.0 Hz, 1H), 7.52-7.45 (m, 2H), 7.04-6.96 (m, 2H), 5.79 (s, 2H), 4.10 (s, 3H), 3.76 (s, 3H).

$^{13}$C NMR (126 MHz, DMSO-$d_6$) δ 174.06, 160.08, 143.40, 138.44, 136.09, 135.36, 131.88, 131.43, 130.78, 130.66, 127.41, 127.09, 126.36, 124.56, 114.74, 55.72, 51.49, 39.29.

HRMS(ESI-TOF) m/z theoretical value: $C_{20}H_{18}N_3O_3$[M - I]$^+$, 348.1343, measured value: 348.1347.

Compound 116:

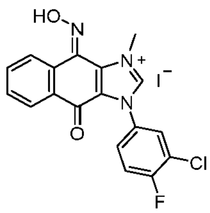

(E)-1-(3-chloro-4-fluorophenyl)-4-(hydroxylimino)-3-methyl-9-oxo-4,9-dihydro-1H-naphthol [2,3-d] imidazole-3-iodide

$^1$H NMR (500 MHz, DMSO-$d_6$) δ 14.66 (s, 1H), 10.02 (s, 1H), 9.28 (dd, J = 10.0, 1.0 Hz, 1H), 8.26 (dd, J = 10.0, 1.5 Hz, 1H), 8.17 (dd, J = 10.0, 2.5 Hz, 1H), 7.99 (td, J = 5.0, 1.5 Hz, 1H), 7.88-7.77 (m, 3H), 4.20 (s, 3H).

$^{13}$C NMR (126 MHz, DMSO-$d_6$) δ 173.01, 159.77 (157.78, J = 250.0 Hz), 144.77, 138.33, 135.39, 135.23, 132.01, 131.44, 130.92, 130.75 (130.72, J = 3.8 Hz), 129.46, 128.10 (1210.03, J = 8.8 Hz), 127.49, 126.88, 125.30, 120.56 (120.40, J = 20.0 Hz), 118.20 (118.02, J = 22.5 Hz), 39.46.

HRMS(ESI-TOF) m/z theoretical value: $C_{18}H_{12}ClFN_3O_2$[M-I]$^+$, 356.0597, measured value: 356.0593.

Compound 117:

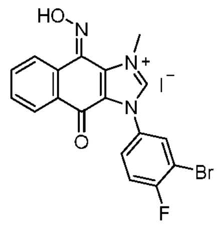

(E)-1-(3-bromo-4-fluorophenyl)-4-(hydroxylimino)-3-methyl-9-oxo-4,9-dihydro-1H-naphthol [2,3-d] imidazole-3-iodide

$^1H$ NMR (400 MHz, DMSO-$d_6$) δ 14.65 (s, 1H), 10.02 (s, 1H), 9.28 (dd, J = 8.0, 1.2 Hz, 1H), 8.33-8.20 (m, 2H), 8.02-7.95 (m, 1H), 7.90-7.80 (m, 2H), 7.75 (t, J = 8.0 Hz, 1H), 4.19 (s, 3H).

$^{13}C$ NMR (101 MHz, DMSO) δ 173.01, 161.09 (158.62, J = 247.0 Hz), 144.79, 138.33, 135.38, 135.20, 132.12 (131.99, J = 13.0 Hz), 131.44, 130.97, 130.93, 130.92, 128.70 (128.61, J = 9.0 Hz), 127.48, 126.88, 125.32, 117.93 (117.69, J = 24.0 Hz), 108.84 (108.62, J = 22.0 Hz), 39.44.

HRMS(ESI-TOF) m/z theoretical value: $C_{18}H_{12}BrFN_3O_2[M-I]^+$, 400.0091, measured value: 400.0088.

Compound 118:

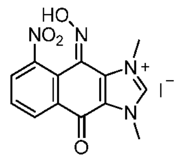

(E)-4-(hydroxylimino)-1,3-dimethyl-5-nitro-9-oxo-4,9-dihydro-1H-naphthol [2,3-d] imidazole-3-iodide

1H NMR (400 MHz, DMSO-d6) δ 14.67 (s, 1H), 9.67 (s, 1H), 8.41 (d, J = 7.8 Hz, 1H), 8.30 (d, J = 7.9 Hz, 1H), 8.19 (t, J = 7.8 Hz, 1H), 4.14 (d, J = 21.7 Hz, 6H).

HRMS(ESI-TOF) m/z theoretical value: $C_{18}H_{12}BrFN_3O_2[M-I]^+$, 287.0775, measured value: 287.0778.

## Example 12

Compound 1 (0.05 mmol) and 310 μL of iodomethane were added to 3 mL of acetonitrile for reaction for 48 h at 70°C. At the end of the reaction, solvent was removed to obtain a yellow solid, and the solid was pulped for half an hour and subjected to suction filtration to obtain the final product.

Different synthetic intermediate compounds above were used to prepare Compounds 119-126 according to the method in Example 12; other materials and reaction conditions for the compounds were kept the same and raw materials could be purchased commercially:

Compound 119:

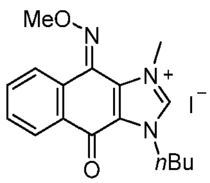

(E) and (Z)-1-butyl-4-(methoxylimino)-3-methyl -9-oxo-4,9-dihydro-1H-naphthol [2,3-d] imidazole-3-iodide

$^1H$ NMR (500 MHz, DMSO-$d_6$) δ 9.65 (s, 1H), 9.02 (dd, J = 5.0, 0.5 Hz, 1H), 8.35 (dd, J = 10.0, 1.0 Hz, 1H), 7.99-7.92 (m, 1H), 7.88 (td, J = 5.0, 1.0 Hz, 1H), 4.60 (t, J = 7.0 Hz, 2H), 4.41 (s, 3H), 4.13 (s, 3H), 1.93-1.81 (m, 2H), 1.43-1.35 (m, 2H), 0.95 (t, J = 5.0 Hz, 3H).

$^{13}C$ NMR (126 MHz, DMSO-$d_6$) δ 173.98, 143.58, 138.41, 135.35, 134.86, 132.54, 131.66, 131.22, 127.73, 126.70, 125.33, 66.37, 49.06, 39.25, 31.67, 19.28, 13.86.

HRMS(ESI-TOF) m/z theoretical value: $C_{17}H_{20}N_3O_2[M - I]^+$, 298.1550, measured value: 298.1555.

Compound 120:

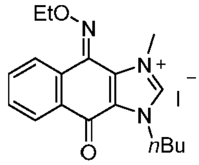

(E) and (Z)-1-butyl-4-(ethoxylimino)-3-methyl -9-oxo-4,9-dihydro-1H-naphthol [2,3-d] imidazole-3-iodide

yellow solid, m. p. 134-136 °C.

$^1H$ NMR (500 MHz, DMSO-$d_6$) δ 9.67 (s, 1H), 9.05 (d, J = 10.0 Hz, 1H), 8.34 (dd, J = 10.0, 1.5 Hz, 1H), 7.98 (td, J = 10.0 1.5 Hz, 1H), 7.88 (td, J = 10.0, 1.0 Hz, 1H), 4.67 (q, J = 7.0 Hz, 2H), 4.60 (t, J = 7.0 Hz, 2H), 4.13 (s, 3H), 1.94-1.82 (m, 2H), 1.50 (t, J = 7.0 Hz, 3H), 1.42-1.33 (m, 2H), 0.95 (t, J = 7.5 Hz, 3H).

$^{13}C$ NMR (126 MHz, DMSO-$d_6$) δ 173.97, 143.54, 138.27, 135.36, 135.06, 132.45, 131.55, 131.19, 127.70, 126.73, 125.24, 74.48, 49.06, 39.33, 31.67, 19.28, 15.04, 13.87.

HRMS(ESI-TOF) m/z theoretical value: $C_{18}H_{22}N_3O_2[M - I]^+$, 312.1707, measured value: 312.1707.

Compound 121:

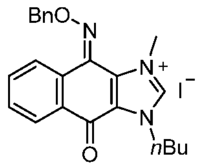

(E) and (Z)-4-((benzyloxy)imino)-1-butyl-3-methyl-9-oxo-4,9-dihydro-1H-naphthol [2,3-d] imidazole-3-iodide

yellow solid, m. p. 122-124°C.

$^1H$ NMR (500 MHz, DMSO-$d_6$) δ 9.62 (s, 1H), 9.09 (d, J = 10.0 Hz, 1H), 8.34 (dd, J = 10.0, 1.0 Hz, 1H), 8.01-7.94 (m, 1H), 7.88 (td, J = 10.0, 1.0 Hz, 1H), 7.61-7.54 (m, 2H), 7.48-7.37 (m, 3H), 5.68 (s, 2H), 4.57 (t, J = 7.0 Hz, 2H), 4.03 (s, 3H), 1.90-1.77 (m, 2H), 1.41-1.32 (m, 2H), 0.97-0.90 (m, 3H).

$^{13}C$ NMR (126 MHz, DMSO) δ 173.94, 143.58, 138.73, 136.71, 135.39, 134.89, 132.57, 131.60, 131.25, 129.47, 129.21, 129.18, 127.76, 126.70, 125.35, 80.08, 49.03, 39.33, 31.66, 19.24, 13.85.

HRMS(ESI-TOF) m/z theoretical value: $C_{23}H_{23}N_3O_2[M - I]^+$, 374.1863, measured value: 374.1863.

Compound 122:

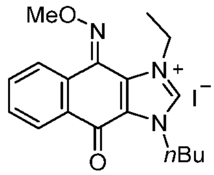

(E) and (Z)-1-butyl-3-ethyl-4-(methoxylimino)-9-oxo-4,9-dihydro-1H-naphthol [2,3-d] imidazole-3-iodide

yellow solid, m. p. 135-137°C.

$^1H$ NMR (500 MHz, DMSO-$d_6$) δ 9.71 (s, 1H), 9.02 (d, J = 10.0 Hz, 1H), 8.34 (d, J = 10.0, 1H), 7.97 (td, J = 10.0, 1.5 Hz, 1H), 7.88 (t, J = 10.0, 1H), 4.65-4.55 (m, 5H), 4.41 (s, 3H), 1.92-1.83 (m, 3H), 1.54 (t, J = 7.0 Hz, 3H), 1.47-1.35 (m, 3H), 0.95 (t, J = 7.0 Hz, 4H).

$^{13}C$ NMR (126 MHz, DMSO-$d_6$) δ 174.09, 142.86, 138.14, 135.34, 134.19, 132.51, 131.70, 131.11, 127.70,

126.81, 125.71, 66.27, 49.20, 47.07, 31.65, 19.35, 14.86, 13.89.

HRMS(ESI-TOF) m/z theoretical value: $C_{18}H_{22}N_3O_2$[M - I]$^+$, 312.1707, measured value: 312.1708.

Compound 123:

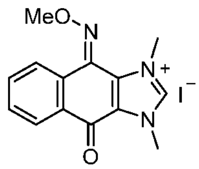

(E) and (Z)-4-(methoxylimino)-1,3-dimethyl -9-oxo-4,9-dihydro-1H-naphthol [2,3-d] imidazole-3-iodide

yellow solid, m. p. 167-169°C.

$^1$H NMR (400 MHz, DMSO-$d_6$) δ 9.58 (s, 1H), 9.01 (d, J = 8.0, 1H), 8.33 (dd, J = 4.0, 1.2 Hz, 1H), 7.97 (td, J = 8.0, 1.6 Hz, 1H), 7.88 (td, J = 8.0, 1.2 Hz, 1H), 4.41 (s, 3H), 4.18 (s, 3H), 4.13 (s, 3H).

$^{13}$C NMR (101 MHz, DMSO) δ 174.16, 144.05, 138.25, 135.38, 134.26, 132.56, 131.72, 131.10, 127.63, 126.76, 125.85, 66.37, 39.08, 36.44.

HRMS(ESI-TOF) m/z theoretical value: $C_{14}H_{14}N_3O_2$[M - I]$^+$, 256.1081, measured value: 256.1085.

Compound 124:

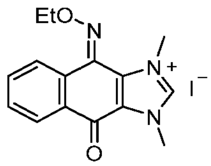

(E) and (Z)-4-(ethoxylimino)-1,3-dimethyl -9-oxo-4,9-dihydro-1H-naphthol [2,3-d] imidazole-3-iodide

yellow solid, m. p. 165-167°C.

$^1$H NMR (500 MHz, DMSO-$d_6$) δ 9.57 (s, 1H), 9.06 (dd, J = 10.0, 1.0 Hz, 1H), 8.34 (dd, J = 10.0, 1.0 Hz, 1H), 7.98 (td, J = 10.0, 1.5 Hz, 1H), 7.88 (td, J = 5.0, 1.0 Hz, 1H), 4.67 (q, J = 7.0 Hz, 2H), 4.18 (s, 3H), 4.13 (s, 3H), 1.50 (t, J = 7.0 Hz, 3H).

$^{13}$C NMR (126 MHz, DMSO-$d_6$) δ 174.18, 144.02, 138.15, 135.39, 134.49, 132.49, 131.62, 131.11, 127.63, 126.81, 125.80, 74.48, 39.12, 36.43, 15.02.

HRMS(ESI-TOF) m/z theoretical value: $C_{15}H_{15}N_3O_2$[M - I]$^+$, 270.1243, measured value: 270.1244.

Compound 125:

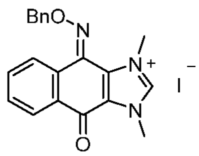

(E)-4-((benzyloxy)imino)-1,3-dimethyl-9-oxo-4,9-dihydro-1H-naphthol [2,3-d] imidazole-3-iodide

yellow solid, m. p. 177-179°C.

$^1$H NMR (500 MHz, DMSO-$d_6$) δ 9.53 (s, 1H), 9.08 (dd, J = 10.0, 1.0 Hz, 1H), 8.33 (dd, J = 10.0, 1.0 Hz, 1H), 8.01-7.93 (m, 1H), 7.88 (td, J = 10.0, 1.0 Hz, 1H), 7.60-7.55 (m, 2H), 7.49-7.38 (m, 3H), 5.68 (s, 2H), 4.16 (s, 3H), 4.03 (s, 3H).

$^{13}$C NMR (126 MHz, DMSO-$d_6$) δ 174.15, 144.04, 138.61, 136.66, 135.42, 134.32, 132.61, 131.66, 131.16, 129.49, 129.23, 129.19, 127.67, 126.77, 125.90, 80.09, 39.13, 36.39.

(Z)-4-((benzyloxy)imino)-1,3-dimethyl-9-oxo-4,9-dihydro-1H-naphthol [2,3-d] imidazole-3-iodide

$^1$H NMR (400 MHz, DMSO-$d_6$) δ 9.57 (s, 1H), 8.22 (d, J = 8.1 Hz, 1H), 8.15 (d, J = 7.8 Hz, 1H), 7.86 (t, J = 7.7 Hz, 1H), 7.75 (t, J = 7.6 Hz, 1H), 7.54 (d, J = 7.1 Hz, 2H), 7.41 (d, J = 8.2 Hz, 3H), 5.57 (s, 2H), 4.13 (s, 3H), 4.00 (s, 3H).

(E) and (Z)-4-((benzyloxy)imino)-1,3-dimethyl -9-oxo-4,9-dihydro-1H-naphthol [2,3-d] imidazole-3-iodide

$^1$H NMR (400 MHz, DMSO-$d_6$) δ 9.55 (s, 3H), 9.50 (s, 1H), 9.04 (d, J = 8.0 Hz, 1H), 8.29 (dd, J = 8.0, 1.6 Hz, 1H), 8.20 (dd, J = 8.0, 1.2 Hz, 3H), 8.13 (dd, J = 7.8, 1.4 Hz, 3H), 7.97 - 7.90 (m, 1H), 7.84 (td, J = 7.6, 1.6 Hz, 4H), 7.73 (td, J = 7.6, 1.2 Hz, 3H), 7.59 - 7.48 (m, 8H), 7.47 - 7.33 (m, 12H), 5.64 (s, 2H), 5.55 (s, 6H), 4.11 (s, 3H), 4.11 (s, 6H), 3.99 (s, 3H), 3.98 (s, 6H).

HRMS(ESI-TOF) m/z theoretical value: $C_{20}H_{18}N_3O_2$[M - I]$^+$, 332.1394, measured value: 332.1402.

Compound 126:

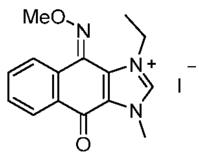

(E) and (Z)-3-ethyl-4-(methoxylimino)-1-methyl-9-oxo-4,9-dihydro-1H-naphthol [2,3-d] imidazole-3-iodide

yellow solid, m. p. 162-164°C.

$^1$H NMR (500 MHz, DMSO-$d_6$) δ 9.57 (s, 1H), 9.06 (d, J = 10.0 Hz, 1H), 8.34 (dd, J = 10.0, 2.0 Hz, 1H), 7.98 (td, J = 10.0, 2.0 Hz, 1H), 7.88 (td, J = 10.0, 1.5 Hz, 1H), 4.67 (q, J = 7.0 Hz, 2H), 4.18 (s, 3H), 4.13 (s, 3H), 1.50 (t, J = 7.0 Hz, 3H).

$^{13}$C NMR (126 MHz, DMSO-$d_6$) δ 174.18, 144.02, 138.15, 135.39, 134.49, 132.49, 131.62, 131.11, 127.63, 126.81, 125.80, 74.48, 39.12, 36.43, 15.02.

HRMS(ESI-TOF) m/z theoretical value: $C_{15}H_{16}N_3O_2$[M - I]$^+$, 270.1237, measured value: 270.1241.

**Example 13**

Compound 1 (0.05 mmol) and 150 μL of iodomethane were added to 3 mL of acetonitrile for reaction for 8 h at 100°C. At the end of the reaction, solvent was removed to obtain a yellow solid, and the solid was pulped for half an hour and subjected to suction filtration to obtain the final product.

Different synthetic intermediate compounds above were used to prepare Compounds 127-151 according to the method in Example 13; other materials and reaction conditions for the compounds were kept the same and raw materials could be purchased commercially:

Compound 127:

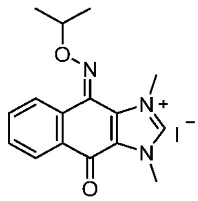

(E) and (Z)-4-(isopropoxyimino)-1,3-dimethyl -9-oxo-4,9-dihydro-1H-naphthol [2,3-d] imidazole-3-iodide

yellow solid, m. p. 187-189°C.

$^1$H NMR (400 MHz, DMSO-$d_6$) δ 9.58 (s, 1H), 9.07 (dd, J = 8.0, 0.4 Hz, 1H), 8.34 (dd, J = 8.0, 1.2 Hz, 1H), 8.03-7.95 (m, 1H), 7.88 (td, J = 8.0, 1.2 Hz, 1H), 4.92-4.82 (m, 1H), 4.18 (s, 3H), 4.14 (s, 3H), 1.51 (d, J = 8.0 Hz, 6H).

$^{13}$C NMR (101 MHz, DMSO-$d_6$) δ 174.16, 143.98, 137.79, 135.40, 134.67, 132.39, 131.52, 131.09, 127.60, 126.84, 125.73, 81.42, 39.23, 36.45, 21.77.

HRMS(ESI-TOF) m/z theoretical value: $C_{16}H_{18}N_3O_2$[M - I]$^+$, 284.1394, measured value: 284.1399.

Compound 128:

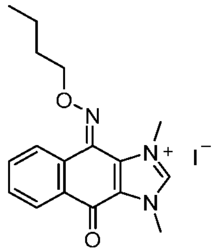

(E) and (Z)-4-(butoxyimino)-1,3-dimethyl-9-oxo-4,9-dihydro-1H-naphthol [2,3-d] imidazole-3-iodide

yellow solid, m. p. 201-203°C.

$^{1}H$ NMR (500 MHz, DMSO-$d_6$) δ 9.59 (s, 1H), 9.03 (dd, J = 8.0, 1.0 Hz, 1H), 8.34 (dd, J = 8.0, 1.5 Hz, 1H), 8.02-7.93 (m, 1H), 7.90-7.85 (m, 1H), 4.64 (t, J = 6.5 Hz, 2H), 4.18 (s, 3H), 4.12 (s, 3H), 1.95-1.77 (m, 2H), 1.53-1.42 (m, 2H), 0.98 (t, J = 7.5 Hz, 3H).

$^{13}C$ NMR (126 MHz, DMSO-$d_6$) δ 174.16, 144.02, 138.15, 135.43, 134.46, 132.49, 131.48, 131.10, 127.64, 126.80, 125.78, 78.53, 39.11, 36.43, 31.07, 19.09, 14.20.

HRMS(ESI-TOF) m/z theoretical value: $C_{17}H_{20}N_3O_2[M - I]^+$, 298.1550, measured value: 298.1551.

Compound 129:

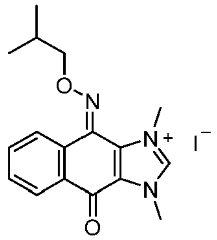

(E) and (Z)-4-(isobutoxyimino)-1,3-dimethyl -9-oxo-4,9-dihydro-1H-naphthol [2,3-d] imidazole-3-iodide

yellow solid, m. p. 139-141 °C.

$^{1}H$ NMR (500 MHz, DMSO-$d_6$) δ 9.57 (s, 1H), 9.03 (J = 10.0 Hz, 1H), 8.35 (dd, J = 10.0, 1.5 Hz, 1H), 8.05-7.98 (m, 1H), 7.89 (t, J = 7.5 Hz, 1H), 4.44 (d, J = 6.5 Hz, 2H), 4.18 (s, 3H), 4.12 (s, 3H), 2.30-2.15 (m, 1H), 1.05 (d, J = 5.0 Hz, 6H).

$^{13}C$ NMR (126 MHz, DMSO-$d_6$) δ 174.16, 144.03, 138.16, 135.45, 134.47, 132.51, 131.41, 131.13, 127.69, 126.82, 125.81, 84.91, 39.11, 36.43, 28.32, 19.37.

HRMS(ESI-TOF) m/z theoretical value: $C_{17}H_{20}N_3O_2[M - I]^+$, 298.1550, measured value: 298.1551.

Compound 130:

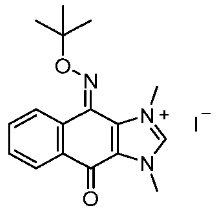

(E) and (Z)-4-(tert-butoxyimino)-1,3-dimethyl -9-oxo-4,9-dihydro-1H-naphthol [2,3-d] imidazole-3-iodide

yellow solid, m. p. 221-223 °C.

$^{1}H$ NMR (500 MHz, DMSO-$d_6$) δ 9.59 (s, 1H), 9.10 (dd, J = 10.0 Hz, 1H), 8.35 (dd, J = 5.0, 1.5 Hz, 1H), 8.02-7.95 (m, 1H), 7.91-7.85 (m, 1H), 4.19 (s, 3H), 4.15 (s, 3H), 1.56 (s, 9H).

$^{13}C$ NMR (126 MHz, DMSO-$d_6$) δ 174.19, 143.95, 137.64, 135.39, 134.97, 132.32, 131.49, 131.12, 127.59,

126.94, 125.70, 85.60, 39.44, 36.44, 27.70, 27.65.

HRMS(ESI-TOF) m/z theoretical value: $C_{17}H_{20}N_3O_2[M - I]^+$, 298.1550, measured value: 298.1551.

Compound 131:

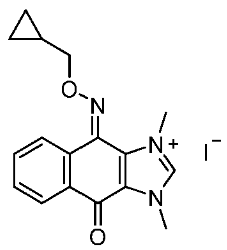

(E) and (Z)-4-((cyclopropylmethoxy)imino)-1,3-dimethyl -9-oxo-4,9-dihydro-1H-naphthol [2,3-d] imidazole-3-iodide

yellow solid, m. p. 170-172 °C.

$^1$H NMR (400 MHz, DMSO-$d_6$) δ 9.58 (s, 1H), 9.09 (dd, J = 8.0, 0.4 Hz, 1H), 8.34 (dd, J = 8.0, 1.6 Hz, 1H), 8.03-7.95 (m, 1H), 7.88 (td, J = 8.0, 1.2 Hz, 1H), 4.47 (d, J = 8.0 Hz, 2H), 4.18 (s, 4H), 4.13 (s, 3H), 1.47-1.35 (m, 1H), 0.70-0.62 (m, 2H), 0.51-0.42 (m, 2H).

$^{13}$C NMR (101 MHz, DMSO-$d_6$) δ 174.17, 144.01, 1310.08, 135.38, 134.48, 132.46, 131.56, 131.11, 127.62, 126.88, 125.79, 83.19, 39.14, 36.44, 10.70, 3.76.

HRMS(ESI-TOF) m/z theoretical value: $C_{17}H_{18}N_3O_2[M - I]^+$, 296.1394, measured value: 296.1397.

Compound 132:

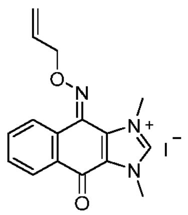

(E) and (Z)-4-((allyloxy)imino)-1,3-dimethyl -9-oxo-4,9-dihydro-1H-naphthol [2,3-d] imidazole-3-iodide

yellow solid, m. p. 139-141 °C.

$^1$H NMR (400 MHz, DMSO-$d_6$) δ 9.57 (s, 1H), 9.04 (dd, J = 8.0, 1.2 Hz, 1H), 8.34 (dd, J = 8.0, 1.2 Hz, 1H), 7.99 (td, J = 8.0, 1.2 Hz, 1H), 7.89 (td, J = 8.0, 1.2 Hz, 1H), 6.28-6.15 (m, 1H), 5.58-5.50 (m, 1H), 5.46-5.39 (m, 1H), 5.12 (d, J = 8.0 Hz, 2H), 4.18 (s, 3H), 4.10 (s, 3H).

$^{13}$C NMR (101 MHz, DMSO-$d_6$) δ 174.16, 144.07, 138.48, 135.41, 134.38, 133.51, 132.59, 131.66, 131.13, 127.67, 126.74, 125.88, 120.67, 79.10, 39.17, 36.44.

HRMS(ESI-TOF) m/z theoretical value: $C_{16}H_{16}N_3O_2[M - I]^+$, 282.1237, measured value: 282.1239.

Compound 133:

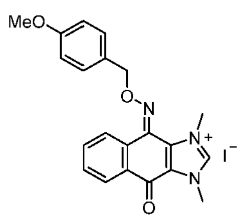

(E) and (Z)-4-(((4-methoxybenzyl)oxy)imino)-1,3-dimethyl -9-oxo-4,9-dihydro-1H-naphthol [2,3-d] imidazole-3-iodide

$^1$H NMR (400 MHz, DMSO-$d_6$) δ 9.54 (s, 1H), 9.05 (dd, J = 10.0, 0.8 Hz, 1H), 8.32 (dd, J = 8.0, 1.2 Hz, 1H),

8.02-7.92 (m, 1H), 7.86 (td, J = 8.0, 1.2 Hz, 1H), 7.56-7.49 (m, 2H), 7.03-6.95 (m, 2H), 5.60 (s, 2H), 4..16 (s, 3H), 4.06 (s, 3H), 3.77 (s, 3H).

$^{13}C$ NMR (101 MHz, DMSO-$d_6$) δ 174.13, 160.14, 144.02, 138.38, 135.38, 134.37, 132.53, 131.58, 131.44, 131.11, 128.50, 127.63, 126.79, 125.83, 114.51, 79.91, 55.65, 39.22, 36.41.

HRMS(ESI-TOF) m/z theoretical value: $C_{21}H_{20}N_3O_3[M-I]^+$, 363.11499, measured value: 363.1499.

Compound 134:

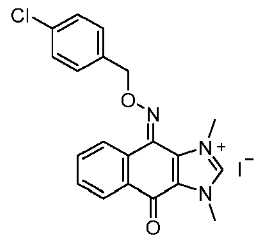

(E) and (Z)-4-(((4-chlorobenzyl)oxy)imino)-1,3-dimethyl -9-oxo-4,9-dihydro-1H-naphthol [2,3-d] imidazole-3-iodide

$^1H$ NMR (400 MHz, DMSO-$d_6$) δ 9.54 (s, 1H), 9.07 (dd, J = 8.0, 0.8 Hz, 1H), 8.33 (dd, J = 8.0, 1.6 Hz, 1H), 8.01-.95 (m, 1H), 7.88 (td, J = 8.0, 1.2 Hz, 1H), 7.65-7.58 (m, 2H), 7.55-7.48 (m, 2H), 5.67 (s, 2H), 4.16 (s, 3H), 4.02 (s, 3H).

$^{13}C$ NMR (101 MHz, DMSO-$d_6$) δ 174.14, 144.05, 138.81, 135.74, 135.43, 134.25, 133.88, 132.66, 131.75, 131.41, 131.16, 129.19, 127.68, 126.73, 125.93, 78.99, 39.17, 36.41.

HRMS(ESI-TOF) m/z theoretical value: $C_{20}H_{17}ClN_3O_2[M-I]^+$, 366.1004, measured value: 366.1006.

Compound 135:

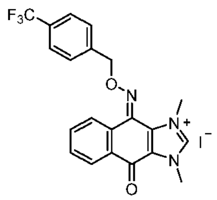

(E) and (Z)-1,3-dimethyl -9-oxo-4-(((4-(trifluoromethyl)benzyl)oxy)imino)-4,9-dihydro-1H-naphthol [2,3-d] imidazole-3-iodide

yellow solid, m. p. 158-160 °C.

$^1H$ NMR (400 MHz, DMSO-$d_6$) δ 9.53 (s, 1H), 9.11 (dd, J = 8.0, 0.8 Hz, 1H), 8.34 (dd, J = 8.0, 0.8 Hz, 1H), 7.99 (td, J = 8.0, 1.6 Hz, 1H), 7.89 (td, J = 8.0, 1.2 Hz, 1H), 7.86-7.76 (m, 4H), 5.78 (s, 2H), 4.16 (s, 3H), 4.00 (s, 3H).

$^{13}C$ NMR (101 MHz, DMSO-$d_6$) δ 174.14, 144.07, 141.53, 139.03, 135.46, 134.20, 132.73, 131.84, 131.19, 129.63, 129.32, 127.71, 126.72, 126.11 (126.08, 126.04, 126.00, J = 4.0 Hz), 123.26, 78.84, 39.12, 36.41.

HRMS (ESI): m/z theoretical value: $C_{21}H_{17}F_3N_3O_2$ $[M-I]^+$, 400.1267, measured value: 400.1268.

Compound 136:

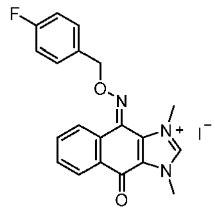

(E) and (Z)-4-(((4-fluorobenzyl)oxy)imino)-1,3-dimethyl -9-oxo-4,9-dihydro-1H-naphthol [2,3-d] imidazole-3-iodide

yellow solid, m. p. 175-177 °C.

$^1H$ NMR (400 MHz, DMSO-$d_6$) δ 9.54 (s, 1H), 9.07 (d, J = 8.0 Hz, 1H), 8.32 (dd, J = 8.0, 1.2 Hz, 1H), 7.97 (td, J = 8.0, 1.6 Hz, 1H), 7.88 (td, J = 8.0, 1.2 Hz, 1H), 7.69-7.56 (m, 2H), 7.35-7.24 (m, 2H), 5.66 (s, 2H), 4.16 (s, 3H), 4.03 (s, 3H).

$^{13}C$ NMR (101 MHz, DMSO-$d_6$) δ 174.14, 163.99 (161.55, J = 244.0 Hz), 144.04, 138.68, 135.40, 134.29, 133.02 (132.99, J = 3.0 Hz), 132.62, 131.98 (131.89, J = 9.0 Hz), 131.70, 131.15, 127.66, 126.75, 125.91, 116.14 (115.93, J = 21.0 Hz), 79.14, 39.18, 36.41.

HRMS(ESI-TOF) m/z theoretical value: $C_{20}H_{17}FN_3O_2[M-I]^+$, 350.1299, measured value: 350.1302.

Compound 137:

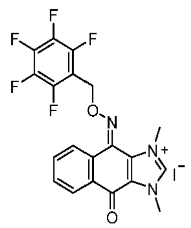

(E) and (Z)-1,3-dimethyl-9-oxo-4-(((perfluorophenyl)methoxy)imino)-4,9-dihydro-1H-naphthol [2,3-d] imidazole-3-iodide

$^1H$ NMR (400 MHz, DMSO-$d_6$) δ 9.54 (s, 1H), 8.94 (d, J = 8.0 Hz, 1H), 8.30 (d, J = 8.0 Hz, 1H), 8.02-7.92 (m, 1H), 7.87 (t, J = 8.0 Hz, 1H), 5.78 (s, 2H), 4.14 (s, 3H), 4.03 (s, 3H).

$^{13}C$ NMR (126 MHz, DMSO-$d_6$) δ 175.44, 174.10, 144.09, 139.74, 135.84, 135.44, 133.92, 132.92, 132.27, 131.84, 131.26, 127.79, 127.39, 126.66, 126.23, 66.49, 38.71, 36.47.

HRMS (ESI): m/z theoretical value: $C_{20}H_{13}F_5N_3O_2[M-I]^+$, 422.0922, measured value: 422.0920.

Compound 138:

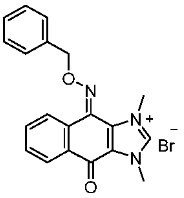

(E) and (Z)-4-((benzyloxy)imino)-1,3-dimethyl -9-oxo-4,9-dihydro-1H-naphthol [2,3-d] imidazole-3-iodide

$^1H$ NMR (500 MHz, DMSO-$d_6$) δ 9.53 (s, 1H), 9.08 (dd, J = 10.0, 1.0 Hz, 1H), 8.33 (dd, J = 10.0, 1.0 Hz, 1H), 8.01-7.93 (m, 1H), 7.88 (td, J = 10.0, 1.0 Hz, 1H), 7.60-7.55 (m, 2H), 7.49-7.38 (m, 3H), 5.68 (s, 2H), 4.16 (s, 3H), 4.03 (s, 3H).

$^{13}C$ NMR (126 MHz, DMSO-$d_6$) δ 174.15, 144.04, 138.61, 136.66, 135.42, 134.32, 132.61, 131.66, 131.16, 129.49, 129.23, 129.19, 127.67, 126.77, 125.90, 80.09, 39.13, 36.39.

HRMS(ESI-TOF) m/z theoretical value: $C_{20}H_{18}N_3O_2[M - I]^+$, 322.1394, measured value: 322.1393.

Compound 139:

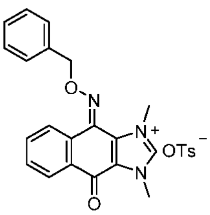

(E) and (Z)-4-((benzyloxy)imino)-1,3-dimethyl -9-oxo-4,9-dihydro-1H-naphthol [2,3-d] imidazole-3-p-toluenesulfonamide

$^1H$ NMR (500 MHz, DMSO-$d_6$) δ 9.53 (s, 1H), 9.08 (dd, J = 10.0, 1.0 Hz, 1H), 8.33 (dd, J = 10.0, 1.0 Hz, 1H), 8.01-7.93 (m, 1H), 7.88 (td, J = 10.0, 1.0 Hz, 1H), 7.60-7.55 (m, 2H), 7.49-7.38 (m, 3H), 5.68 (s, 2H), 4.16 (s, 3H), 4.03 (s, 3H).
$^{13}C$ NMR (126 MHz, DMSO-$d_6$) δ 174.15, 144.04, 138.61, 136.66, 135.42, 134.32, 132.61, 131.66, 131.16, 129.49, 129.23, 129.19, 127.67, 126.77, 125.90, 80.09, 39.13, 36.39.
HRMS(ESI-TOF) m/z theoretical value: $C_{20}H_{18}N_3O_2$[M - I]$^+$, 322.1394, measured value: 322.1393.

Compound 140:

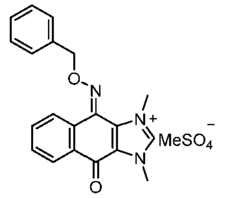

(E) and (Z)-4-((benzyloxy)imino)-1,3-dimethyl -9-oxo-4,9-dihydro-1H-naphthol [2,3-d] imidazole-3-dimethyl sulfate

$^1H$ NMR (500 MHz, DMSO-$d_6$) δ 9.53 (s, 1H), 9.08 (dd, J = 10.0, 1.0 Hz, 1H), 8.33 (dd, J = 10.0, 1.0 Hz, 1H), 8.01-7.93 (m, 1H), 7.88 (td, J = 10.0, 1.0 Hz, 1H), 7.60-7.55 (m, 2H), 7.49-7.38 (m, 3H), 5.68 (s, 2H), 4.16 (s, 3H), 4.03 (s, 3H).
$^{13}C$ NMR (126 MHz, DMSO-$d_6$) δ 174.15, 144.04, 138.61, 136.66, 135.42, 134.32, 132.61, 131.66, 131.16, 129.49, 129.23, 129.19, 127.67, 126.77, 125.90, 80.09, 39.13, 36.39.
HRMS(ESI-TOF) m/z theoretical value: $C_{20}H_{18}N_3O_2$[M - I]$^+$, 322.1394, measured value: 322.1395.

Compound 141:

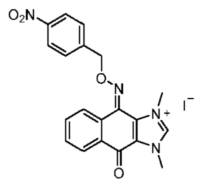

(E) and (Z) -1,3-dimethyl-4-(((4-nitrobenzyl)oxy)imino)-9-oxo-4,9-dihydro-1H-naphthol [2,3-d] imidazole-3-iodide

$^1H$ NMR (500 MHz, DMSO-$d_6$) δ 9.54 (s, 1H), 9.11 (d, J = 10.0 Hz, 1H), 8.35-8.28 (m, 3H), 8.00 (td, J = 10.0, 1.5 Hz, 1H), 7.90 (td, J = 10.0, 1.0 Hz, 1H), 7.84 (d, J = 10.0 Hz, 2H), 5.83 (s, 2H), 4.16 (s, 3H), 3.98 (s, 3H).
$^{13}C$ NMR (126 MHz, DMSO-$d_6$) δ 174.14, 147.97, 144.44, 144.09, 139.22, 135.47, 134.14, 132.79, 131.92, 131.21, 130.21, 127.74, 126.70, 126.04, 124.28, 78.38, 39.14, 36.44.
HRMS(ESI-TOF) m/z theoretical value: $C_{20}H_{17}N_4O_4$[M - I]$^+$, 377.1244, measured value: 377.1247.

Compound 142:

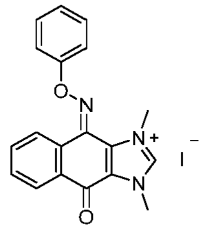

(E) and (Z)-4-((phenoxy)imino)-1,3-dimethyl -9-oxo-4,9-dihydro-1H-naphthol [2,3-d] imidazole-3-iodide

$^1H$ NMR (400 MHz, DMSO-$d_6$) δ 9.62 (s, 3H), 9.56 (s, 1H), 9.07 (d, J = 8.0 Hz, 1H), 8.35 (dd, J = 8.0, 1.6 Hz, 1H), 8.29 (dd, J = 8.0, 1.2 Hz, 3H), 8.18 (dd, J = 7.8, 1.4 Hz, 3H), 8.03 - 7.95 (m, 1H), 7.89 (td, J = 7.6, 1.6 Hz, 4H), 7.79 (td, J = 7.6, 1.2 Hz, 3H), 7.69 - 7.58 (m, 8H), 7.49 - 7.36 (m, 12H), 4.14 (s, 3H), 4.13 (s, 6H), 4.02 (s, 3H), 4.00 (s, 6H).

HRMS(ESI-TOF) m/z theoretical value: $C_{20}H_{18}N_3O_2$[M - I]$^+$, 318.1237, measured value: 318.1238.

Compound 143:

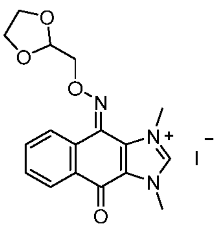

(E)-4-(((1,3-dioxoheterocycle-2-yl)methoxy)imino)-1,3-dimethyl-9-oxo-4,9-dihydro-1H-naphthol [2,3-d] imidazole-3-iodide

$^1H$ NMR (400 MHz, DMSO-$d_6$) δ 9.56 (s, 1H), 9.03 (d, J = 8.0 Hz, 1H), 8.35 (d, J = 8.0 Hz, 1H), 7.98 (t, J = 8.0 Hz, 1H), 7.87 (t, J = 8.0 Hz, 1H), 5.46 (t, J = 4.0 Hz, 2H), 4.18 (s, 3H), 4.06 (s, 3H), 4.01 (t, J = 4.0 Hz, 2H), 3.98 (s, J = 4.0 Hz, 2H).

HRMS(ESI-TOF) m/z theoretical value: $C_{20}H_{18}N_3O_2$[M-I]$^+$, 328.1292, measured value: 328.1297.

Compound 144:

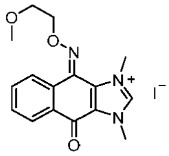

(E)-4-((2-methoxyethoxy)imino)-1,3-dimethyl -9-oxo-4,9-dihydro-1H-naphthol [2,3-d] imidazole-3-iodide

$^1H$ NMR (400 MHz, DMSO-$d_6$) δ 9.55 (s, 1H), 9.02 (d, J = 8.0 Hz, 1H), 8.32 (d, J = 8.0 Hz, 1H), 7.96 (t, J = 8.0 Hz, 1H), 7.86 (t, J = 8.0 Hz, 1H), 4.73 (t, J = 4.0 Hz, 2H), 4.16 (s, 3H), 4.11 (s, 3H), 3.81 (t, J = 4.0 Hz, 2H), 3.32 (s, 3H).

$^{13}C$ NMR (126 MHz, DMSO-$d_6$) δ 174.17, 144.02, 138.40, 135.39, 134.38, 132.57, 131.62, 131.14, 127.65, 126.78, 125.88, 77.73, 70.56, 58.77, 39.12, 36.45.

HRMS(ESI-TOF) m/z theoretical value: $C_{16}H_{18}N_3O_3$[M-I]$^+$, 300.1343, measured value: 300.1347.

Compound 145:

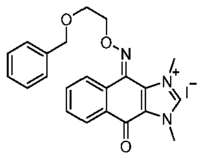

(E)-4-((2-(benzyloxy)ethoxy)imino)-1,3-dimethyl-9-oxo-4,9-dihydro-1H-naphthol [2,3-d] imidazole-3-iodide

$^1H$ NMR (400 MHz, DMSO-$d_6$) δ 9.53 (s, 1H), 9.04 (d, J = 8.0 Hz, 1H), 8.32 (d, J = 8.0 Hz, 1H), 7.94 (t, J = 8.0 Hz, 1H), 7.87 (t, J = 8.0 Hz, 1H), 7.34-7.19 (m, 4H), 4.76 (t, J = 4.0 Hz, 2H), 4.56 (s, 2H), 4.16 (s, 3H), 4.03 (s, 3H), 3.90 (t, J = 4.0 Hz, 2H).

$^{13}C$ NMR (126 MHz, DMSO-$d_6$) δ 174.17, 144.00, 138.69, 138.43, 135.32, 134.33, 132.59, 131.65, 131.15, 128.71, 1210.04, 127.98, 127.65, 126.79, 125.87, 77.75, 72.48, 68.19, 39.03, 36.46.

HRMS(ESI-TOF) m/z theoretical value: $C_{22}H_{22}N_3O_3$[M-I]$^+$, 376.1656, measured value: 376.1656.

Compound 146:

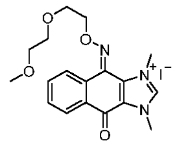

(E)-4-((2-(2-methoxyethoxy)ethoxy)imino)-1,3-dimethyl -9-oxo-4,9-dihydro-1H-naphthol [2,3-d] imidazole-3-iodide

$^{1}H$ NMR (400 MHz, DMSO-$d_6$) δ 9.58 (s, 1H), 9.07 (dd, J = 8.0, 0.8 Hz, 1H), 8.34 (dd, J = 8.0, 1.2 Hz, 1H), 8.00-7.93 (m, 1H), 7.89 (td, J = 8.0, 1.2 Hz, 1H), 4.82-4.69 (m, 2H), 4.18 (s, 3H), 4.13 (s, 3H), 3.95-3.86 (m, 2H), 3.68-3.59 (m, 2H), 3.53-3.44 (m, 2H), 3.23 (s, 3H).

$^{13}C$ NMR (101 MHz, DMSO-$d_6$) δ 174.17, 144.01, 138.40, 135.34, 134.37, 132.57, 131.68, 131.13, 127.62, 126.77, 125.86, 77.83, 71.77, 70.18, 69.12, 58.57, 39.08, 36.43.

HRMS(ESI-TOF) m/z theoretical value: $C_{18}H_{22}N_3O_4[M - I]^+$, 344.1605, measured value: 344.1609.

Compound 147:

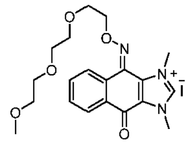

(E) and (Z)-4-((2-(2-(2-methoxyethoxy)ethoxy)ethoxy)imino)-1,3-dimethyl -9-oxo-4,9-dihydro-1H-naphthol [2,3-d] imidazole-3-iodide

$^{1}H$ NMR (400 MHz, DMSO-$d_6$) δ 9.63 (s, 1H), 9.56 (s, 1H), 9.02 (d, J = 8.0 Hz, 1H), 8.34-8.09 (m, 3H), 8.01-7.70 (m, 4H), 4.78-4.58 (m, 4H), 4.21-4.01 (m, 11H), 3.90-3.80 (m, 4H), 3.63- 3.42 (m, 10H), 3.40-3.30 (m, 5H), 3.16 (d, J = 12.0 Hz, 5H).

$^{13}C$ NMR (101 MHz, DMSO) δ 175.23, 174.18, 144.73, 144.01, 138.39, 137.03, 135.84, 135.36, 134.81, 133.19, 132.56, 131.67, 131.13, 131.03, 130.53, 129.74, 128.12, 127.62, 127.38, 126.61, 125.85, 125.38, 77.84, 76.78, 71.73, 70.42, 70.26, 70.22, 70.10, 70.05, 70.01, 69.12, 68.72, 58.54, 58.50, 41.50, 39.09, 36.70, 36.62, 36.42.

HRMS(ESI-TOF) m/z theoretical value: $C_{20}H_{26}N_3O_5[M-I]^+$, 388.1867, measured value: 388.1866.

Compound 148:

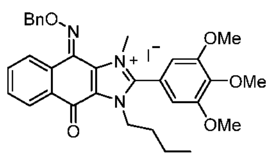

(E) and (Z)-4-((benzyloxy)imino)-1-butyl-3-methyl-9-oxo-2-(3,4,5-trimethoxyphenyl)-4,9-dihydro-1H-naphthol [2,3-d] imidazole-3-iodide

$^{1}H$ NMR (400 MHz, DMSO-$d_6$) δ 9.01 (d, J = 8.1 Hz, 1H), 8.27 (dd, J = 7.8, 1.6 Hz, 1H), 8.17 - 8.08 (m, 5H), 7.93 - 7.89 (m, 1H), 7.85 - 7.79 (m, 3H), 7.72 (t, J = 7.4 Hz, 3H), 7.54 - 7.47 (m, 7H), 7.45-7.30 (m, 9H), 5.64 (s, 2H), 5.54 (s, 2H), 5.53 (s, 3H), 4.10 (d, J = 3.4 Hz, 8H), 4.02 (s, 2H), 3.95 (s, 3H), 3.84 (s, 6H), 2.76 (s, 3H), 2.75 (s, 5H), 2.67 (s, 3H).

HRMS(ESI-TOF) m/z theoretical value: $C_{20}H_{26}N_3O_5[M-I]^+$, 540.2493, measured value: 540.2495.

Compound 149:

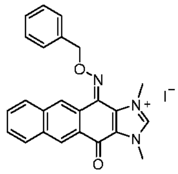

(E) and (Z)-4-((benzyloxy)imino)-1,3-dimethyl -11-oxo-4,11-dihydro-1H-anthracene [2,3-d] imidazole-3-iodide

$^1$H NMR (400 MHz, DMSO-$d_6$) δ 9.67 (s, 1H), 9.60 (s, 1H), 9.54 (s, 1H), 8.88 (s, 1H), 8.82 (s, 1H), 8.74 (s, 1H), 8.31 - 8.13 (m, 4H), 7.84 - 7.68 (m, 4H), 7.68-7.55 (m, 4H), 7.51 - 7.38 (m, 6H), 5.71 (s, 2H), 5.61 (s, 2H), 4.17 (m, 6H), 4.02 (s, 3H), 4.00 (s, 3H).
$^{13}$C NMR (101 MHz, DMSO-$d_6$) δ 174.07, 144.77, 144.17, 138.54, 137.03, 136.19, 135.13, 135.08, 133.61, 132.73, 130.69, 130.54, 130.41, 130.37, 130.17, 130.10, 129.85, 129.75, 129.40, 129.26, 129.23, 129.17, 129.13, 129.04, 128.92, 128.47, 126.26, 125.23, 122.71, 79.71, 78.95.
HRMS(ESI-TOF) m/z theoretical value: $C_{20}H_{26}N_3O_5[M-I]^+$, 382.1550, measured value: 382.1555.

Compound 150:

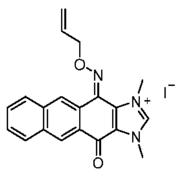

(E) and (Z)-4-((allyloxy)imino)-1,3-dimethyl -11-oxo-4,11-dihydro-1H-anthracene [2,3-d] imidazole-3-iodide

$^1$H NMR (400 MHz, DMSO-$d_6$) δ 9.67 (s, 2H), 9.58 (s, 2H), 8.98 (s, 2H), 8.87 (s, 1H), 8.75 (s, 1H), 8.38 - 8.34 (m, 2H), 8.23 (dd, J = 6.4, 2.8 Hz, 3H), 7.87 - 7.79 (m, 5H), 6.32 - 6.17 (m, 5H), 5.18 (dt, J = 5.8, 1.2 Hz, 3H), 5.07 (d, J = 6.0 Hz, 2H), 4.23 - 4.20 (m, 5H), 4.20 (s, 3H), 4.11 (d, J = 1.6 Hz, 8H).
HRMS(ESI-TOF) m/z theoretical value: $C_{20}H_{26}N_3O_5[M-I]^+$, 332.1334, measured value: 332.1335.

Compound 151:

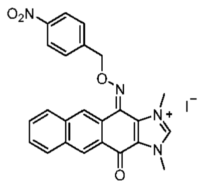

(E) and (Z) -1,3-dimethyl-4-(((4-nitrobenzyl)oxy)imino)-11-oxo-4,11-dihydro-1H-anthracene [2,3-d] imidazole-3-iodide

$^1$H NMR (400 MHz, DMSO-$d_6$) δ 9.74 (s, 1H), 9.53 (s, 1H), 8.94 (s, 1H), 8.34 - 8.24 (m, 5H), 7.86 - 7.79 (m, 3H), 5.86 (s, 2H), 4.18 (s, 3H), 3.97 (s, 3H).
HRMS(ESI-TOF) m/z theoretical value: $C_{20}H_{26}N_3O_5[M-I]^+$, 427.1401, measured value: 427.1405.
Compounds obtained by the method of Examples 1-10 may be used to obtain the final imidazolium derivative in Examples 11-13. In addition, other similar quinonoxime imidazolium derivatives and synthetic intermediates thereof may be prepared by the method recited in the above examples or preparation methods, or prepared by some change methods commonly known by those skilled in the art.
Effects of the compounds in the present invention are confirmed by the following tests.

## Test Example 1. *In vitro* inhibition test for cancer cell proliferation

Test method: pancreatic cancer cells CFPAC-1 were cultured in a Dalbeco-improved Eagle medium with the

addition of 10% FBS and 5% double-antibody. CFPAC-1 cancer cells were inoculated into a 96-well plate for incubation for 24 h; in the following day, test compounds having various concentrations of (100, 20, 10, 1, 0.1, 0.01, and 0.001 μM) and positive control agents were added per well; corresponding volume of fresh medium was added to blank control. After addition for 24 h or 48 h, cell proliferation was evaluated by chromogenic reaction of MTT formazan.

Test result: compounds of the present invention inhibit proliferation of cancer cells well; the median lethal concentration $IC_{50}$ value is below 1 μM, and the optimal compound may be up to 10 nM below.

| Compound No. | $IC_{50}$(A549) | $IC_{50}$(HeLa) | $IC_{50}$(MCF-7 ) | $IC_{50}$(L929 ) |
|---|---|---|---|---|
| 91 | 0.53 | 0.67 | 0.84 | 4.70 |
| 92 | 0.54 | 1.63 | 0.94 | 13.47 |
| 93 | 1.03 | 2.18 | 1.43 | 32.73 |
| 94 | 0.67 | 1.43 | 0.84 | 39.23 |
| 95 | 1.89 | 18.3 | 2.00 | 26.28 |
| 96 | 2.43 | 9.70 | 1.26 | 57.38 |
| 97 | 1.37 | 1.97 | 1.05 | 74.02 |
| 98 | 0.59 | 2.18 | 1.81 | 38.90 |
| 99 | 1.72 | 5.74 | 1.63 | 19.8 |
| 100 | 0.62 | 2.18 | 0.87 | 6.64 |
| 101 | 0.64 | 1.80 | 0.87 | 13.05 |
| 102 | 0.63 | 1.05 | 0.89 | 37.35 |
| 103 | 0.60 | 2.73 | 1.63 | >100 |
| 104 | 12.2 | 12.48 | 1.43 | >100 |
| 105 | 1.11 | 22.58 | 6.12 | >100 |
| 106 | 11.2 | 9.2 | 20.2 | >100 |
| 107 | 0.60 | >100 | 9.85 | >100 |
| 108 | 0.50 | 2.38 | 1.63 | >100 |
| 109 | 11.2 | 50.0 | 57.8 | >100 |
| 110 | 17.2 | 49.0 | 42.3 | >100 |
| 111 | 11.6 | 19.7 | 24.6 | >100 |
| 112 | 0.57 | 7.7 | 3.7 | 27.3 |
| 113 | 0.57 | 7.1 | 10.0 | 73.4 |
| 114 | 2.42 | 33.8 | 33.0 | >100 |
| 115 | 0.13 | 2.5 | 1.64 | 17.6 |
| 116 | 2.3 | >100 | >100 | >100 |
| 117 | 5.2 | 87.8 | 67.7 | >100 |
| 118 | 0.54 | 0.78 | 0.89 | 12.9 |
| 119 | 0.52 | 33.0 | 18.1 | 4.6 |
| 120 | 0.69 | 26.0 | 13.0 | >100 |
| 121 | 0.09 | 28.0 | 6.8 | 61.0 |
| 122 | 1.8 | 52.0 | 33.0 | >100 |
| 123 | 0.007 | 23.0 | 1.8 | 3.8 |
| 124 | 0.009 | 11.3 | 3.3 | 4.6 |
| 125 | 0.005 | 5.3 | 4.5 | 45.3 |

(continued)

| Compound No. | $IC_{50}$(A549) | $IC_{50}$(HeLa) | $IC_{50}$(MCF-7 ) | $IC_{50}$(L929 ) |
|---|---|---|---|---|
| 126 | 0.59 | 39.0 | 12.2 | 85.2 |
| 127 | 0.09 | 25.1 | 7.9 | 29.6 |
| 128 | 0.004 | 4.8 | 2.9 | 6.5 |
| 129 | 0.07 | 8.7 | 12.1 | 26.4 |
| 130 | 0.6 | 48.4 | 56.2 | >100 |
| 131 | 0.07 | 21.0 | 9.9 | 24.7 |
| 132 | 0.06 | 46.3 | 56.1 | 2.4 |
| 133 | 0.08 | 23.9 | 17.9 | 16.0 |
| 134 | 0.07 | 17.5 | 25.6 | 4.9 |
| 135 | 0.16 | 45.4 | 34.9 | 4.03 |
| 136 | 0.06 | 23.9 | 29.8 | 4.4 |
| 137 | 0.05 | 15.4 | 24.8 | 9.7 |
| 138 | 0.004 | 4.2 | 5.9 | 25.9 |
| 139 | 0.005 | 6.8 | 8.6 | 19.9 |
| 140 | 0.007 | 9.8 | 10.9 | 34.5 |
| 141 | 0.05 | 23.9 | 45.6 | 23.4 |
| 142 | 0.06 | 34.0 | 43.5 | 61.3 |
| 143 | 0.03 | 25.8 | 34.9 | 34.8 |
| 144 | 0.06 | 23.3 | 33.0 | 46.6 |
| 145 | 0.04 | 34.2 | 34.8 | 45.1 |
| 146 | 0.009 | 23.0 | 32.8 | 24.6 |
| 147 | 0.02 | 13.2 | 26.8 | 15.7 |
| 148 | 0.09 | 23.6 | 34.6 | 44.1 |
| 149 | 0.33 | 67.4 | 75.3 | >100 |
| 150 | 0.46 | 34.4 | 45.9 | 97.8 |
| 151 | 0.12 | 56.8 | 78.8 | 89.6 |

In addition, compounds (I), (II), (III), and (IV) of the present invention have good inhibition activity against the proliferation of other cancer cells, including non-small cell lung cancer cell (H460, H1299), liver tumor cell (HepG2), rectal cancer cell (HT29), leukemia cell (CCRF-CEM), prostate cancer cell (PC-3), ovarian carcinoma cell (SKOV-3), melanoma cell (A375), myeloma cell (RPMI-8226), esophageal carcinoma cell (TE-1), pancreatic cancer cell (CFPAC-1, PANC-1, SW1990, ASPC-1, and BXPC-3), bladder cancer cell (5637), and gastric cancer cell (KATO-III). Results are as follows:

| $IC_{50}$ | Compound 123 | Compound 124 | Compound 125 | Compound 128 |
|---|---|---|---|---|
| H460 | 0.22 | 0.34 | 0.16 | 0.14 |
| H1299 | 0.43 | 0.45 | 0.26 | 0.17 |
| HepG2 | 0.01 | 0.02 | 0.009 | 0.02 |
| HT29 | 6.7 | 7.9 | 5.4 | 9.8 |
| CCRF-CEM | 0.12 | 0.22 | 0.09 | 0.53 |
| PC-3 | 13.5 | 23.1 | 26.5 | 8.8 |
| SKOV-3 | 0.09 | 0.10 | 0.09 | 0.08 |

(continued)

| $IC_{50}$ | Compound 123 | Compound 124 | Compound 125 | Compound 128 |
|---|---|---|---|---|
| A375 | 4.65 | 5.65 | 2.21 | 1.66 |
| RPMI-8226 | 90.9 | 99.2 | 89.0 | 78.9 |
| TE-1 | 0.07 | 0.08 | 0.06 | 0.009 |
| CFPAC-1 | 0.007 | 0.009 | 0.006 | 0.008 |
| PANC-1 | 0.76 | 0.89 | 0.97 | 0.1 |
| SW1990 | 0.008 | 0.009 | 0.009 | 0.009 |
| ASPC-1 | 0.007 | 0.008 | 0.005 | 0.008 |
| BXPC-3 | 0.004 | 0.006 | 0.003 | 0.006 |
| 5637 | 0.05 | 0.06 | 0.04 | 0.01 |
| KATO-III | 0.01 | 0.02 | 0.009 | 0.009 |

**Test Example 2. *In vivo* inhibition test of cancer cell proliferation**

Test method: $3\times10^7$ CFPAC-1 pancreatic cancer cells were transplanted to subcutaneous tissues of armpit of a Balb/c naked mouse; administration was conducted when the tumor grew to 50-100 $mm^3$, 100-200 $mm^3$, and greater than 200 $mm^3$; the mouse was administered once every other three days, 7 times in total, and 7 mg/kg each time. Normal saline was injected in the control group. Tumor length and diameter were measured by calipers and weight of the moue was weighed until the next day of the final administration. Gross tumor volume was calculated by the following equation:

$$\text{gross tumor volume (mm}^3\text{)} = 1/2 \times [\text{short diameter (mm)}]^2 \times \text{long diameter (mm)}$$

Test result: in this test, compounds (I) and (II) of the present invention inhibit cancer cell proliferation well; for example, Examples 123, 124, 125, and 128 show 100% proliferation inhibition activity at an administration dosage of 7 mg/kg compared with the positive control group; compounds 121, 132, 137, 138, 139, 140, 143, 144, 145, 146, and 147 have inhibition ratios of being up to 80% above, and are dose-dependent obviously.

The compounds of the present invention also show good an inhibition effect on cancer cell proliferation after being transplanted into animal models with other cancer cells (non-small cell lung cancer A549).

**Test Example 3. Compound toxicity test**

Test method: the compounds 121, 123, 124, 125, 128, 132, 137, 138, 139, 140, 143, 144, 145, 146, and 147 in the examples of the present invention were administered every day via tail intravenous injection at a dose of 5 mg/kg for 7 consecutive days, and then body weight changes of the balb/c mice were monitored.

Test result: the mice are in good condition without obviously reduced weight, but gemcitabine mice in the positive control group have obviously reduced weight and are dispirited.

**Test Example 4. Antiviral activity**

Experimental method: 4.59 mg of the compound 125 were weighed and put to a 15 mL centrifugal tube with the addition of 10 mL of sterile water, and dissolved via vortex oscillation until there was no obvious yellow particle, then subpackaged and stored at -20°C, 200 μL/piece (concentration: 1 mM); 2.79 mg Cidofovir (molecular weight: 279) were weighed and added to the 15 mL centrifugal tube, dissolved with the addition of 10 mL sterile water, then subpackaged and stored at -20°C, 200 μL/piece (concentration: 1 mM).

Plaque test:

1. 293A cells were paved into a 24-well plate with 150,000 cells per well, and cultured at 37°C for 12-24 h, then used for test after growing to 90%.

2. 2% low-melting point agarose gel was melted and put to a 56°C water bath kettle for further use.
3. Adenovirus having a titer of $10^9$ $TCID^{50}$/mL was diluted by $10^5$ folds; medium in the 24-well plate was removed, and 200 μL of adenoviral diluent was added per well and incubated in a 37°C 5% $CO_2$ incubator, shaken once every 30 min, and incubated for 1 h in total.
4. The drug compound 125 to be tested and Cidofovir were double diluted with 2 x MEM (16 μL compound 125 was added to 8 mL of 2 x MEM, oscillated and mixed well, then 4 mL mixed solution was taken and added to fresh 4 mL of 2 X MEM and mixed well, and 5 dilutions were conducted, being 2 μM, 1 μM, 500 nM, 250 nM, and 125 nM, respectively; the maximum concentration of the compound 125 was 1 μM. Similarly, 400 μL Cidofovir was added to 7.6 mL of 2 x MEM, oscillated and mixed well, then 4 mL mixed solution was taken and added to fresh 4 mL of 2 X MEM and mixed well, and 5 dilutions were conducted, being 50 μM, 25 μM, 12.5 μM, 6.25 μM, and 3.125 μM, respectively); the diluted solution was put at 37°C for further use.
4. The viral solution was discarded, and agarose gel preheated at 56°C was added to 2xMEM by the same volume and mixed well, and then 2 mL of mixed solution was added per well, and incubated at room temperature for 30 min (gel).
5. The incubation was conducted in a 37°C incubator, and during the incubation, a fluorescent microscope was used to observe the replication of virus.

Experimental result: 4 days later, lots of blank plaques formed by non-CPE could be observed in the Cidofovir group; 5 days later, non-CPE blank plaque also appeared in the low concentration group of the Compound 127. The Compound 125 has an inhibiting effect on viral replication, and is very obviously dose-dependent.

Compared with the prior art, the present invention has the following beneficial effects:

1. In the present invention, the quinone-fused imidazole framework is modified systematically to obtain a series of quinonoxime imidazole derivatives and quinonoxime quinone imidazole derivatives having novel structures.
2. The present invention adopts exploratory novel preparation methods; synthesis is simpler; raw materials are easier to get and low in cost; moreover, the present invention is suitable for large-scale preparation.

Detailed examples of the present invention are described above. It need be understood that the present invention is not limited to the above specific embodiments. Those skilled in the art would make various transformations or amendments within the scope of the claims, which will affect the substantive content of the present invention.

## Claims

**1.** A fused quinonoxime imidazolium derivative, having a structure represented by the following Formula (I) or (II):

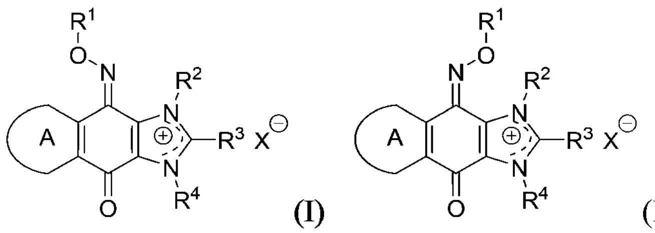

(I) (II);

wherein,

a ring A is selected from an aromatic ring having zero or one substituent, or a heteroaromatic ring having zero or one substituent;
$R^1$, $R^2$, $R^3$, and $R^4$ are the same or different, and each represent -hydrogen atom, -lower linear/branched alkyl having zero or one substituent, -lower alkenyl having zero or one substituent, -lower alkynyl having zero or one substituent, -saturated heterocyclyl having zero or one substituent, -cycloalkyl having zero or one substituent, -cycloalkenyl having zero or one substituent, -aryl having zero or one substituent, and -heteroaryl having zero or one substituent;
and substituents on the ring A as well as in the $R^1$, the $R^2$, the $R^3$, and the $R^4$ are selected from a radical of group B;
the group B: -lower linear/branched alkyl having zero or one substituent, -lower alkenyl having zero or one substituent, -lower alkynyl having zero or one substituent, -saturated heterocyclyl having zero or one substituent, -cycloalkyl having zero or one substituent, - cycloalkenyl having zero or one substituent, -aryl having zero or one substituent, -heteroaryl having zero or one substituent, $-OR^a$, $-SR^a$, -O-lower alkylene-$OR^a$, -O-lower alkylene-O-lower alkylene-$OR^a$, -O-lower alkylene-O-lower alkylene-O-lower alkylene-$OR^a$, -O-lower alkylene-$NR^aR^b$, -O-lower alkylene-O-lower alkylene-$NR^aR^b$, -O-lower alkylene-$NR^c$-lower alkylene-$NR^aR^b$, -O-CO-$NR^aR^b$, -$SOR^a$, -$SO_2R^a$, -$SO_2NR^aR^b$, -$NR^a$-$SO_2R^b$, -$NR^aR^b$, -$NR^c$-lower alkylene-$NR^aR^b$, -N(-lower alkylene-$NR^aR^b)_2$, -$NO_2$, -CN, -halogen, -$CO_2R^a$, -$COO^-$, - $CONR^aR^b$, -$CONR^a$-O-$R^b$, -$NR^a$-$COR^b$, -$NR^a$-CO-$NR^bR^c$, -$OCOR^a$, and -$COR^a$;

$R^a$, $R^b$, and $R^c$ are the same or different, and each represent -hydrogen atom, -lower linear/branched alkyl having zero or one substituent, -lower alkenyl having zero or one substituent, -lower alkynyl having zero or one substituent, -saturated heterocyclyl having zero or one substituent, -cycloalkyl having zero or one substituent, -cycloalkenyl having zero or one substituent, -aryl having zero or one substituent, -heteroaryl having zero or one substituent, -lower alkylene-(5 to 7-membered saturated heterocycle having zero or one substituent), -lower alkylene-(cycloalkyl having zero or one substituent), -lower alkylene-(aryl having zero or one substituent), and -lower alkylene-(heteroaryl having zero or one substituent), wherein the substituents refer to lower alkyl or heteroalkyl;

$X^-$ is a coanion comprising a halide ion, a sulfonate ion, an acetate ion, a trifluoroacetate ion, a carbonate ion, a sulfate ion; and when an anion on a substituent and an imidazolium cation form an inner salt, $X^-$ is absent;

and wherein the lower linear/branched alkyl and lower alkenyl is a hydrocarbon having 1 to 6 carbon atoms.

**2.** The fused quinonoxime imidazolium derivative according to claim 1, wherein in the structural formula,

the ring A is selected from a benzene ring, a naphthalene ring, or a $NO_2$-substituted benzene ring;

the $R^1$ is selected from -hydrogen atom, -lower alkyl, -lower alkynyl, -lower alkenyl, - lower linear/branched alkyl-O-lower linear/branched alkyl, -lower linear/branched alkyl-O-aryl, -lower linear/branched alkyl-O-lower linear/-branched alkyl-O-lower linear/branched alkyl, -lower linear/branched alkyl-O-lower linear/branched alkyl-O-lower linear/branched alkyl-O-lower linear/branched alkyl, -aryl, -(5 to 7-membered) saturated heterocycle, and -heteroaryl;

the $R^2$ is selected from lower linear/branched alkyl;

the $R^3$ is selected from -hydrogen atom, -lower linear/branched alkyl, -(3 to 7-membered) saturated cycloalkyl, -heterocyclyl, -aryl, -aryl substituted by lower linear/branched alkyl, - aryl substituted by halogen, -heteroaryl, -heteroaryl substituted by lower linear/branched alkyl, -O-lower linear/branched alkyl, -S-lower linear/branched alkyl, -acyl, and -acyl substituted by lower alkyl; and

the $R^4$ is selected from -hydrogen atom, -lower linear/branched alkyl, -lower alkenyl, - lower alkynyl, -aryl, -CN, -(3 to 7-membered) saturated cycloalkyl, -aryl substituted by halogen, -aryl substituted by lower alkyl, -heteroaryl, -$CO_2R^a$, -saturated heterocyclyl, -lower linear/branched alkyl-$OR^a$, -lower linear/branched alkyl-O-lower linear/-branched alkyl, - lower linear/branched alkyl-S-lower linear/branched alkyl, -lower linear/branched alkyl-O-lower linear/branched alkyl-$OR^a$, wherein the $R^a$ is -lower linear/branched alkyl.

**3.** A fused quinonoxime imidazole derivative and a hydrochloride thereof, having a structure represented by the following Formula (III) or (IV):

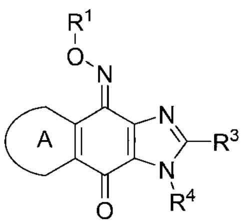
(III)
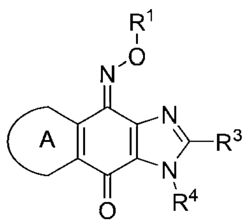
(IV);

wherein in the structural formula,

the ring A is selected from a -benzene ring, a -naphthalene ring, or a $NO_2$-substituted benzene ring;

the $R^1$ is selected from -hydrogen atom, -lower alkyl, -lower alkynyl, -lower alkenyl, - lower linear/branched alkyl-O-lower linear/branched alkyl, -lower linear/branched alkyl-O-aryl, -lower linear/branched alkyl-O-lower linear/-branched alkyl-O-lower linear/branched alkyl, -lower linear/branched alkyl-O-lower linear/branched alkyl-O-lower linear/branched alkyl-O-lower linear/branched alkyl, -aryl, -(5 to 7-membered) saturated heterocycle, and -heteroaryl;

the $R^3$ is selected from -hydrogen atom, -lower linear/branched alkyl, -(3 to 7-membered) saturated cycloalkyl, -heterocyclyl, -aryl, -aryl substituted by lower linear/branched alkyl, - aryl substituted by halogen, -heteroaryl, -heteroaryl substituted by lower linear/branched alkyl, -O-lower linear/branched alkyl, -S-lower linear/branched

alkyl, -acyl, and acyl substituted by lower alkyl; and
the $R^4$ is selected from -hydrogen atom, -lower linear/branched alkyl, -lower alkenyl, - lower alkynyl, -aryl, -CN, -(3 to 7-membered) saturated cycloalkyl, -aryl substituted by halogen, -aryl substituted by lower alkyl, -heteroaryl, $-CO_2R^a$, -saturated heterocyclyl, -lower linear/branched alkyl-$OR^a$, -lower linear/branched alkyl-O-lower linear/-branched alkyl, - lower linear/branched alkyl-S-lower linear/branched alkyl, -lower linear/branched alkyl-O-lower linear/branched alkyl-$OR^a$, wherein the $R^a$ is lower linear/branched alkyl.
and wherein the lower linear/branched alkyl and lower alkenyl is a hydrocarbon having 1 to 6 carbon atoms.

**4.** A pharmaceutical composition, comprising one or more of the quinonoxime imidazolium derivative according to claim 1 or 2, and a pharmaceutically acceptable carrier.

**5.** The pharmaceutical composition according to claim 4 for use in preparing an anticancer, anti-bacterial, or antiviral drug.

**6.** A preparation method of the fused quinonoxime imidazolium derivative according to claim 1 or 2, or the fused quinonoxime imidazole derivative according to claim 3, comprising the following steps:

A, serving a protic solvent as a solvent, and subjecting a substituted quinone imidazole derivative

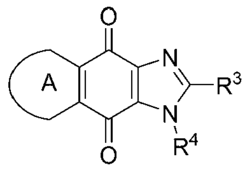
(V)

to an oximation reaction with $R^1ONH_2$•HCl under a catalytic action of a weak base or with corresponding $R^1ONH_2$ under an action of a weakbase hydrochloride, to obtain quinonoxime imidazole derivatives

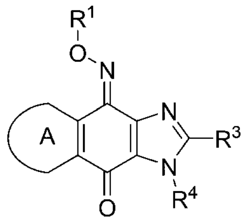
(III)

and

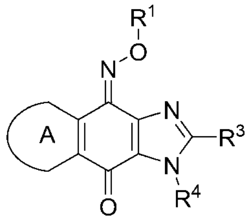
(IV);

and
B, performing an N-alkylation reaction on the quinonoxime imidazole derivative (III) or (IV) and a corresponding halide reagent $R^2X$ in an organic solvent, to obtain the quinonoxime imidazolium derivative (I) or (II).

**7.** The preparation method according to claim 6, wherein in the step A, a molar ratio of the quinone imidazole derivative

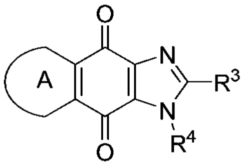

(V),

to the $R^1ONH_2$•HCl, and to the weak base is 1:1.2-5:0.001-0.01; the protic solvent is selected from methanol, ethanol, and isopropanol; the weak base is selected from pyridine, triethylamine, and potassium carbonate; and the reaction is performed at 90-125°C for 12-48 h.

8. The preparation method according to claim 6, wherein in the step B, a molar ratio of the quinonoxime imidazole derivative (III) or (IV) to the halide reagent $R^2X$ is 1:1-1:100; the solvent is selected from acetonitrile, ethyl acetate, and tetrahydrofuran; and the reaction is performed at 50-120°C for 8-48 h.

## Patentansprüche

1. Kondensiertes Chinonoxim-Imidazolium-Derivat, das eine Struktur aufweist, die durch die folgende Formel (I) oder (II) dargestellt wird:

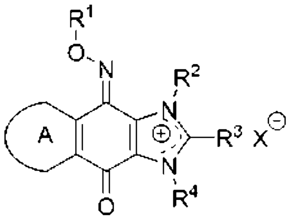

(I)

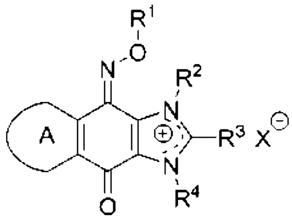

(II);

wobei,

ein Ring A ausgewählt ist aus einem aromatischen Ring, der null oder einen Substituenten aufweist, oder einem heteroaromatischen Ring, der null oder einen Substituenten aufweist;
$R^1$, $R^2$, $R^3$ und $R^4$ gleich oder unterschiedlich sind, und jeweils -Wasserstoffatom, -niederes lineares/verzweigtes Alkyl, das null oder einen Substituenten aufweist, -niederes Alkenyl, das null oder einen Substituenten aufweist, -niederes Alkinyl, das null oder einen Substituenten aufweist, -gesättigtes Heterocyclyl, das null oder einen Substituenten aufweist, -Cycloalkyl, das null oder einen Substituenten aufweist, -Cycloalkenyl, das null oder einen Substituenten aufweist, -Aryl, das null oder einen Substituenten aufweist, und -Heteroaryl, das null oder einen Substituenten aufweist, darstellen;
und Substituenten an dem Ring A sowie in dem $R^1$, dem $R^2$, dem $R^3$ und dem $R^4$ ausgewählt sind aus einem Rest der Gruppe B;
die Gruppe B: -niederes lineares/verzweigtes Alkyl, das null oder einen Substituenten aufweist, -niederes Alkenyl, das null oder einen Substituenten aufweist, -niederes Alkinyl, das null oder einen Substituenten aufweist, -gesättigtes Heterocyclyl, das null oder einen Substituenten aufweist, -Cycloalkyl, das null oder einen Substituenten aufweist, -Cycloalkenyl, das null oder einen Substituenten aufweist, -Aryl, das null oder einen Substituenten aufweist, -Heteroaryl, das null oder einen Substituenten aufweist, $-OR^a$, $-SR^a$, -O-niederes Alkylen-$OR^a$, -O-niederes Alkylen-O-niederes Alkylen-$OR^a$, -O-niederes Alkylen-O-niederes Alkylen-O-niederes Alkylen-$OR^a$, -O-niederes Alkylen-$NR^aR^b$, -O-niederes Alkylen-O-niederes Alkylen-$NR^aR^b$, -O-niederes Alkylen-$NR^c$-niederes Alkylen-$NR^aR^b$, -O-CO-$NR^aR^b$, $-SOR^a$, $-SO_2R^a$, $-SO_2NR^aR^b$, $-NR^a-SO_2R^b$, $-NR^aR^b$, $-NR^c$-niederes Alkylen-$NR^aR^b$, -N(-niederes Alkylen-$NR^aR^b)_2$, -NO2, -CN, -Halogen, $-CO_2R^a$, $-COO^-$, $-CONR^aR^b$, $-CONR^a-O-R^b$, $-NR^a-COR^b$, $-NR^a-CO-NR^bR^c$, $-OCOR^a$ und $-COR^a$;
$R^a$, $R^b$ und $R^c$ gleich oder unterschiedlich sind und jeweils -Wasserstoffatom, -niederes lineares/verzweigtes Alkyl, das null oder einen Substituenten aufweist, -niederes Alkenyl, das null oder einen Substituenten aufweist, -niederes Alkinyl, das null oder einen Substituenten aufweist, -gesättigtes Heterocyclyl, das null oder einen Substituenten aufweist, -Cycloalkyl, das null oder einen Substituenten aufweist, -Cycloalkenyl, das null oder einen Substituenten aufweist, -Aryl, das null oder einen Substituenten aufweist, -Heteroaryl, das null oder einen Substituenten aufweist, -niederes Alkylen-(5- bis 7-gliedriger gesättigter Heterozyklus, der null oder einen

Substituenten aufweist), -niederes Alkylen-(Cycloalkyl, das null oder einen Substituenten aufweist), -niederes Alkylen-(Aryl, das null oder einen Substituenten aufweist) und -niederes Alkylen-(Heteroaryl, das null oder einen Substituenten aufweist) darstellen, wobei sich die Substituenten auf niederes Alkyl oder Heteroalkyl beziehen;
$X^-$ ein Coanion ist, umfassend ein Halogenidion, ein Sulfonatanion, ein Acetatanion, ein Trifluoracetatanion, ein Carbonatanion, ein Sulfatanion; und wenn ein Anion an einem Substituenten und ein Imidazoliumkation ein inneres Salz ausbilden, $X^-$ abwesend ist;
und wobei das niedere lineare/verzweigte Alkyl und niedere Alkenyl ein Kohlenwasserstoff, der 1 bis 6 Kohlenstoffatomen aufweist, ist.

2. Kondensiertes Chinonoxim-Imidazolium-Derivat nach Anspruch 1, wobei in der Strukturformel,

der Ring A ausgewählt ist aus einem Benzolring, einem Naphthalinring oder einem NO2-substituierten Benzolring;
das $R^1$ ausgewählt ist aus -Wasserstoffatom, -niederem Alkyl, -niederem Alkinyl, -niederem Alkenyl, -niederem linearem/verzweigtem Alkyl-O-niederem linearem/verzweigtem Alkyl, -niederem linearem/verzweigtem Alkyl-O-Aryl, -niederem linearem/verzweigtem Alkyl-O-niederem linearem/verzweigtem Alkyl-O-niederem linearem/-verzweigtem Alkyl, -niederem linearem/verzweigtem Alkyl-O-niederem linearem/verzweigtem Alkyl-O-niederem linearem/verzweigtem Alkyl-O-niederem linearem/verzweigtem Alkyl, -Aryl, -(5- bis 7-gliedrigem) gesättigtem Heterozyklus und -Heteroaryl;
das $R^2$ ausgewählt ist aus niedrigerem linearem/verzweigtem Alkyl;
das $R^3$ ausgewählt ist aus -Wasserstoffatom, -niedrigem linearem/verzweigtem Alkyl, -(3- bis 7-gliedrigem) gesättigtem Cycloalkyl, -Heterocyclyl, -Aryl, -Aryl substituiert durch niedriges lineares/verzweigtes Alkyl, -Aryl substituiert durch Halogen, -Heteroaryl, -Heteroaryl substituiert durch niedriges lineares/verzweigtes Alkyl, -O-niedrigem linearem/verzweigtem Alkyl, -S-niedrigem linearem/verzweigtem Alkyl, -Acyl und -Acyl substituiert durch niedriges Alkyl; und
das $R^4$ ausgewählt ist aus -Wasserstoffatom, -niedrigem linearem/verzweigtem Alkyl, -niedrigem Alkenyl, -niedrigem Alkinyl, -Aryl, -CN, -(3-bis 7-gliedrigem) gesättigtem Cycloalkyl, -durch Halogen substituiertem Aryl, -durch niedriges Alkyl substituiertem Aryl, -Heteroaryl, -CO2$R^a$, -gesättigtem Heterocyclyl, -niedrigem linearem/verzweigtem Alkyl-O$R^a$, -niedrigem linearem/verzweigtem Alkyl-O-niedrigem linearem/verzweigtem Alkyl, -niedrigem linearem/verzweigtem Alkyl-S-niedrigem linearem/verzweigtem Alkyl, -niedrigem linearem/verzweigtem Alkyl-O-niedrigem linearem/verzweigtem Alkyl-O$R^a$, wobei das $R^a$ -niedriges lineares/verzweigtes Alkyl ist.

3. Kondensiertes Chinonoxim-Imidazol-Derivat und ein Hydrochlorid davon,, das eine Struktur aufweist, die durch die folgende Formel (III) oder (IV) dargestellt wird:

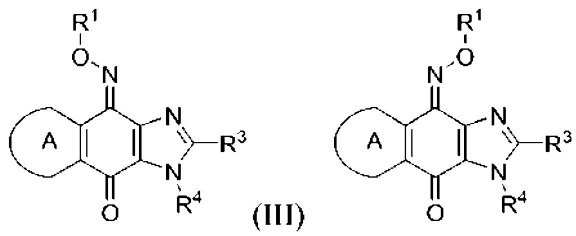
(III) (IV);

wobei in der Strukturformel,
der Ring A ausgewählt ist aus einem -Benzolring, einem -Naphthalinring oder einem $NO_2$-substituierten Benzolring;
das $R^1$ ausgewählt ist aus -Wasserstoffatom, -niederem Alkyl, -niederem Alkinyl, -niederem Alkenyl, -niederem linearem/verzweigtem Alkyl-O-niederem linearem/verzweigtem Alkyl, -niederem linearem/verzweigtem Alkyl-O-Aryl, -niederem linearem/verzweigtem Alkyl-O-niederem linearem/verzweigtem Alkyl-O-niederem linearem/-verzweigtem Alkyl, -niederem linearem/verzweigtem Alkyl-O-niederem linearem/verzweigtem Alkyl-O-niederem linearem/verzweigtem Alkyl-O-niederem linearem/verzweigtem Alkyl, -Aryl, -(5- bis 7-gliedrigem) gesättigtem Heterozyklus und -Heteroaryl;
das $R^3$ ausgewählt ist aus -Wasserstoffatom, -niedrigem linearem/verzweigtem Alkyl, -(3- bis 7-gliedrigem) gesättigtem Cycloalkyl, -Heterocyclyl, -Aryl, -Aryl substituiert durch niedriges lineares/verzweigtes Alkyl, -Aryl substituiert durch Halogen, -Heteroaryl, -Heteroaryl substituiert durch niedriges lineares/verzweigtes Alkyl, -O-niedrigem linearem/verzweigtem Alkyl, -S-niedrigem linearem/verzweigtem Alkyl, -Acyl und Acyl substituiert

durch niedriges Alkyl; und

das $R^4$ ausgewählt ist aus -Wasserstoffatom, -niedrigem linearem/verzweigtem Alkyl, -niedrigem Alkenyl, -niedrigem Alkinyl, -Aryl, -CN, - (3- bis 7-gliedrigem) gesättigtem Cycloalkyl, -durch Halogen substituiertem Aryl, -durch niedriges Alkyl substituiertem Aryl, -Heteroaryl, $-CO_2R^a$, -gesättigtem Heterocyclyl, -niedrigem linearem/verzweigtem Alkyl-$OR^a$, -niedrigem linearem/verzweigtem Alkyl-O-niedrigem linearem/verzweigtem Alkyl, -niedrigem linearem/verzweigtem Alkyl-S-niedrigem linearem/verzweigtem Alkyl, -niedrigem linearem/-verzweigtem Alkyl-O-niedrigem linearem/verzweigtem Alkyl-$OR^a$, wobei das $R^a$ niedriges lineares/verzweigtes Alkyl,

und wobei das niedere lineare/verzweigte Alkyl und niedere Alkenyl ein Kohlenwasserstoff, der 1 bis 6 Kohlenstoffatomen aufweist, ist.

**4.** Pharmazeutische Zusammensetzung, umfassend ein oder mehrere der Chinonoxim-Imidazolium-Derivate nach Anspruch 1 oder 2 und einen pharmazeutisch verträglichen Träger.

**5.** Pharmazeutische Zusammensetzung nach Anspruch 4 zur Verwendung bei einem Herstellen eines Krebsbekämpfungs-, antibakteriellen oder antiviralen Arzneimittels.

**6.** Herstellungsverfahren des kondensierten Chinonoxim-Imidazolium-Derivats nach Anspruch 1 oder 2, oder des kondensierten Chinonoxim-Imidazol-Derivats nach Anspruch 3, umfassend die folgenden Schritte:

A, unter Verwendung eines protischen Lösungsmittels als Lösungsmittel, und Unterwerfen eines substituierten Chinonimidazol-Derivats

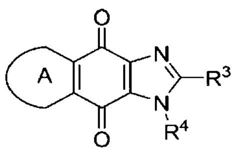

(V) einer Oximierungsreaktion mit $R^1ONH_2$•HCl unter katalytischer Wirkung einer schwachen Base oder mit entsprechendem $R^1ONH_2$ unter Wirkung eines schwachbasischen Hydrochlorids, um Chinonoxim-Imidazol-Derivate

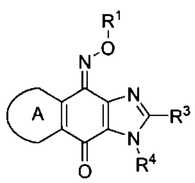
(III) und
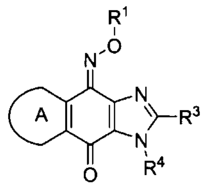
(IV)

zu erhalten; und

B, Durchführen einer N-Alkylierungsreaktion an dem Chinonoxim-Imidazol-Derivat (III) oder (IV) und einem entsprechenden Halogenid-Reagenz $R^2X$ in einem organischen Lösungsmittel, um das Chinonoxim-Imidazolium-Derivat (I) oder (II) zu erhalten.

**7.** Herstellungsverfahren nach Anspruch 6, wobei in dem Schritt A ein Molverhältnis des Chinonimidazol-Derivats

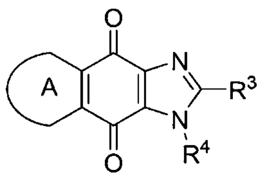
(V)

zu dem $R^1ONH_2$•HCl und zu der schwachen Base 1:1,2-5:0,001-0,01 beträgt; das protische Lösungsmittel ausgewählt ist aus Methanol, Ethanol und Isopropanol; die schwache Base ausgewählt ist aus Pyridin, Triethylamin und

Kaliumcarbonat; und die Reaktion bei 90-125 °C für 12-48 h durchgeführt wird.

**8.** Herstellungsverfahren nach Anspruch 6, wobei in dem Schritt B ein Molverhältnis des Chinonoxim-Imidazol-Derivats (III) oder (IV) zu dem Halogenid-Reagenz $R^2X$ 1:1-1:100 beträgt; das Lösungsmittel ausgewählt ist aus Acetonitril, Ethylacetat und Tetrahydrofuran; und die Reaktion bei 50-120 °C für 8-48 h durchgeführt wird.

## Revendications

**1.** Dérivé de quinonoxime imidazolium condensé, ayant une structure représentée par la Formule (I) ou (II) suivante :

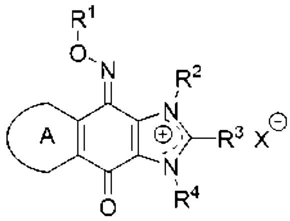 (I) 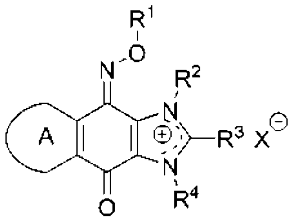 (II);

dans lequel,

un noyau A est choisi parmi un noyau aromatique ayant zéro ou un substituant, ou un noyau hétéroatomique ayant zéro ou un substituant ;

$R^1$, $R^2$, $R^3$ et $R^4$ sont identiques ou différents, et représentent chacun -atome d'hydrogène, -alkyle inférieur linéaire/ramifié ayant zéro ou un substituant, -alcényle inférieur ayant zéro ou un substituant, -alcynyle inférieur ayant zéro ou un substituant, -hétérocyclyle saturé ayant zéro ou un substituant, -cycloalkyle ayant zéro ou un substituant, -cycloalcényle ayant zéro ou un substituant, -aryle ayant zéro ou un substituant, et -hétéroaryle ayant zéro ou un substituant ;

et les substituants sur le noyau A ainsi que dans le $R^1$, le $R^2$, le $R^3$ et le $R^4$ sont choisis parmi un radical du groupe B ;

le groupe B : -alkyle inférieur linéaire/ramifié ayant zéro ou un substituant, - alcényle inférieur ayant zéro ou un substituant, -alcynyle inférieur ayant zéro ou un substituant, -hétérocyclyle saturé ayant zéro ou un substituant, -cycloalkyle ayant zéro ou un substituant, -cycloalcényle ayant zéro ou un substituant, -aryle ayant zéro ou un substituant, -hétéroaryle ayant zéro ou un substituant, $-OR^a$, $-SR^a$, -O-alkylène inférieur-$OR^a$, -O-alkylène inférieur-O- alkylène inférieur-$OR^a$, -O-alkylène inférieur-O-alkylène inférieur-O-alkylène inférieur-$OR^a$, -O-alkylène inférieur-$NR^aR^b$, -O-alkylène inférieur-O-alkylène inférieur-$NR^aR^b$, -O-alkylène inférieur-$NR^c$-alkylène inférieur-$NR^aR^b$, -O-CO-$NR^aR^b$, $-SOR^a$, $-SO_2R^a$, $-SO_2NR^aR^b$, $-NR^a-SO_2R^b$, $-NR^aR^b$, $-NR^c$-alkylène inférieur-$NR^aR^b$, -N(-alkylène inférieur-$NR^aR^b)_2$, $-NO_2$, -CN, -halogène, - $CO_2R^a$, $-COO^-$, - $CONR^aR^b$, $-CONR^a-O-R^b$, $-NR^a-COR^b$, $-NR^a-CO-NR^bR^c$, $-OCOR^a$ et $-COR^a$ ;

$R^a$, $R^b$ et $R^c$ sont identiques ou différents, et représentent chacun -atome d'hydrogène, -alkyle inférieur linéaire/ramifié ayant zéro ou un substituant, -alcényle inférieur ayant zéro ou un substituant, -alcynyle inférieur ayant zéro ou un substituant, -hétérocyclyle saturé ayant zéro ou un substituant, -cycloalkyle ayant zéro ou un substituant, -cycloalcényle ayant zéro ou un substituant, -aryle ayant zéro ou un substituant, -hétéroaryle ayant zéro ou un substituant, -alkylène inférieur-(hétérocycle saturé de 5 à 7 chaînons ayant zéro ou un substituant), -alkylène inférieur-(cycloalkyl ayant zéro ou un substituant), -alkylène inférieur-(aryl ayant zéro ou un substituant), et -alkylène inférieur-(hétéroaryl ayant zéro ou un substituant), dans lequel les substituants font référence à un alkyle inférieur ou à un hétéroalkyle ;

$X^-$ est un co-anion comprenant un ion halogénure, un ion sulfonate, un ion acétate, un ion trifluoroacétate, un ion carbonate, un ion sulfate ; et lorsqu'un anion sur un substituant et un cation imidazolium forment un sel interne, $X^-$ est absent ;

et dans lequel l'alkyle inférieur linéaire/ramifié et l'alcényle inférieur sont un hydrocarbure ayant 1 à 6 atomes de carbone.

**2.** Dérivé de quinonoxime imidazolium condensé selon la revendication 1, dans lequel dans la formule structurelle,

le noyau A est choisi parmi un noyau benzène, un noyau naphtalène, ou un noyau benzène substitué par $NO_2$ ;

le $R^1$ est choisi parmi -atome d'hydrogène, -alkyle inférieur, -alcynyle inférieur, -alcényle inférieur, - alkyle inférieur

linéaire/ramifié-O-alkyl inférieur linéaire/ramifié, -alkyle inférieur linéaire/ramifié-O-aryl, -alkyle inférieur linéaire/ramifié-O-alkyl inférieur linéaire/ramifié-O-alkyl inférieur linéaire/ramifié, -alkyle inférieur linéaire/ramifié-O-alkyl inférieur linéaire/ramifié-O-alkyl inférieur linéaire/ramifié-O-alkyl inférieur linéaire/ramifié, -aryle, -hétérocycle saturé (de 5 à 7 chaînons), et - hétéroaryle ;

le $R^2$ est choisi parmi alkyle inférieur linéaire/ramifié ;

le $R^3$ est choisi parmi -atome d'hydrogène, -alkyle inférieur linéaire/ramifié, - cycloalkyle saturé (de 3 à 7 chaînons), -hétérocyclyle, -aryle, -aryle substitué par alkyle inférieur linéaire/ramifié, -aryle substitué par halogène, -hétéroaryle, - hétéroaryle substitué par alkyle inférieur linéaire/ramifié, -O-alkyle inférieur linéaire/-ramifié, -S-alkyle inférieur linéaire/ramifié, -acyle, et -acyle substitué par alkyle inférieur ; et

le $R^4$ est choisi parmi -atome d'hydrogène, -alkyle inférieur linéaire/ramifié, - alcényle inférieur, - alcynyle inférieur, -aryle, -CN, -cycloalkyle saturé (de 3 à 7 chaînons), -aryle substitué par halogène, -aryle substitué par alkyle inférieur, - hétéroaryle, $-CO_2R^a$, -hétérocyclyle saturé, -alkyle inférieur linéaire/ramifié-$OR^a$, - alkyle inférieur linéaire/ramifié-O-alkyl inférieur linéaire/ramifié, -alkyle inférieur linéaire/ramifié-S-alkyl inférieur linéaire/ramifié, -alkyle inférieur linéaire/ramifié-O-alkyl inférieur linéaire/ramifié-$OR^a$, dans lequel le $R^a$ est -alkyle inférieur linéaire/ramifié.

**3.** Dérivé de quinonoxime imidazole condensé et chlorhydrate de celui-ci, ayant une structure représentée par la Formule (III) ou (IV) suivante :

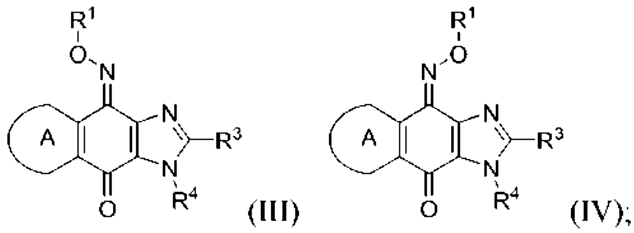

(III) (IV);

dans lequel dans la formule structurelle,

le noyau A est choisi parmi un -noyau benzène, un -noyau naphtalène, ou un noyau benzène substitué par $NO_2$ ;

le $R^1$ est choisi parmi -atome d'hydrogène, -alkyle inférieur, -alcynyle inférieur, -alcényle inférieur, - alkyle inférieur linéaire/ramifié-O-alkyl inférieur linéaire/ramifié, -alkyle inférieur linéaire/ramifié-O-aryl, -alkyle inférieur linéaire/ramifié-O-alkyl inférieur linéaire/ramifié-O-alkyl inférieur linéaire/ramifié, -alkyle inférieur linéaire/ramifié-O-alkyl inférieur linéaire/ramifié-O-alkyl inférieur linéaire/ramifié-O-alkyl inférieur linéaire/ramifié, -aryle, -hétérocycle saturé (de 5 à 7 chaînons), et - hétéroaryle ;

le $R^3$ est choisi parmi -atome d'hydrogène, -alkyle inférieur linéaire/ramifié, - cycloalkyle saturé (de 3 à 7 chaînons), -hétérocyclyle, -aryl, -aryle substitué par alkyle inférieur linéaire/ramifié, -aryle substitué par halogène, -hétéroaryle, -hétéroaryle substitué par alkyle inférieur linéaire/ramifié, -O-alkyle inférieur linéaire/ramifié, -S-alkyle inférieur linéaire/ramifié, -acyle, et -acyle substitué par alkyle inférieur ; et

le $R^4$ est choisi parmi -atome d'hydrogène, -alkyle inférieur linéaire/ramifié, - alcényle inférieur, -alcynyle inférieur, -aryle, -CN, -cycloalkyle saturé (de 3 à 7 chaînons), -aryle substitué par halogène, -aryle substitué par alkyle inférieur, - hétéroaryle, $-CO_2R^a$, -hétérocyclyle saturé, -alkyle inférieur linéaire/ramifié-$OR^a$, - alkyle inférieur linéaire/ramifié-O-alkyl inférieur linéaire/ramifié, -alkyle inférieur linéaire/ramifié-S-alkyl inférieur linéaire/ramifié, -alkyle inférieur linéaire/ramifié-O-alkyle inférieur linéaire/ramifié-$OR^a$, dans lequel le $R^a$ est alkyle inférieur linéaire/ramifié.

et dans lequel l'alkyle inférieur linéaire/ramifié et l'alcényle inférieur sont un hydrocarbure ayant 1 à 6 atomes de carbone.

**4.** Composition pharmaceutique, comprenant un ou plusieurs des dérivés de quinonoxime imidazolium selon la revendication 1 ou 2, et un support pharmaceutiquement acceptable.

**5.** Composition pharmaceutique selon la revendication 4 pour utilisation dans la préparation d'un médicament anticancéreux, antibactérien ou antiviral.

**6.** Procédé de préparation du dérivé de quinonoxime imidazolium condensé selon la revendication 1 ou 2, ou du dérivé de quinonoxime imidazole condensé selon la revendication 3, comprenant les étapes suivantes :

A, le fait de se servir d'un solvant protique en tant que solvant, et de soumettre un dérivé de quinone imidazole

substitué

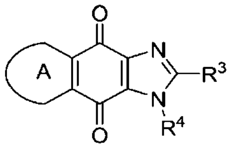

(V)

à une réaction d'oximation avec $R^1ONH_2$•HCl sous une action catalytique d'une base faible ou avec un $R^1ONH_2$ correspondant sous une action d'un chlorhydrate faiblement basique, pour obtenir des dérivés de quinonoxime imidazole

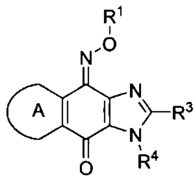

(III)

et

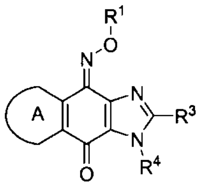

(IV) ;

et

B, la mise en œuvre d'une réaction de N-alkylation sur le dérivé de quinonoxime imidazole (III) ou (IV) et un réactif halogénure correspondant $R^2X$ dans un solvant organique, pour obtenir le dérivé de quinonoxime imidazolium (I) ou (II).

**7.** Procédé de préparation selon la revendication 6, dans lequel dans l'étape A, un rapport molaire du dérivé de quinone imidazole

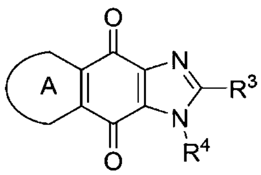

(V),

au $R^1ONH_2$•HCl, et à la base faible est de 1:1,2 à 5:0,001 à 0,01 ; le solvant protique est choisi parmi méthanol, éthanol et isopropanol ; la base faible est choisie parmi pyridine, triéthylamine et carbonate de potassium ; et la réaction est mise en œuvre à 90 à 125 °C pendant 12 à 48 h.

**8.** Procédé de préparation selon la revendication 6, dans lequel dans l'étape B, un rapport molaire du dérivé de quinonoxime imidazole (III) ou (IV) au réactif halogénure $R^2X$ est de 1:1 à 1:100 ; le solvant est choisi parmi acétonitrile, acétate d'éthyle et tétrahydrofurane ; et la réaction est mise en œuvre à 50 à 120 °C pendant 8 à 48 h.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20090163545 A1
- WO 2009023773 A2
- WO 2012149523 A1
- JP 2014156400 A
- US 20150086535 A1
- WO 2001060803 A1
- WO 20010823 A1
- WO 2004092160 A1
- WO 20041028 A1
- WO 2012161177 A1
- WO 20121129 A1
- WO 2013148649 A1
- WO 20131003 A1
- WO 2014142220 A1
- WO 20140918 A1